(19) Europäisches Patentamt
European Patent Office
Office européen des brevets

(11) Veröffentlichungsnummer: **0 419 944 B1**

# (12) EUROPÄISCHE PATENTSCHRIFT

(45) Veröffentlichungstag der Patentschrift: **15.03.95**

(51) Int. Cl.[6]: **C07D 263/34**, C07D 277/56, A01N 43/76, A01N 43/78

(21) Anmeldenummer: **90117567.9**

(22) Anmeldetag: **12.09.90**

## (54) Oxazol- bzw. Thiazolcarbonsäureamide.

(30) Priorität: **26.09.89 DE 3932052**

(43) Veröffentlichungstag der Anmeldung:
**03.04.91 Patentblatt 91/14**

(45) Bekanntmachung des Hinweises auf die Patenterteilung:
**15.03.95 Patentblatt 95/11**

(84) Benannte Vertragsstaaten:
**BE CH DE ES FR GB IT LI NL**

(56) Entgegenhaltungen:
**DE-A- 2 254 944**
**FR-A- 1 516 777**
**FR-A- 2 201 895**
**US-A- 4 260 765**

**BULLETIN DE LA SOCIETE CHIMIOUE DE FRANCE, Nr. 6, Juni 1969, Seiten 2152-2152, Paris, FR; M Robba etal.:"Synthèse d'intermédiairesde la thiazolo [4,5-d]pyridazine. II. Amides et hydrazides d'acides thiazole-carboxyliques"**

(73) Patentinhaber: **BASF Aktiengesellschaft**
**Carl-Bosch-Strasse 38**
**D-67063 Ludwigshafen (DE)**

(72) Erfinder: **Ditrich, Klaus, Dr.**
**Paray-le-Monial-Strasse 12**
**D-6702 Bad Duerkheim (DE)**
Erfinder: **Maywald, Volker, Dr.**
**Berner Weg 24**
**D-6700 Ludwigshafen (DE)**
Erfinder: **Hamprecht, Gerhard, Dr.**
**Rote-Turm-Strasse 28**
**D-6940 Weinheim (DE)**
Erfinder: **Harreus, Albrecht, Dr.**
**Teichgasse 13**
**D-6700 Ludwigshafen (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**

**EP 0 419 944 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Oxazol- bzw. Thiazolcarbonsäureamide der Formeln Ia und Ib

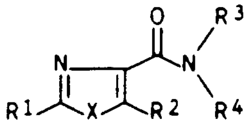

Ia

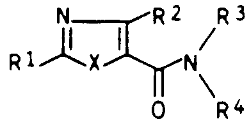

Ib

in denen die Substituenten folgende Bedeutung heben:

X Sauerstoff oder Schwefel;

$R^1$ Wasserstoff; Halogen; $C_1$-$C_6$-Alkyl, welches ein bis fünf Halogenatome und/oder einen oder zwei der folgenden Reste tragen kann: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio oder Cyano;
Benzyl, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
Cyclopropyl oder Cyclopentyl, welche einen bis drei der folgenden Reste tragen können: $C_1$-$C_4$-Alkyl oder Halogen;
$C_2$-$C_6$-Alkenyl, welches ein bis drei der folgenden Reste tragen kann: Halogen, $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
$C_2$-$C_6$-Alkinyl, welches ein bis drei der folgenden Reste tragen kann: Halogen oder $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
$C_1$-$C_4$-Alkoxy; $C_1$-$C_4$-Alkylthio; $C_1$-$C_4$-Halogenalkoxy; $C_1$-$C_4$-Halogenalkylthio;
Phenoxy oder Phenylthio, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$R^2$ formyl, 4,5-Dihydrooxazol-2-yl oder den Rest -$COYR^5$;

Y Sauerstoff oder Schwefel;

$R^5$ Wasserstoff;
$C_1$-$C_6$-Alkyl, welches ein bis fünf Halogenatome oder Hydroxygruppen und/oder einen der folgenden Reste tragen kann: $C_1$-$C_4$-Alkoxy, $C_2$-$C_4$-Alkoxy-$C_1$-$C_4$-alkoxy, Cyano, Trimethylsilyl, $C_1$-$C_3$-Alkylthio, $C_1$-$C_3$-Alkylamino, $C_1$-$C_3$-Dialkylamino, $C_3$-$C_7$-Cycloalkylamino, $C_1$-$C_3$-Alkylsulfinyl, $C_1$-$C_3$-Alkylsulfonyl, Carboxyl, $C_1$-$C_3$-Alkoxycarbonyl, $C_1$-$C_3$-Dialkylaminocarbonyl, $C_1$-$C_3$-Dialkoxyphosphonyl, Alkaniminoxy, Thienyl, Furyl, Tetrahydrofuryl, Phthalimido, Pyridyl, Benzyloxy, Benzoyl, wobei die cyclischen Reste ihrerseits eine bis drei der folgenden Gruppen tragen können: $C_1$-$C_3$-Alkyl, $C_1$-$C_3$-Alkoxy oder Halogen;
Benzyl, das eine bis drei der folgenden Gruppen tragen kann: $C_1$-$C_3$-Alkyl, $C_1$-$C_3$-Alkoxy, $C_1$-$C_3$-Halogenalkyl, Halogen, Nitro und Cyano;
$C_3$-$C_8$-Cycloalkyl;
Phenyl, das eine bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoxycarbonyl, Halogen, Nitro und Cyano;
$C_3$-$C_8$-Alkenyl, $C_5$-$C_6$-Cycloalkenyl oder $C_3$-$C_8$-Alkinyl, wobei diese Reste eine der folgenden Gruppen tragen können: Hydroxy, $C_1$-$C_4$-Alkoxy, Halogen oder einen Phenylring, welcher seinerseits eine bis drei der folgenden Gruppen tragen kann: $C_1$-$C_4$-Alkyl, $C_1$-$C_4$-Alkoxy, $C_1$-$C_4$-Halogenalkyl, Halogen, Nitro und Cyano;
einen fünf- bis sechsgliedrigen heterocyclischen Rest enthaltend ein oder zwei Heteroatome, ausgewählt aus der Gruppe Sauerstoff, Schwefel und Stickstoff oder einen Benzotriazolrest;
Phthalimido; Tetrahydrophthalimido; Succinimido; Maleinimido;
ein Äquivalent eines Kations aus der Gruppe der Alkali- oder Erdalkalimetalle, Mangan, Kupfer, Eisen, Ammonium und substituiertes Ammonium;

einen Rest -N=$CR^6R^7$;

$R^6$, $R^7$ Wasserstoff; $C_1$-$C_4$-Alkyl ; $C_3$-$C_6$-Cycloalkyl; Phenyl oder Furyl oder zusammen eine Methylenkette der Formel -$(CH_2)_m$- mit m = 4 bis 7 Kettengliedern;

$R^3$ Wasserstoff; $C_1$-$C_6$-Alkyl, das einen bis drei der folgenden Substituenten tragen kann: Hydroxy, Halogen, $C_1$-$C_4$-Alkoxy, $C_1$-$C_4$-Alkylthio oder Di-$C_1$-$C_3$-Alkylamino;
$C_3$-$C_8$-Cycloalkyl, welches ein bis drei der folgenden Reste tragen kann: $C_1$-$C_4$-Alkyl, Halogen und $C_1$-$C_4$-Halogenalkyl;

$R^4$ Hydroxy; $C_1$-$C_4$-Alkoxy;
$C_1$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, Halogen, $C_3$-$C_8$-Cycloalkyl oder Phenyl, welches seinerseits ein bis drei der folgenden Reste tragen kann: Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;
$C_3$-$C_8$-Cycloalkyl, das eine bis drei der folgenden Grupen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano;
$C_3$-$C_6$-Alkenyl oder $C_3$-$C_6$-Alkinyl, das ein- bis dreimal durch Halogen und/oder einmal durch Phenyl substituiert sein kann, wobei der Phenylring seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
ein 5- bis 6-gliedriger heterocyclischer Rest enthaltend ein oder zwei Heteroatome, ausgewählt aus der Gruppe Sauerstoff, Schwefel oder Stickstoff, welcher ein bis drei der folgenden Reste tragen kann: $C_1$-$C_4$-Alkyl oder Halogen;
Phenyl, das eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, $C_1$-$C_4$-Alkanoyl, $C_1$-$C_4$-Halogenalkanoyl oder $C_1$-$C_4$-Alkoxycarbonyl;
Naphthyl, das ein- bis dreimal durch $C_1$-$C_4$-Alkyl oder Halogen substituiert sein kann,
oder
$R^3$ und $R^4$ gemeinsam einen Rest der Struktur -$(CH_2)_n$-$Y_p$-$(CH_2)_q$-, wobei n und q 1, 2 oder 3, p 0 oder 1 und Y Sauerstoff, Schwefel oder N-Methyl bedeuten oder den Rest der Formel -$(CH_2)_3$-CO- bilden, sowie deren umweltverträgliche Salze, ausgenommen Verbindungen der Formel Ia, in der $R^1$ für unsubstituiertes Ethyl steht, wenn gleichzeitig X = Schwefel, $R^2$ = COOH, $R^3$ Wasserstoff und $R^4$ eine $C_1$-$C_4$-Dialkylamino-$C_1$-$C_3$-alkylgruppe bedeuten.

Weiterhin betrifft die Erfindung herbizide Mittel, enthaltend die Verbindungen Ia bzw. Ib und Verfahren zur Herstellung dieser Verbindungen.

Gegenstand der Erfindung ist ferner ein Verfahren zur Bekämpfung unerwünschten Pflanzenwuchses, das dadurch gekennzeichnet ist, daß man die unerwünschten Pflanzen und/oder die von unerwünschten Pflanzenwuchs freizuhaltende Fläche mit einer herbizid wirksamen Menge eines Oxazol- bzw. Thiazolcarbonsäureamids der Formeln Ia' bzw. Ib'

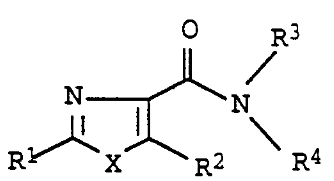

Ia'

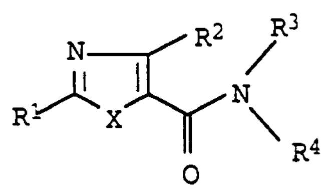

Ib'

in denen die Substituenten die für die Formeln Ia und Ib angegebene Bedeutung haben und $R^1$ ferner bedeutet:

Cyclobutyl, Cyclohexyl, Cycloheptyl und Cyclooctyl, welche jeweils ein bis drei $C_1$-$C_4$-Alkyl oder Halogenreste tragen können; ein 5- bis 6-gliedriger heterocyclischer Rest, enthaltend ein oder zwei Heteroatome, ausgewählt aus der Gruppe Sauerstoff, Schwefel und Stickstoff, wobei der Ring ein oder zwei der folgenden Reste tragen kann: $C_1$-$C_3$-Alkyl, Halogen, $C_1$-$C_3$-Alkoxy oder $C_1$-$C_3$-Alkoxycarbonyl;

Phenyl, welches eine bis drei der folgenden Gruppen tragen kann: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro und Cyano, behandelt.

Oxazol- und Thiazolcarbonsäure bzw. deren Derivate sind bekannt z.B. aus DE-A 22 54 955, Bull. Soc. Chim. Fr., 1969, 2152, sowie DE-A 22 21 647. FR-A 1 516 777 offenbart Thiazolcarbonsäureamide mit einem Phenyl- oder heteroaromatischen Rest in 2-Position als Zwischenprodukte bzw. für therapeutische Anwendungen.

In dieser Schrift werden ferner 2-Ethyl-thiazol-4,5-diamid und die zugrundeliegende 4,5-Dicarbonsäure bzw. der entsprechende Diethylester expressis verbis genannt. Auf Seite 16, Punkt E der allgemeinen Beschreibung wird auf 2-Ethylthiazolcarbonsäureamide hingewiesen, wobei die unsubstituierte Amidgruppe ($CONH_2$) aufgrund der ausgeführten Beispiele als besonders bevorzugt anzusehen ist.

FR-A 2 201 895 beschreibt 2-Pyridyl substituierte Thiazole als Pharmazeutika.

Mögliche Anwendungen der im Stand der Technik offenbarten Substanzen als herbizide Mittel sind nicht beschrieben.

Aufgabe der vorliegenden Erfindung war es, neue herbizid wirksame Verbindungen zu finden und zu synthetisieren.

Demgemäß wurden die eingangs definierten Oxazol- bzw. Thiazolcarbonsäureamide Ia und Ib gefunden.

Außerdem wurden Verfahren zu ihrer Herstellung gefunden und herbizide Mittel, enthaltend Oxazol- bzw. Thiazolcarbonsäureamide Ia und Ib.

Die erfindungsgemäßen Oxazol- bzw. Thiazolcarbonsäureamide Ia und Ib sind auf verschiedenen Wegen herstellbar. Man erhält sie beispielsweise nach den folgenden Verfahren:

1. Verfahren zur Herstellung der Verbindungen Ia und Ib, in denen $R^2$ $CO_2R^5$ und $R^5$ $C_1$-$C_6$-Alkyl bedeutet

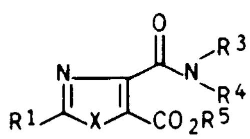

Ia

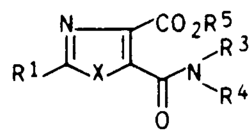

Ib

($R^5$ = $C_1$-$C_6$-Alkyl)

Man erhält diese Oxazol- bzw. Thiazolcarbonsäureamide Ia und Ib dadurch, daß man einen Diester der Formel II in an sich bekannter Weise mit einem Äquivalent einer wäßrigen Base zum Monoester IIIa bzw. IIIb hydrolysiert und IIIa und IIIb danach getrennt oder im Gemisch zunächst in das Halogenid oder eine andere aktivierte Form der Carbonsäure überführt und diese Derivate anschließend mit einem Amin IV amidiert.

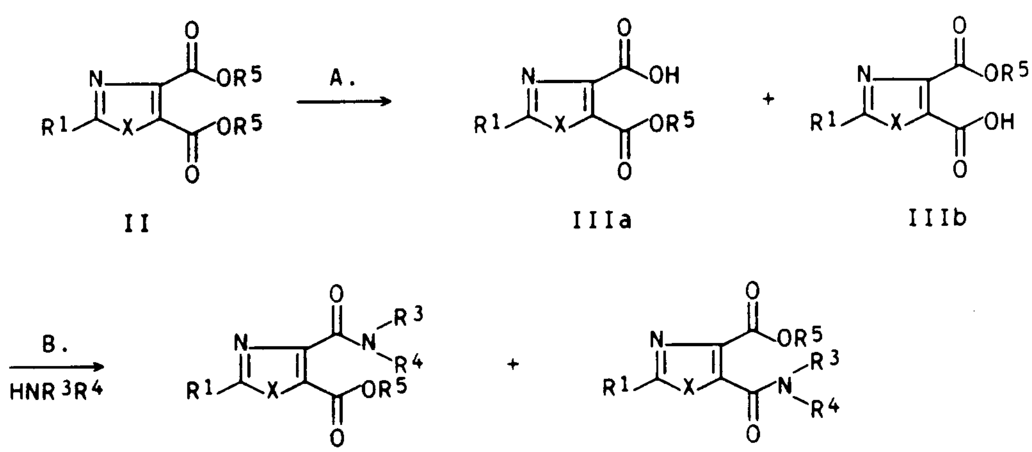


II IIIa IIIb

IV Ia Ib

Die einzelnen Reaktionsschritte A und B dieser Synthesesequenz können wie folgt durchgeführt werden:

Reaktionsschritt A:

Die partielle Verseifung des Diesters II zum Monoester Va und Vb wird üblicherweise bei Temperaturen von -20 bis 60 °C, vorzugsweise -10 bis 30 °C, in einem inerten, mit Wasser mischbaren organischen Lösungsmittel in Gegenwart von 1,0 bis 1,2 mol-äq. einer Base durchgeführt.

Als Basen eignen sich insbesondere Hydroxyde von Alkalimetall-Kationen. Die Base wird im allgemeinen als 5 bis 20 %ige wäßrige Lösung zugesetzt.

Bevorzugte Lösungsmittel für diese Umsetzung sind beispielsweise Dioxan oder der der Esterkomponente in der Formel II entsprechende Alkohol.

Zur Aufarbeitung wird das Reaktionsgemisch üblicherweise angesäuert, wobei sich das gewünschte Produkt als Feststoff oder als öl abscheidet. Die Isolierung erfolgt in üblicher Weise durch Filtration bzw. Extraktion.

Das Gemisch der beiden isomeren Monoester IIIa und IIIb kann durch fraktionierte Kristallisation oder auf chromatographischem Wege getrennt werden oder es kann ohne Trennung weiter umgesetzt werden.

Reaktionsschritt B:

Man erhält die Verbindungen Ia bzw. Ib aus den Monoestern IIIa und IIIb, in dem man IIIa und IIIb zunächst in an sich bekannter Weise in das Halogenid oder eine andere aktivierte Form der Carbonsäurefunktion überführt und diese Derivate anschließend mit einem Amin IV amidiert.

Aktivierte Formen der Carbonsäure sind neben Halogeniden wie insbesondere den Chloriden und den Bromiden beispielsweise auch Imidazolide. Im allgemeinen werden die Halogenide bevorzugt.

Man erhält sie durch Umsetzung der Carbonsäuren IIIa und IIIb mit einem Halogenierungsmittel wie Thionylchlorid, Thionylbromid, Phosphoroxychlorid bzw. -bromid, Phosphortri- und -pentachlorid bzw. -bromid, Phosgen sowie elementarem Chlor und Brom.

Das Halogenierungsmittel wird in 1 bis 5 mol-äq., vorzugsweise 1 bis 2 mol.äq., eingesetzt.

Die Umsetzung verläuft bei Temperaturen von 0 °C bis zum Siedepunkt des Halogenierungsmittels bzw. sofern man in Gegenwart eines inerten organischen Lösungsmittels arbeitet, auch dessen Siedepunkt, vorzugsweise 20 bis 120 °C.

Als Lösungsmittel eignen sich beispielsweise Kohlenwasserstoffe und Halogenkohlenwasserstoffe wie Tetrachlorethan, Methylenchlorid, Chloroform, Dichlorethan, Chlorbenzol, 1,2-Dichlorbenzol, Benzol, Toluol und Xylol.

Üblicherweise werden die aktivierten Carbonsäurederivate isoliert, beispielsweise durch Abdestillieren des Halogenierungsmittels und sofern vorhanden des Lösungsmittels und erst anschließend mit den Aminen IV umgesetzt.

In diesem Fall wird die Amidierung bei Temperaturen von -20 bis 100 °C, vorzugsweise -10 bis 20 °C in einem inerten aprotisch polaren organischen Lösungsmittel durchgeführt.

Für diese Umsetzung eignen sich insbesondere Halogenkohlenwasserstoffe wie Dichlormethan und Ether wie Diethylether und tert.-Butylmethylether als Lösungsmittel.

Da bei der Amidierung von Säurehalogeniden Halogenwasserstoff gebildet wird, empfiehlt es sich, das Amin IV in 2 bis 5 mol.-äq. überschuß, vorzugsweise 2 bis 3 mol.-äq. zuzusetzen. Sofern das Amin in äquimolaren Mengen (1 bis 1,2 mol-äq.) eingesetzt wird, sollte zum Binden des Halogenwasserstoffs eine Base, insbesondere ein tertiäres Amin wie Triethylamin oder Pyridin zugegeben werden.

Sofern man von einem Gemisch der Monoester IIIa und IIIb ausgeht erhält man bei der Umsetzung ein Gemisch aus den isomeren Carbonsäureamiden Ia und Ib. Dieses Gemisch kann auf herkömmliche Weise, beispielsweise durch fraktionierte Kristallisation oder Chromatographie in die Einzelkomponenten aufgetrennt werden.

Die für diese Synthesesequenz benötigten Edukte II sind bekannt (Bull. Soc. Chim. Fr. 1974, 2079) oder nach bekannten Methoden (Bull. Soc. Chim. Fr. 1969, 1762; J. Chem. Soc., 1953, 93) zugänglich.

2. Verfahren zur Herstellung der Verbindungen Ia und Ib, in denen X Schwefel und $R^2$ $CO_2H$ bedeutet

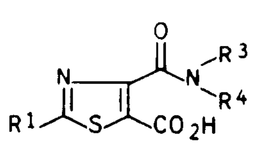

Ia

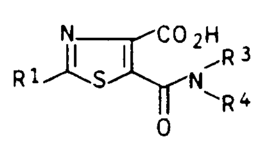

Ib

Man erhält diese Thiazolcarbonsäureamide Ia und Ib besonders vorteilhaft, indem man ein Dicarbonsäureanhydrid der Formel V in an sich bekannter Weise mit einem Amin der Formel IV zu den Isomeren Ia und Ib umsetzt und anschließend das Gemisch in die Isomeren auftrennt.

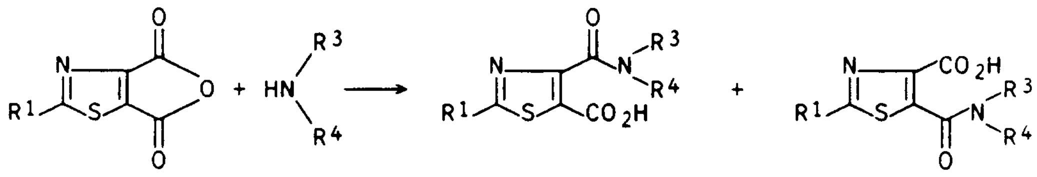

V IV Ia Ib

Die Umsetzung wird üblicherweise bei Temperaturen von -10 bis 150 °C, vorzugsweise 20 bis 120 °C in einem inerten aprotisch polaren organischen Lösungsmittel durchgeführt.

Insbesondere kommen als Lösungsmittel Halogenkohlenwasserstoffe, z.B. Tetrachlorethan, Methylenchlorid, Chloroform, Dichlorethan, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel. z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromate, z.B. Benzol, Toluol, Xylol, Pyridin und Chinolin; Ketone, z.B. Aceton, Methylethylketon oder entsprechende Gemische zur Anwendung.

Das Amin IV wird im allgemeinen in äuqimolaren Mengen oder im überschuß, vorzugsweise in Mengen von 1,0 bis 5,0 mol-äq. bezogen auf V eingesetzt.

Die für dieses Verfahren benötigten Dicarbonsäureanhydride sind bekannt oder können nach bekannten Methoden hergestellt werden (Bull. Soc. Chim. Fr. 1969, 1762; CS-A-195 369; CS-A-195 370).

3. Verfahren zur Herstellung der Verbindungen Ia und Ib, in denen $R^1$ nicht Halogen und $R^2$ Carboxyl oder Formyl bedeutet

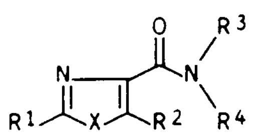

Ia

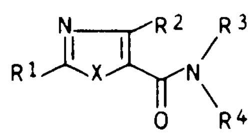

Ib

($R^1 \neq$ Hal; $R^2$ = $CO_2H$, CHO)

Man erhält diese isomeren Oxazol- bzw. Thiazolcarbonsäureamide, indem man eine Carbonsäure IIIc bzw. IIId gemäß den unter 1 B geschilderten Bedingungen aktiviert und amidiert und die so erhaltenen Amide VIa und VIb anschließend in an sich bekannter Weise in Gegenwart eines Carboxylierungs- oder Formylierungsreagens' umsetzt.

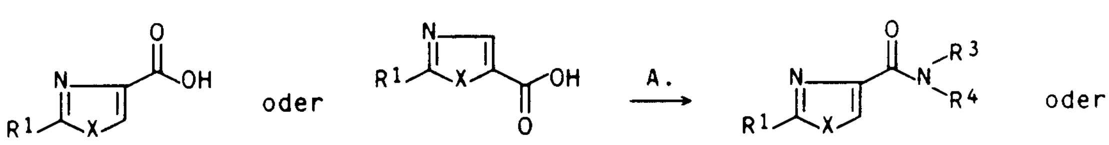


IIIc IIId VIa

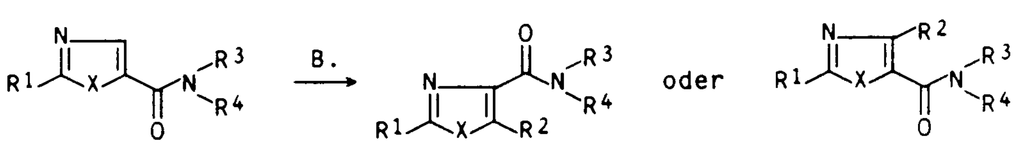


VIb Ia Ib

Der Reaktionsschritt A. dieser Synthesesequenz wird im allgemeinen und im besonderen entsprechend den im Verfahren 1 unter Punkt B beschriebenen Bedingungen durchgeführt.

Reaktionsschritt B.

Die Carboxylierung bzw. Formylierung der Oxazol- bzw. Thiazolcarbonsäureamide VIa bzw. VIb erfolgt in der Regel bei Temperaturen von 0 bis -100 °C, vorzugsweise -50 bis -80 °C in einem aprotisch polaren inerten organischen Lösungsmittel in Gegenwart einer Base unter Ausschluß von Feuchtigkeit.

Bevorzugtes Carboxylierungsreagens ist gasförmiges oder festes Kohlendioxid, als Formylierungsreagens dient insbesondere Dimethylformamid und N-Formylmorpholin.

Geeignete Lösungsmittel sind insbesondere Ether, z.B. Diethylether, Methyl-tert.-butylether, Dimethoxyethan, Diethylenglykoldimethylether, Tetrahydrofuran und Dioxan.

Als Basen finden bevorzugt Organometallverbindungen Methyllithium, n-Butyllithium, s-Butyllithium, t-Butyllithium oder Phenyllithium Verwendung.

Die Umsetzung wird üblicherweise so durchgeführt, daß zunächst eine Lösung des Oxazol- bzw. Thiazolcarbonsäureamids VIa bzw. VIb mit bis 3 mol-äq der gelösten Base versetzt wird, wobei ein am Heterocyclus metalliertes Derivat entsteht, welches bei der anschließenden Zugabe des elektrophilen Carboxylierungs- bzw. Formylierungsreagens' zum gewünschten Produkt Ia bzw. Ib abreagiert.

Sofern $R^3$ Wasserstoff bedeutet werden entsprechend mehr mol-äq. der Base benötigt, da in diesem Fall zunächst der Amid-Stickstoff deprotoniert wird. Vorzugsweise verwendet man daher bei der Umetzung von Carbonsäureamiden VIa bzw. VIb, in denen $R^3$ Wasserstoff bedeutet 2 bis 2,5 mol-äq der Base.

Verbindungen VIa bzw. VIb, in denen $R^1$ Wasserstoff bedeutet werden bei der Umsetzung mit der Base zunächst in 2-Position des Heterocyclus' metalliert.

Um in diesem Fall den Carboxyl- bzw. Formylrest in Nachbarstellung zur Amidgruppe einzuführen ist es notwendig, von Oxazol- bzw. Thiazolcarbonsäureamiden VIa bis VIb auszugehen, in denen $R^3$ Wasserstoff bedeutet.

Oxazol- bzw. Thiazolcarbonsäureamide Ia bzw. Ib,in denen $R^1$ Wasserstoff und $R^3$ nicht Wasserstoff bedeutet,erhält man aus den nach dem vorstehenden Verfahren zugänglichen Verbindungen, in denen $R^1$ und $R^3$ Wasserstoff bedeutet, in an sich bekannter Weise durch nachträgliche Alkylierung oder Cycloalkylierung.

Die für das vorstehende Verfahren benötigten Carbonsäuren IIIc und IIId sind literaturbekannt Beilstein, (Band 27, 1.-5. Erg.Werte) oder sie können nach bekannten Methoden, beispielsweise durch Oxidation der entsprechenden Alkohole oder Aldehyde oder durch Hydrolyse der entsprechenden Nitrile hergestellt werden (J.V. Metzger in "The Chemistry of Heterocyclic Compounds, Vol. 34, Part 1, Thiazol and its Derivatives", Arnold Weissberger and E.D. Ward C. Taylor (Editors), John Wiley and Sons, S. 519 ff, I.J. Turchi in "The Chemistry of Heterocyclic Compounds, Vol. 45, Oxazoles", Arnold Weissberger and E.D. Ward, C. Taylor (Editors), John Wiley and Sons).

4. Verfahren zur Herstellung der Verbindungen Ia und Ib, in denen $R^2$ Carboxyl bedeutet.

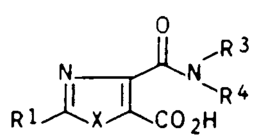

Ia

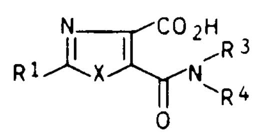

Ib

Man erhält diese Oxazol- bzw. Thiazolcarbonsäureamide Ia und Ib beispielsweise dadurch, daß man ein entsprechendes Carbonsäureamid Ia bzw. Ib, in dem $R^2$ für $CO_2R^5$ und $R^5$ für $C_1$-$C_6$-Alkyl steht in an sich bekannter Weise mit einem Äquivalent einer wäßrigen Base hydrolysiert. Die Umsetzung ist im folgenden Schema lediglich für die Carbonsäureamide Ia gezeigt. Sofern man von den entsprechenden Carbonsäureamiden Ib ausgeht verläuft sie analog.

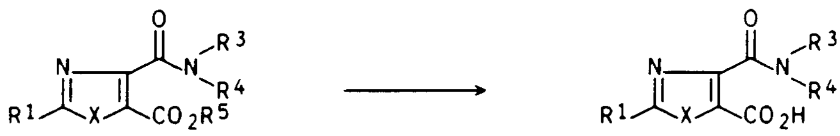

Ia ($R^5$ = $C_1$-$C_6$-Alkyl) Ia

Diese Synthese wird im allgemeinen und im besonderen entsprechend den im Verfahren 1 unter Punkt A beschriebenen Bedingungen durchgeführt.

5. Verfahren zur Herstellung der Verbindungen Ia und Ib, in denen $R^2$ COY$R^5$ bedeutet:

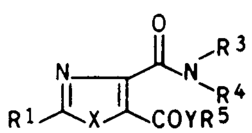

Ia

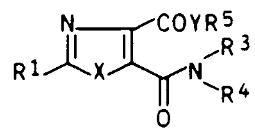

Ib

Man erhält diese Carbonsäureamide Ia und Ib, in dem man eine entsprechende Carbonsäure Ia bzw. Ib ($R^2$ = $CO_2H$) aktiviert und anschließend in an sich bekannter Weise mit einer Verbindung VII umsetzt.

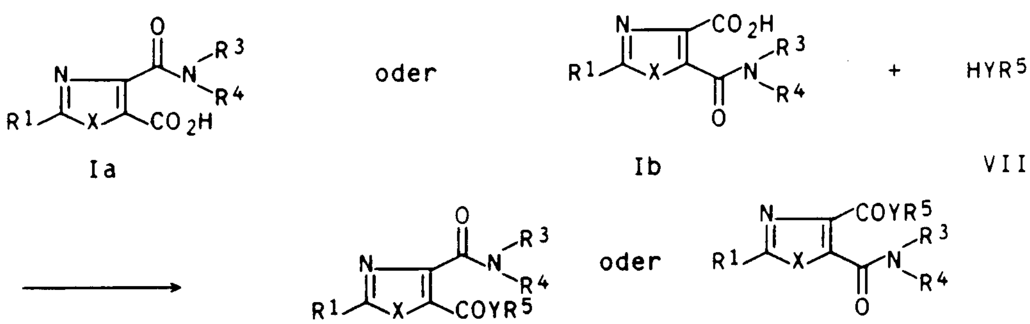

Ia oder Ib + HYR5 VII

Ia oder Ib

Die umsetzung kann bei Temperaturen von -20 °C bis zur Rückflußtemperatur des Lösungsmittels bzw. -gemisches, vorzugsweise bei 0 bis 60 °C durchgeführt werden.

Zweckmäßigerweise verwendet man für diese Umsetzungen Lösungsmittel wie Halogenkohlenwasserstoffe, z.B. Tetrachlorethan, Methylenchlorid, Chloroform, Dichlorethan, Chlorbenzol und 1,2-Dichlorbenzol; Ether z.B. Diethylether, Methyl-tert.-butylether, Dimethoxiethan, Diethylenglykoldimethylether, Tetrahydrofuran und Dioxan; Aromaten z.B. Benzol, Toluol oder Xylol; oder entsprechende Gemische.

Als wasserentziehende Mittel kommen Dicyclohexylcarbodiimid oder Propanphosphonsäureanhydrid in Betracht.

Die molaren Verhältnisse, in denen die benötigten Ausgangsverbindungen miteinander umgesetzt werden, betragen im allgemeinen 0,5:1 bis 2:1 für das Verhältnis von Carbonsäure IVa zu Alkohol oder Thiol und 1:1 bis 1:3 für das Verhältnis von Carbonsäure IVa zu wasserentziehendem Mittel.

Die Konzentration der Edukte im Lösungsmittel beträgt im allgemeinen 0,1 bis 5 mol/l, bevorzugt 0,2 bis 2 mol/l.

Besonders bevorzugt arbeitet man in Ethern wie Diethylether, Tetrahydrofuran oder Dioxan; mit Propanphosphonsäureanhydrid als wasserentziehendem Mittel bei 20 bis 60 °C.

6. Verfahren zur Herstellung der Verbindungen Ia und Ib, in der $R^2$ eine 4,5-Dihydro-oxazol-2-yl-gruppe bedeutet

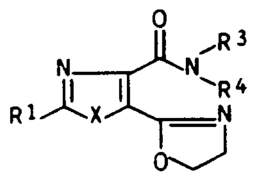

Ia

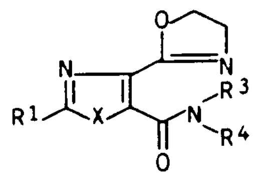

Ib

Man erhält diese Verbindungen dadurch, daß man ein entsprechendes Carbonsäurederivat Ia bzw. Ib, in dem $R^2$ eine Gruppe $CO_2R'$ oder COOH und R' $C_1$-$C_4$-Alkyl bedeutet, in an sich bekannter Weise mit einem Aminoalkohol der Formel VIII umsetzt.

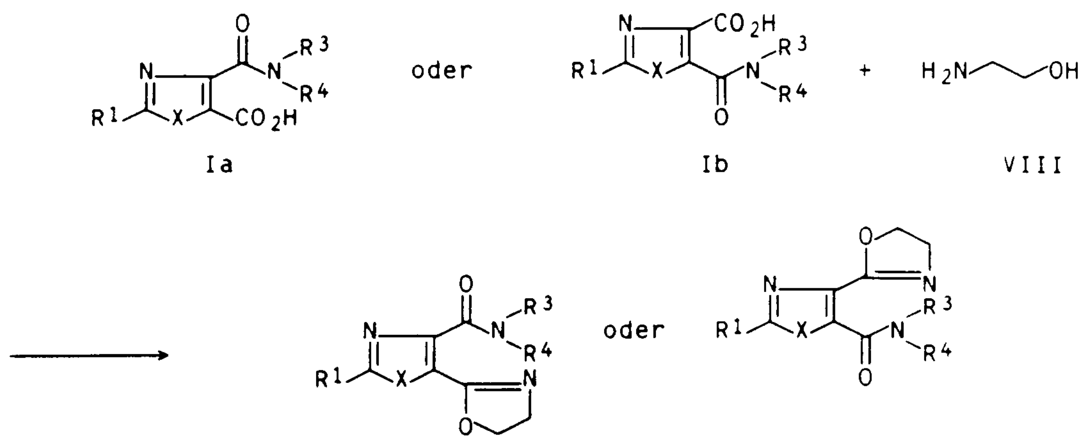

Ia oder Ib + VIII

Ia oder Ib

Die Reaktion wird so durchgeführt, daß man die Verbindungen bei 0 bis 180 °C, vorzugsweise bei Rückflußtemperatur des verwendeten Gemisches mit einem Aminoalkohol VIII, gegebenenfalls in Gegenwart eines inerten Lösungsmittels umsetzt. Ester oder Carbonsäure Ia bzw. Ib und Aminoalkohol VIII werden dabei im Verhältnis 1:1 bis 1:2,5, vorzugsweise 1:1 bis 1:1,5 eingesetzt.

Als Lösungsmittel verwendet man zweckmäßigerweise Halogenkohlenwasserstoffe wie Chlorbenzol und 1,2-Dichlorbenzol, Ether, z.B. Methyl-tert.-butylether, 1,2-Dimethoxyethan, Diethylenglykol-dimethylether, Tetrahydrofuran und Dioxan; Alkohole wie Methanol, Ethanol, Propanol oder Ethylenglykol, dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on oder Aromaten, z.B. Benzol, Toluol und Xylol. Die Konzentration der Edukte im Lösungsmittel beträgt im allgemeinen 0,1 bis 5,0 mol/l, bevorzugt 0,2 bis 2,0 mol/l.

Die Umsetzung ist im allgemeinen nach 14 Stunden beendet; die Carbonsäureamide Ia und Ib werden dann gegebenenfalls durch Zugabe von Wasser ausgefällt, abgesaugt oder mit einem organischen Lösungsmittel extrahiert und mit üblichen Standardmethoden wie Umkristallisation oder Chromatographie gereinigt.

Man erhält die Verbindungen der Formel VIa, in denen $R^1$ einen Alkohol oder Thiolrest $-ZR^8$ bedeutet, in an sich bekannter Weise (Helv. Chim. Acta, 37, 2059 (1954)) durch Umsetzung eines 2-Halogen-thiazol-4-carbonsäureamids VIa (DE 22 41 035) in einem inerten organischen Lösungsmittel in Gegenwart einer Base mit einem Alkohol oder Thiol.

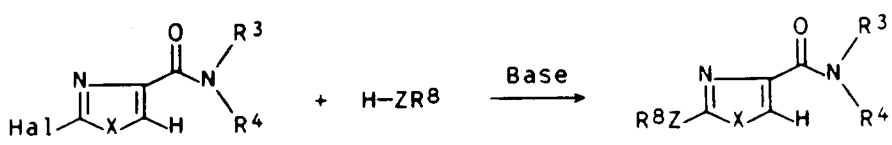


VIa ($R^1$ = Hal) VIa'

Hal in Formel VIa bedeutet dabei ein Halogenatom wie Fluor, Chlor, Brom und Iod; insbesondere eignen sich Verbindungen VIa, in denen Hal, Chlor oder Brom bedeutet.

$R^1$ in Formel VIa' bedeutet $C_1$-$C_4$-Alkoxy, $C_1$-$C_4$-Alkylthio, die bis zu dreimal mit Halogen substituiert sein können, insbesondere Methoxy, Ethoxy, 1-Methyl-ethoxy, 1,1-Dimethylethoxy, Trifluormethoxy, Methylthio, Ethylthio, Difluormethylthio; oder Phenoxy oder Phenylthio, die bis zu dreimal durch $C_1$-$C_4$-Alkyl, $C_1$-$C_4$-Halogenalkyl, $C_1$-$C_4$-Alkylthio, $C_1$-$C_4$-Halogenalkylthio, Halogen, Cyano oder Nitro substituiert sein können, insbesondere 2,4-Dichlorphenoxy, 2,4-Difluorphenoxy, 2,4,6-Trifluorphenoxy, p-Trifluormethylphenoxy, 2-Chlor-4-Trifluorphenoxy, 3-Cyanophenoxy, 4-Cyano-2-methoxyphenoxy, 4-Nitrophenoxy, 2-Fluorthiophenyl, 4-Trifluormethylthiophenyl, 3-Cyanothiophenyl.

Zweckmäßigerweise verwendet man für diese Umsetzungen Lösungsmittel wie Halogenkohlenwasserstoffe, z.B. Tetrachlorethan, Methylenchlorid, Chloroform, Dichlorethan, Chlorbenzol und 1,2-Dichlorbenzol; Ether, z.B. Diethylether, Methyl-tert.-butylether, Dimethoxiethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon; Alkohole, z.B. Methanol, Ethanol, iso-Propanol und tert.-Butanol oder entsprechende Gemische.

Die Umsetzung kann bei Temperaturen von -100 °C bis zur Rückflußtemperatur des jeweiligen Lösungsmittels bzw. -gemisches, vorzugsweise bei -60 °C bis 150 °C, durchgeführt werden.

Als Basen dienen Hydride und Alkoxide von Alkali- und Erdalkalimetallkationen, insbesondere NaH, KH, $CaH_2$, LiH und KO-t.-Bu. Mitunter ist es auch nützlich Kombinationen der oben angeführten Basen zu verwenden.

Die molaren Verhältnisse, in denen die benötigten Ausgangsverbindungen miteinander umgesetzt werden, betragen im allgemeinen 3:1 bis 1:1 für das Verhältnis von Alkohol oder Thiol zu 2-Halogen-thiazol-4-carbonsäureamid VIa und 1:1 bis 1:3 für das Verhältnis von Alkohol oder Thiol zur wirksamen Base.

Die Konzentration der Edukte im Lösungsmittel beträgt im allgemeinen 0,1 bis 5 mol/l, bevorzugt 0,2 bis 2 mol/l.

Besonders bevorzugt arbeitet man in aprotisch dipolaren Solventien wie Acetonitril, Dimethylformamid, Dimethylsulfoxid, N-Methylpyrrolidon, 1,3-Dimethyltetrahydro-2(1H)-pyrimidinon, 1,3-Dimethylimidazolidin-2-on oder Ethern wie 1,2-Dimethoxiethan, Diethylenglykoldimethylether, Tetrahydrofuran oder Dioxan bei Temperaturen zwischen 50 °C und 150 °C unter Verwendung von NaH oder KO-t.-Ba als Basen.

Die für die Umsetzung benötigten 2-Halogen-thiazol-4-carbonsäureamide der Formel IIIa können nach literaturbekannten Methoden aus den entsprechenden Carbonsäurehalogeniden durch Umsetzung mit Aminen gewonnen werden (DE-A 22 41 035).

Die zum Einsatz kommenden Alkohole oder Thiole sind in vielen Fällen kommerziell erhältlich oder können in an sich bekannter Weise hergestellt werden.

Desweiteren erhält man die Verbindungen der Formel VIb in an sich bekannter Weise (Helv. Chim. Acta, 37, 2059 (1954)) durch Umsetzung eines 2-Halogen-thiazol-5-carbonsäureamids IIIb in einem inerten organischen Lösungsmittel in Gegenwart einer Base mit einem Alkohol oder Thiol gemäß Schema 2:

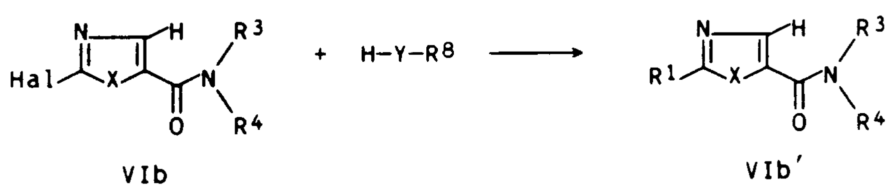

Hal in Formel VIb bedeutet dabei ein Halogenatom wie Fluor, Chlor, Brom und Iod; insbesondere eignen sich Verbindungen VIb, in denen Hal, Chlor oder Brom bedeutet.

$R^1$ in Formel VIb' bedeutet $C_1$-$C_4$-Alkoxy, $C_1$-$C_4$-Alkylthio, die bis zu dreimal mit Halogen substituiert sein können;

insbesondere Methoxy, Ethoxy, 1-Methyl-ethoxy, 1,1-Dimethylethoxy, Trifluormethoxy, Methylthio, Ethylthio, Difluormethylthio; oder Phenoxy oder Phenylthio, die bis zu dreimal durch $C_1$-$C_4$-Alkyl, $C_1$-$C_4$-Halogenalkyl, $C_1$-$C_4$-Alkylthio, $C_1$-$C_4$-Halogenalkylthio, Halogen, Cyano oder Nitro substituiert sein können; insbesondere 2,4-Dichlorphenoxy, 2,4-Difluorphenoxy, 2,4,6-Trifluorphenoxy, p-Trifluormethylphenoxy, 2-Chlor-4-Trifluorphenoxy, 3-Cyanophenoxy, 4-Cyano-2-methoxyphenoxy, 4-Nitrophenoxy, 2-Fluorthiophenyl, 4-Trifluormethylthiophenyl, 3-Cyanothiophenyl.

Zweckmäßigerweise verwendet man für diese Umsetzungen Lösungsmittel wie Halogenkohlenwasserstoffe, z.B. Tetrachlorethan, Methylenchlorid, Chloroform, Dichlorethan, Chlorbenzol und 1,2-Dichlorbenzol; Ether, z.B. Diethylether, Methyl-tert.-butylether, Dimethoxiethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon; Alkohole, z.B. Methanol, Ethanol, iso-Propanol und tert.-Butanol oder entsprechende Gemische.

Die Umsetzung kann bei Temperaturen von -100 °C bis zur Rückflußtemperatur des jeweiligen Lösungsmittels bzw. -gemisches, vorzugsweise bei -60 °C bis 150 °C, durchgeführt werden.

Als Basen dienen Hydride und Alkoxide von Alkali- und Erdalkalimetallkationen, insbesondere NaH, KH, $CaH_2$, LiH und KO-t.-Bu. Mitunter ist es auch nützlich Kombinationen der oben angeführten Basen zu verwenden.

Die molaren Verhältnisse, in denen die benötigten Ausgangsverbindungen miteinander umgesetzt werden, betragen im allgemeinen 3:1 bis 1:1 für das Verhältnis von Alkohol oder Thiol zu 2-Halogen-thiazol-4-carbonsäureamid VIb und 1:1 bis 1:3 für das Verhältnis von Alkohol oder Thiol zur wirksamen Base.

Die Konzentration der Edukte im Lösungsmittel berägt im allgemeinen 0,1 bis 5 mol/l, bevorzugt 0,2 bis 2 mol/l.

Besonders bevorzugt arbeitet man in aprotisch dipolaren Solventien wie Acetonitril, Dimethylformamid, Dimethylsulfoxid, N-Methylpyrrolidon, 1,3-Dimethyltetrahydro-2(1H)-pyrimidinon, 1,3-Dimethylimidazolidin-2-on oder Ethern wie 1,2-Dimethoxiethan, Diethylenglykoldimethylether, Tetrahydrofuran oder Dioxan bei Temperaturen zwischen 50 °C und 150 °C unter Verwendung von NaH oder KO-t.-Butylat als Basen.

Die für die Umsetzung benötigten 2-Halogen-thiazol-4-carbonsäureamide der Formel VIb können nach literaturbekannten Methoden aus den entsprechenden Carbonsäurehalogeniden durch Umsetzung mit Aminen gewonnen werden (US-A-4 001 421).

Verbindungen der Formel IVb können gewonnen werden, indem man Dicarbonsäureester der Formel XI in an sich bekannter Weise mit Aminen umsetzt und die resultierenden Amide IXb gemäß Schema G verseift:

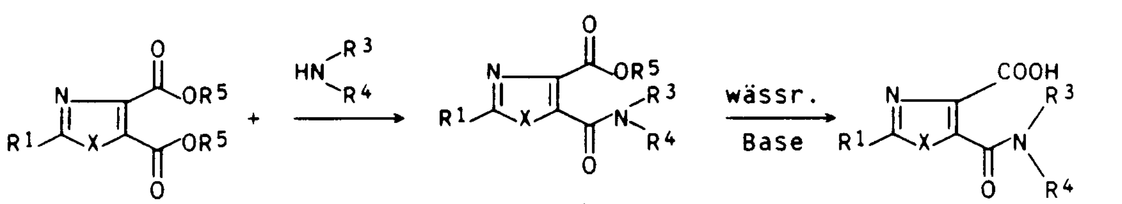


II

Ib
($R^2$ = $CO_2R^5$)

Ib ($R^2$ = $CO_2H$)

Zweckmäßigerweise geht man dabei so vor, daß man den Diester II in einem inerten organischen Lösungsmittel löst und mit einem Amin umsetzt.

Als Lösungsmittel verwendet man für diese Umsetzungen Ether, z.B. Diethylether, Methyl-tert.-butylether, Dimethoxyethan, Diethylenglykoldimethylether, Tetrahydrofuran und Dioxan; Aromaten, z.B. Benzol, Toluol, Xylol oder Mesitylen; Alkohole, z.B. Methanol, Ethanol, iso-Propanol und tert.-Butanol oder entsprechende Gemische.

Die Umsetzung kann bei Temperaturen von -100 °C bis zur Rückflußtemperatur des jeweiligen Lösungsmittels bzw. -gemisches, vorzugsweise bei -60 °C bis 150 °C, durchgeführt werden.

Das molare Verhältnis, in dem Diester II und Amin eingesetzt werden, beträgt 1:1 bis 1:2, vorzugsweise 1:1 bis 1:1,2.

Die Konzentration der Edukte im Lösungsmittel beträgt im allgemeinen 0,1 bis 5 mol/l, bevorzugt 0,2 bis 2,0 mol/l.

Besonders bevorzugt arbeitet man in Alkoholen wie Ethanol in Gegenwart von einem Äquivalent Amin bei 50 bis 100 °C. Die für die Umsetzung benötigten Diester XI, sind literaturbekannt oder können in Anlehnung an beschriebene Methoden hergestellt werden (Bull. Soc. Chim. Fr., 1969, 1762; J. Chem. Soc., 1953, 93).

Neben den vorstehend geschilderten Verfahren 1-7 zur Herstellung der Verbindungen Ia, Ib und Ic gibt es weitere Synthesemöglichkeiten, die den folgenden Literaturstellen zu entnehmen sind:

Beilstein, Hauptwerk sowie 1.-5. Erg.Werk, Band 27; R.W. Wiley, The Chemistry of Heterocyclic Compounds, Five- and Six-Membered Compounds with Nitrogen and Oxygen, Interscience Pubishers, New

York, London (1962), Heterocyclic Chemistry, Vol. 6, Five-membered Rings with Two or More Oxygen, Sulfur or Nitrogen Atoms, Pergamon Press, 1984, J. March, Advanced Organic Chemistry, Third Adition, John Wiley and Sons, 1985, Houben-Weyl, Methoden der organischen Chemie, 4. Auflage, Thieme Verlag, Bände IV, VI, VII, VIII, X.

Im Hinblick auf die bestimmungsgemäße Verwendung der Verbindungen Ia' und Ib' kommen als Substituenten bevorzugt folgende Reste in Betracht:

X Sauerstoff oder Schwefel

$R^1$ Wasserstoff;

Halogen wie Fluor, Chlor, Brom und Iod, insbesondere Fluor und Chlor;

$C_1$-$C_6$-Alkyl, wie Methyl, Ethyl, n-Propyl, iso-Propyl, n-Butyl, sek.-Butyl, iso-Butyl und tert.-Butyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethyl, 1,2-Dimethylbutyl, 1,3-Dimethylbutyl, 2 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl und iso-Propyl, welches ein bis fünf Halogenatome, insbesondere Fluor- und/ oder Chloratome oder einen oder zwei der folgenden Reste tragen kann: Cycloalkyl wie Cyclopropyl, Cyclobutyl, Cyclopentyl und Cyclohexyl, insbesondere Cyclopropyl; Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere Methoxy, Ethoxy, 1-Methylethoxy und 1,1-Dimethylethoxy; Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy; Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio; Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Difluormethylthio und Pentafluorethylthio oder Cyano;

Benzyl, welches ein bis drei der folgenden Reste tragen kann: Alkyl wie vorstehend genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor; Cyano oder Nitro;

Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl, welches ein bis drei der folgenden Reste tragen kann: Alkyl wie vorstehend genannt, insbesondere Methyl oder Halogen wie vorstehend genannt, insbesondere Chlor und Fluor;

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-pentenyl, 4-Pentenyl, 1-Methyl-1-butenyl, 2-Methyl-1-butenyl, 3-Methyl-2-butenyl 1-Methyl-2-butenyl, 2-Methyl-2-butenyl, 1-Methyl-3-butenyl, 2-Methyl-3-butenyl, 3-Methyl-3-butenyl, 1,1-Dimethyl-2-propenyl, 1,1-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Mehyl-3-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-2-propenyl, 1-Ethyl-1-methyl-2-propenyl, 1-Ethyl-2-methyl-1-propenyl und 1-Ethyl-2-methyl-2-propenyl, insbesondere Allyl, welches ein bis drei der folgenden Reste tragen kann: Halogen wie oben genannt, insbesondere Fluor und Chlor; Alkoxy wie obengenannt, insbesondere Methoxy und Ethoxy, und/oder ein Phenyl, das seinerseits

eine bis drei der folgenden Gruppen tragen kann: Alkyl wie vorstehend genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

Alkinyl wie Ethinyl, 1-Propinyl, Propargyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-4-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere Propargyl, welches ein bis drei der folgenden Reste tragen kann: Halogen wie oben genannt, insbesondere Iod; Alkoxy wie oben genannt, insbesondere Methoxy und Ethoxy, und/oder ein Phenyl, das seinerseits eine bis drei der folgenden Gruppen tragen kann: Alkyl wie vorstehend genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

$C_1$-$C_4$-Alkoxy wie obenstehend genannt, insbesondere Methoxy und Ethoxy;

$C_1$-$C_4$-Halogenalkoxy wie obenstehend genannt, insbesondere Trifluormethoxy, Pentafluorethoxy und Trichlormethoxy;

$C_1$-$C_4$-Alkylthio wie obenstehend genannt, insbesondere Methylthio und Ethylthio;

$C_1$-$C_4$-Halogenalkylthio wie obenstehend genannt, insbesondere Difluormethylthio, Pentafluorethylthio und Trifluormethylthio;

Phenoxy oder Phenylthio, wobei diese Reste ein bis drei der folgenden Gruppen tragen können: Alkyl wie vorstehend genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

ein 5- bis 6-gliedriger heterocyclischer Rest enthaltend ein oder zwei Heteroatome, ausgewählt aus der Gruppe Sauerstoff, Schwefel und Stickstoff wie 2-Tetrahydrofuranyl, 3-Tetrahydrofuranyl, 4-Tetrahydropyranyl, 2-Tetrahydropyranyl, 3-Tetrahydropyranyl, 3-Furanyl, 2-Thienyl, 3-Thienyl, 2-Furanyl, 3-Tetrahydrothienyl, 2-Tetrahydropyranyl, 5-Isoxazolyl, 3-Isoxazolyl, 4-Isoxazolyl, 5-Isothiazolyl, 4-Isothiazolyl, 3-Isothiazolyl, 2-Oxazolyl, 4-Thiazolyl, 4-Oxazolyl, 2-Thiazolyl, 5-Oxazolyl, 5-Thiazolyl, 2-Imidazolyl, 4-Imidazolyl, 5-Imidazolyl, 3-Pyrrolyl, 2-Pyrrolyl, 3-Pyrazolyl, 4-Pyrazolyl, 5-Pyrazolyl, 4-Pyridyl, 3-Pyridyl und 2-Pyridyl, wobei dieser Ring ein oder zwei der folgenden Reste tragen kann: Alkyl wie oben genannt, insbesondere Methyl; Halogen wie oben genannt, insbesondere Fluor und Chlor; Alkoxy wie oben genannt, insbesondere Methoxy und Etoxy oder Alkoxycarbonyl wie Methoxycarbonyl und Ethoxycarbonyl, insbesondere Methoxycarbonyl;

Phenyl, welches eine bis drei der folgenden Gruppen tragen kann: Alkyl wie bei $R^1$ genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

$R^2$ Formyl, 4,5-Dihydroxazol-2-yl oder den Rest $-COYR^5$
und

Y Sauerstoff oder Schwefel;

$R^5$ Wasserstoff;

Alkyl, wie unter $R^1$ genannt, insbesondere Methyl, Ethyl, n-Propyl, iso-Propyl und n-Hexyl, welches ein bis fünf Halogenatome wie unter $R^1$ genannt, insbesondere Fluor und Chlor oder Hydroxygruppen und/oder einen der folgenden Reste tragen kann: Alkoxy wie unter $R^1$ genannt, insbesondere Methoxy und Ethoxy; Alkoxy-alkoxy wie Methoxy-ethoxy, Ethoxy-ethoxy, Propoxyethoxy, insbesondere Methoxy-ethoxy; Cyano; Trimethylsilyl; Alkylthio wie unter $R^1$ genannt, insbesondere Methylthio und Ethylthio; Alkylamino wie Methylamino, Ethylamino, Propylamino, iso-Propylamino, insbesondere Methylamino und Ethylamino; Dialkylamino wie Dimethylamino, Diethylamino, Dipropylamino, Diisopropylamino, Methylethylamino, insbesondere Dimethylamino und Methylethylamino; Cycloalkylamino wie Cyclopropylamino, Cyclobutylamino, Cyclopentylamino, Cyclohexylamino und Cycloheptylamino, insbesondere Cyclopropylamino; Alkylsulfinyl wie Methylsulfinyl, Ethylsulfinyl, Propylsulfinyl, iso-Propylsulfinyl, insbesondere Methylsulfinyl und Ethylsulfinyl; Alkylsulfonyl wie Methylsulfonyl, Ethylsulfonyl, Propylsulfonyl, iso-Propylsulfonyl, insbesondere Methylsulfonyl und Ethylsulfonyl; Carboxyl; Alkoxycarbonyl wie unter $R^1$ genannt, insbesondere Methoxycarbonyl; Dialkylaminocarbonyl wie Dimethylaminocarbonyl, Diethylaminocarbonyl, Dipropylaminocarbonyl, Diisopropylaminocarbonyl, Dicyclopropylaminocarbonyl, Methylethylaminocarbonyl, insbesondere Dimethylaminocarbonyl und Diethylaminocarbonyl; Dialkoxyphosphonyl wie Dimethoxyphosphonyl, Diethoxyphosphonyl, Dipropoxyphosphonyl, Diisopropoxyphosphonyl, insbesondere Dimethoxyphosphonyl und Diethoxyphosphonyl; Alkaniminoxy wie insbesondere 2-Propaniminoxy; Thienyl, Furanyl, Tetrahydrofuranyl, Phthalimido, Pyridyl, Benzyloxy; Benzoyl, wobei die cyclischen Reste ihrerseits eine bis drei der folgenden Gruppen tragen können: Alkyl wie unter $R^1$ genannt, insbesondere Methyl und Ethyl; Alkoxy wie unter $R^1$ genannt, insbesondere Methoxy und Ethoxy, oder Halogen wie unter $R^1$ genannt, insbesondere Fluor und Chlor;

Benzyl, das eine bis drei der folgenden Gruppen tragen kann: Alkyl wie unter $R^1$ genannt, insbesondere Methyl und Ethyl; Alkoxy wie unter $R^1$ genannt, insbesondere Methoxy und Ethoxy; Halogenalkyl wie unter $R^1$ genannt, insbesondere Trifluormethyl; Halogen wie unter $R^1$ genannt, insbesondere Fluor und Chlor, Nitro und Cyano;

$C_3$-$C_8$-Cycloalkyl wie unter $R^1$ genannt, insbesondere Cyclopentyl und Cyclohexyl;

Phenyl, das eine bis drei der folgenden Gruppen tragen kann: Alkyl wie unter $R^1$ genannt, insbesondere Methyl und Ethyl; Alkoxy wie unter $R^1$ genannt, insbesondere Methoxy und Ethoxy; Halogenalkyl wie unter $R^1$ genannt, insbesondere Trifluormethyl; Halogenalkoxy wie unter $R^1$ genannt, insbesondere Trifluormethoxy; Alkoxycarbonyl wie vorstehend genannt, insbesondere Methoxycarbonyl; Halogen wie unter $R^1$ genannt, insbesondere Fluor, Chlor und Brom, Nitro und Cyano;

$C_3$-$C_6$-Alkenyl wie unter $R^1$ genannt, insbesondere Allyl und Methallyl, $C_5$-$C_6$-Cycloalkenyl wie 2-Cyclopentenyl und 2-Cyclohexenyl, insbesondere 2-Cyclohexenyl oder $C_3$-$C_6$-Alkinyl wie unter $R^1$ genannt, insbesondere Propargyl, wobei diese Reste eine der folgenden Gruppen tragen können: Hydroxy; Alkoxy wie unter $R^1$ genannt, insbesondere Methoxy und Ethoxy; Halogen wie unter $R^1$ genannt, insbesondere Iod, oder Phenyl, welches seinerseits eine bis drei der folgenden Gruppen tragen kann: Alkyl wie unter $R^1$ genannt, insbesondere Methyl und Ethyl; Alkoxy wie unter $R^1$ genannt, insbesondere Methoxy und Ethoxy; Halogenalkyl wie unter $R^1$ genannt, insbesondere Trifluormethyl; Halogen wie unter $R^1$ genannt, insbesondere Fluor und Chlor, Nitro oder Cyano;

einen fünf- bis sechsgliedrigen heterocyclischen Rest enthaltend ein oder zwei Heteroatome, ausgewählt aus der Gruppe Sauerstoff, Schwefel und Stickstoff wie unter $R^1$ genannt, insbesondere Tetrahydrofuranyl und Tetrahydropyranyl oder einen Benzotriazolrest;

Phthalimido; Tetrahydrophthalimido; Succinimido; Maleinimido;

ein Äquivalent eines Kations aus der Gruppe der Alkali- oder Erdalkalimetalle, Mangan, Kupfer, Eisen, Ammonium und substituiertes Ammonium

oder einen Rest $-N=CR^6R^7$, wobei $R^6$ und $R^7$ unabhängig voneinander Wasserstoff, Alkyl wie unter $R^1$ genannt, insbesondere Methyl, Ethyl und iso-Propyl; Cycloalkyl wie unter $R^1$ genannt, insbesondere Cyclopropyl; Phenyl oder Furyl bedeuten oder zusammen eine Methylenkette der Formel $-(CH_2)_m-$ mit m = 4 bis 7 Kettengliedern,

$R^3$ Wasserstoff,

$C_1$-$C_6$-Alkyl, wie unter $R^1$ genannt, insbesondere Methyl, Ethyl, iso-Propyl, das einen bis drei der

folgenden Substituenten tragen kann: Hydroxy; Halogen wie unter $R^1$ genannt, insbesondere Fluor und Chlor; Alkoxy wie unter $R^1$ genannt, insbesondere Methoxy und Ethoxy; Alkylthio wie unter $R^1$ genannt, insbesondere Methylthio und Ethylthio, oder Dialkylamino wie unter $R^5$ genannt, insbesondere Dimethylamino;

Cycloalkyl wie unter $R^1$ genannt, insbesondere Cyclopropyl, Cyclobutyl, Cyclopentyl und Cyclohexyl, welches ein bis drei der folgenden Reste tragen kann: Alkyl wie unter $R^1$ genannt, insbesondere Methyl, Ethyl und Isopropyl; Halogen wie unter $R^1$ genannt, insbesondere Fluor und Chlor, oder Halogenalkyl wie unter $R^1$ genannt, insbesondere Trifluormethyl;

$R^4$ Hydroxy;

Alkoxy wie unter $R^1$ genannt, insbesondere Methoxy und Ethoxy;

Alkyl wie unter $R^1$ genannt, insbesondere Methyl, Ethyl, n-Propyl, iso-Propyl, n-Butyl, sek.-Butyl, iso-Butyl und tert.-Butyl; das eine bis drei der folgenden Gruppen tragen kann: Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio; Halogenalkylthio wie unter $R^1$ genannt, insbesondere Trifluormethylthio; Dialkylamino wie unter $R^1$ genannt, insbesondere Dimethylamino und Diethylamino; Halogen wie unter $R^1$ genannt, insbesondere Fluor und Chlor; Cycloalkyl wie unter $R^1$ genannt, insbesondere Cyclopropyl, Cyclopentyl und Cyclohexyl, oder Phenyl, welches seinerseits ein bis drei der folgenden Reste tragen kann: Halogen, wie bei $R^1$ genannt, insbesondere Fluor und Chlor; Cyano; Nitro; Alkyl wie unter $R^1$ genannt, insbesondere Methyl und Ethyl ; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio, oder Halogenalkylthio wie unter $R^1$ genannt, insbesondere Trifluormethylthio;

Cycloalkyl wie unter $R^1$ genannt, insbesondere Cyclopropyl, Cyclobutyl, Cyclopentyl und CyclohexyL, das eine bis drei der folgenden Gruppen tragen kann: Alkyl wie unter $R^1$ genannt, insbesondere Methyl, Ethyl und Isopropyl; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Halogen wie unter $R^1$ genannt, insbesondere Fluor und Chlor, Nitro oder Cyano;

Alkenyl oder $C_3$-$C_6$-Alkinyl wie unter $R^1$ genannt, insbesondere Allyl, Methallyl, Propargyl und 1,1-Dimethyl2-2-propinyl, das ein- bis dreimal durch Halogen wie unter $R^1$ genannt, insbesondere Fluor und Chlor, und/oder einmal durch Phenyl substituiert sein kann, wobei der Phenylrest seinerseits eine bis drei der folgenden Gruppen tragen kann: Alkyl wie unter $R^1$ genannt, insbesondere Methyl und Ethyl: Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio; Halogenalkylthio wie unter $R^1$ genannt, insbesondere Trifluormethylthio; Halogen wie unter $R^1$ genannt, insbesondere Fluor und Chlor, Cyano oder Nitro;

ein 5- bis 6-gliedriger heterocyclischer Rest enthaltend ein oder zwei Heteroatome, ausgewählt aus der Gruppe Sauerstoff, Schwefel oder Stickstoff wie unter $R^1$ genannt, welcher ein bis drei der folgenden Gruppen tragen kann: Alkyl wie unter $R^1$ genannt, insbesondere Methyl, Ethyl und iso-Propyl, oder Halogen wie unter $R^1$ genannt, insbesondere Fluor und Chlor;

Phenyl, das eine bis vier der folgenden Gruppen tragen kann: Alkyl wie unter $R^1$ genannt, insbesondere Methyl, Ethyl und Isopropyl; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio; Halogenalkylthio wie unter $R^1$ genannt, insbesondere Trifluormethylthio; Halogen wie unter $R^1$ genannt, insbesondere Fluor und Chlor; Nitro; Cyano; Formyl; Alkanoyl wie Acetyl, Propionyl, Butyryl, insbesondere Acetyl; Halogenalkanoyl, wie Trifluoracetyl, Trichloracetyl, Pentafluorpropionyl, insbesondere Trifluoracetyl, oder Alkoxycarbonyl wie unter $R^1$ genannt, insbesondere Methoxycarbonyl;

Naphthyl, das ein- bis dreimal durch Alkyl wie unter $R^1$ genannt, insbesondere Methyl und Ethyl, oder Halogen wie unter $R^1$ genannt, insbesondere Fluor und Chlor substituiert sein kann,

oder

$R^3$ und $R^4$ gemeinsam einen Rest der Struktur $-(CH_2)_n-Y_p-(CH_2)_q-$, wobei n und q 1, 2 oder 3, p 0 oder 1 und Y Sauerstoff, Schwefel oder N-Methyl wie $-(CH_2)_3-$, $-(CH_2)_4-$, $-(CH_2)_5-$, $-(CH_2)_6-$, $-CH_2-O-CH_2-$, $-CH_2-CH_2-O-CH_2-CH_2-$, $-CH_2-S-CH_2-$, $-CH_2-CH_2-S-CH_2-CH_2-$, $-CH_2-CH_2-N(CH_3)-CH_2-$

$CH_2$-, insbesondere $-(CH_2)_5-$ und $-CH_2-CH_2-O-CH_2-CH_2-$, oder den Rest der Formel $-(CH_2)_3-CO-$ bilden können;

sowie deren umweltverträglichen Salze.

Insbesondere bevorzugt sind Verbindungen Ia' und Ib', in denen $R^3$ Wasserstoff bedeutet sowie solche, in denen die Substituenten folgende Bedeutung haben:

$R^1$ Wasserstoff;

Methyl, Ethyl, Propyl, 1-Methylethyl, Butyl, 1-Methylpropyl, 2-Methylpropyl und 1,1-Dimethylethyl;

Methoxy, Ethoxy, Propyloxy, 1-Methylethoxy, Butyloxy, 1-Methylpropyloxy, 2-Methylpropyloxy und 1,1-Dimethylethoxy;

Difluormethoxy und Trifluormethoxy;

Methylthio, Ethylthio, Propylthio, 1-Methylethylthio, Butylthio, 1-Methylpropylthio, 2-Methylpropylthio und 1,1-Dimethylethylthio; Difluormethylthio und Trifluormethylthio;

$R^2$ einen Rest $-COYR^5$;

$R^5$

Wasserstoff; Phthalimido; Succinimido; Maleinimido, oder einen Rest $-N=CR^6R^7$;

$R^6$, $R^7$

Wasserstoff;

Methyl, Ethyl, Propyl, 1-Methylethyl, Butyl, 1-Methylpropyl, 2-Methylpropyl und 1,1-Dimethylethyl;

Cyclopropyl, Cyclopentyl, Cyclohexyl und Cycloheptyl;

oder gemeinsam eine 4- bis 7-gliedrige Alkylenkette wie $-CH_2CH_2CH_2CH_2-$, $-CH_2CH_2CH_2CH_2CH_2-$, $-CH_2CH_2CH_2CH_2CH_2CH_2-$ und $-CH_2CH_2CH_2CH_2CH_2CH_2CH_2-$;

$R^4$ Methyl, Ethyl, Propyl, 1-Methylethyl, Butyl, 1-Methylpropyl, 2-Methylpropyl und 1,1-Dimethylethyl;

Cyclopropyl, Cyclopentyl, Cyclohexyl und Cycloheptyl;

oder gemeinsam eine 4- bis 7-gliedrige Alkylenkette wie $-CH_2CH_2CH_2CH_2-$, $-CH_2CH_2CH_2CH_2CH_2-$, $CH_2CH_2CH_2CH_2CH_2CH_2-$ und $-CH_2CH_2CH_2CH_2CH_2CH_2CH_2-$;

Beispiele für sehr aktive Verbindungen der Formeln Ia und Ib sind in den nachstehenden Tabellen aufgeführt:

Tabelle A

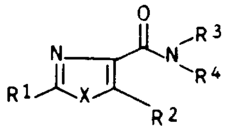 Ia

(X = O oder S)

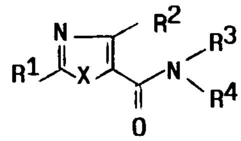 Ib

| $R^1$ | $R^2$ | $R^3$ | $R^4$ |
|---|---|---|---|
| H | COOH | H | tert.-Butyl |
| F | COOH | H | tert.-Butyl |
| Cl | COOH | H | tert.-Butyl |
| Methyl | COOH | H | tert.-Butyl |
| Ethyl | COOH | H | tert.-Butyl |
| n-Propyl | COOH | H | tert.-Butyl |
| iso-Propyl | COOH | H | tert.-Butyl |
| n-Butyl | COOH | H | tert.-Butyl |
| iso-Butyl | COOH | H | tert.-Butyl |
| sek.-Butyl | COOH | H | tert.-Butyl |
| tert.-Butyl | COOH | H | tert.-Butyl |
| cyclo-Propyl | COOH | H | tert.-Butyl |
| cyclo-Pentyl | COOH | H | tert.-Butyl |
| 1-Methylcyclopropyl | COOH | H | tert.-Butyl |
| Trifluormethyl | COOH | H | tert.-Butyl |
| Chlordifluormethyl | COOH | H | tert.-Butyl |
| Pentafluorethyl | COOH | H | tert.-Butyl |
| Methoxymethyl | COOH | H | tert.-Butyl |
| 1-Methylmethoxymethyl | COOH | H | tert.-Butyl |
| 1-Methylmethoxyethyl | COOH | H | tert.-Butyl |
| Ethoxymethyl | COOH | H | tert.-Butyl |
| Vinyl | COOH | H | tert.-Butyl |
| Allyl | COOH | H | tert.-Butyl |
| Methallyl | COOH | H | tert.-Butyl |
| Crotyl | COOH | H | tert.-Butyl |
| Ethinyl | COOH | H | tert.-Butyl |
| Propargyl | COOH | H | tert.-Butyl |
| Phenylethinyl | COOH | H | tert.-Butyl |
| Methoxy | COOH | H | tert.-Butyl |
| Ethoxy | COOH | H | tert.-Butyl |
| Trifluormethoxy | COOH | H | tert.-Butyl |
| Methylthio | COOH | H | tert.-Butyl |

| $R^1$ | $R^2$ | $R^3$ | $R^4$ |
|---|---|---|---|
| Trifluormethylthio | COOH | H | tert.-Butyl |
| Phenoxy | COOH | H | tert.-Butyl |
| 4-Cl-Phenoxy | COOH | H | tert.-Butyl |
| 2,4-(Cl,Cl)-Phenoxy | COOH | H | tert.-Butyl |
| 4-$CF_3$-Phenoxy | COOH | H | tert.-Butyl |
| 2-F-Phenylthio | COOH | H | tert.-Butyl |
| iso-Propoxy | COOH | H | tert.-Butyl |
| H | COOH | H | cyclo-Propyl |
| F | COOH | H | cyclo-Propyl |
| Cl | COOH | H | cyclo-Propyl |

| $R^1$ | $R^2$ | $R^3$ | $R^4$ |
|---|---|---|---|
| Methyl | COOH | H | cyclo-Propyl |
| Ethyl | COOH | H | cyclo-Propyl |
| n-Propyl | COOH | H | cyclo-Propyl |
| iso-Propyl | COOH | H | cyclo-Propyl |
| n-Butyl | COOH | H | cyclo-Propyl |
| iso-Butyl | COOH | H | cyclo-Propyl |
| sek.-Butyl | COOH | H | cyclo-Propyl |
| tert.-Butyl | COOH | H | cyclo-Propyl |
| cyclo-Propyl | COOH | H | cyclo-Propyl |
| 1-Methylcyclopropyl | COOH | H | cyclo-Propyl |
| Trifluormethyl | COOH | H | cyclo-Propyl |
| Chlordifluormethyl | COOH | H | cyclo-Propyl |
| Pentafluorethyl | COOH | H | cyclo-Propyl |
| Methoxymethyl | COOH | H | cyclo-Propyl |
| 1-Methylmethoxymethyl | COOH | H | cyclo-Propyl |
| 1-Methylmethoxyethyl | COOH | H | cyclo-Propyl |
| Ethoxymethyl | COOH | H | cyclo-Propyl |
| Vinyl | COOH | H | cyclo-Propyl |
| Allyl | COOH | H | cyclo-Propyl |
| Methallyl | COOH | H | cyclo-Propyl |
| Crotyl | COOH | H | cyclo-Propyl |
| Ethinyl | COOH | H | cyclo-Propyl |
| Propargyl | COOH | H | cyclo-Propyl |
| Phenylethinyl | COOH | H | cyclo-Propyl |
| Methoxy | COOH | H | cyclo-Propyl |
| Ethoxy | COOH | H | cyclo-Propyl |
| Trifluormethoxy | COOH | H | cyclo-Propyl |
| Methylthio | COOH | H | cyclo-Propyl |
| Trifluormethylthio | COOH | H | cyclo-Propyl |
| Phenoxy | COOH | H | cyclo-Propyl |
| 4-Cl-Phenoxy | COOH | H | cyclo-Propyl |
| 2,4-(Cl,Cl)-Phenoxy | COOH | H | cyclo-Propyl |
| 4-$CF_3$-Phenoxy | COOH | H | cyclo-Propyl |
| 2-F-Phenylthio | COOH | H | cyclo-Propyl |

| $R^1$ | $R^2$ | $R^3$ | $R^4$ |
|---|---|---|---|
| iso-Propoxy | COOH | H | cyclo-Propyl |
| H | COOH | Methyl | tert.-Butyl |
| F | COOH | Methyl | tert.-Butyl |
| Cl | COOH | Methyl | tert.-Butyl |
| Methyl | COOH | Methyl | tert.-Butyl |
| Ethyl | COOH | Methyl | tert.-Butyl |
| n-Propyl | COOH | Methyl | tert.-Butyl |
| iso-Propyl | COOH | Methyl | tert.-Butyl |
| n-Butyl | COOH | Methyl | tert.-Butyl |
| iso-Butyl | COOH | Methyl | tert.-Butyl |
| sek.-Butyl | COOH | Methyl | tert.-Butyl |
| tert.-Butyl | COOH | Methyl | tert.-Butyl |
| cyclo-Propyl | COOH | Methyl | tert.-Butyl |

| $R^1$ | $R^2$ | $R^3$ | $R^4$ |
|---|---|---|---|
| cyclo-Pentyl | COOH | Methyl | tert.-Butyl |
| 1-Methylcyclopropyl | COOH | iso-Propyl | tert.-Butyl |
| Trifluormethyl | COOH | iso-Propyl | tert.-Butyl |
| Chlordifluormethyl | COOH | iso-Propyl | tert.-Butyl |
| Pentafluorethyl | COOH | iso-Propyl | tert.-Butyl |
| Methoxymethyl | COOH | iso-Propyl | tert.-Butyl |
| 1-Methylmethoxymethyl | COOH | iso-Propyl | tert.-Butyl |
| 1-Methylmethoxyethyl | COOH | iso-Propyl | tert.-Butyl |
| Ethoxymethyl | COOH | iso-Propyl | tert.-Butyl |
| Vinyl | COOH | iso-Propyl | tert.-Butyl |
| Allyl | COOH | iso-Propyl | tert.-Butyl |
| Methallyl | COOH | iso-Propyl | tert.-Butyl |
| Crotyl | COOH | iso-Propyl | tert.-Butyl |
| Ethinyl | COOH | iso-Propyl | tert.-Butyl |
| Propargyl | COOH | iso-Propyl | tert.-Butyl |
| Phenylethinyl | COOH | iso-Propyl | tert.-Butyl |
| Methoxy | COOH | iso-Propyl | tert.-Butyl |
| Ethoxy | COOH | iso-Propyl | tert.-Butyl |
| Trifluormethoxy | COOH | iso-Propyl | tert.-Butyl |
| H | COOH | Methyl | cyclo-Propyl |
| F | COOH | Methyl | cyclo-Propyl |
| Cl | COOH | Methyl | cyclo-Propyl |
| Methyl | COOH | Methyl | cyclo-Propyl |
| Ethyl | COOH | Methyl | cyclo-Propyl |
| n-Propyl | COOH | Methyl | cyclo-Propyl |
| iso-Propyl | COOH | Methyl | cyclo-Propyl |
| n-Butyl | COOH | Methyl | cyclo-Propyl |
| iso-Butyl | COOH | iso-Propyl | cyclo-Propyl |
| sek.-Butyl | COOH | iso-Propyl | cyclo-Propyl |
| tert.-Butyl | COOH | iso-Propyl | tert.-Butyl |
| cyclo-Propyl | COOH | iso-Propyl | cyclo-Propyl |
| cyclo-Pentyl | COOH | iso-Propyl | cyclo-Propyl |
| 1-Methylcyclopropyl | COOH | Methyl | cyclo-Propyl |
| Trifluormethyl | COOH | Methyl | cyclo-Propyl |
| Chlordifluormethyl | COOH | Methyl | cyclo-Propyl |

| $R^1$ | $R^2$ | $R^3$ | $R^4$ |
|---|---|---|---|
| Pentafluorethyl | COOH | Methyl | cyclo-Propyl |
| Methoxymethyl | COOH | iso-Propyl | cyclo-Propyl |
| 1-Methylmethoxymethyl | COOH | iso-Propyl | cyclo-Propyl |
| 1-Methylmethoxyethyl | COOH | iso-Propyl | cyclo-Propyl |
| Ethoxymethyl | COOH | iso-Propyl | cyclo-Propyl |
| Vinyl | COOH | iso-Propyl | cyclo-Propyl |
| Allyl | COOH | iso-Propyl | cyclo-Propyl |
| Methallyl | COOH | iso-Propyl | cyclo-Propyl |
| Crotyl | COOH | Methyl | cyclo-Propyl |
| Ethinyl | COOH | Methyl | cyclo-Propyl |
| Propargyl | COOH | Methyl | cyclo-Propyl |
| Phenylethinyl | COOH | Methyl | cyclo-Propyl |
| Methoxy | COOH | Methyl | cyclo-Propyl |
| Ethoxy | COOH | Methyl | cyclo-Propyl |
| Trifluormethoxy | COOH | Methyl | cyclo-Propyl |

Tabelle A-1

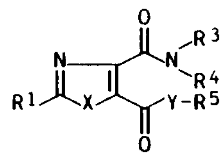 Ia bzw. 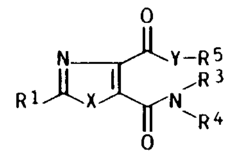 Ib

| $R^1$ | $R^3$ | $R^4$ | $R^5$ | X | Y |
|---|---|---|---|---|---|
| Chlor | H | tert.-Butyl | 4-Hydroxy-2-butinyl | S | O |
| Chlor | H | tert.-Butyl | $N=C(C_2H_5)_2$ | S | O |
| Chlor | H | tert.-Butyl | $N=C(cyclo-C_3H_5)_2$ | S | O |
| Chlor | H | tert.-Butyl | 2-Butanimino | S | O |
| Chlor | H | tert.-Butyl | Cyclohexanimino | S | O |
| Chlor | H | tert.-Butyl | Cyclooctanimino | S | O |
| Methyl | H | tert.-Butyl | $N=CH-C_6H_5$ | S | O |
| Methyl | H | tert.-Butyl | 2-Furyl-methanimino | S | O |
| Methyl | H | tert.-Butyl | $CH_2CH_2N(CH_3)_2$ | S | O |
| Methyl | H | tert.-Butyl | $CH_2CH_2N^+(CH_3)_3I^-$ | S | O |
| Methyl | H | tert.-Butyl | $CH_2CF_3$ | S | O |
| Methyl | H | tert.-Butyl | $CH_2CH_2Cl$ | S | O |
| Methyl | H | tert.-Butyl | $CH_2CH_2CN$ | S | O |
| iso-Propyl | H | tert.-Butyl | $CH_2CCl_3$ | S | O |
| iso-Propyl | H | tert.-Butyl | $CH_2CH_2Si(CH_3)_3$ | S | O |
| iso-Propyl | H | tert.-Butyl | $CH_2CH_2O-N=C(CH_3)_2$ | S | O |
| iso-Propyl | H | tert.-Butyl | $CH_2PO(OC_2H_5)_2$ | S | O |
| iso-Propyl | H | tert.-Butyl | $CH(CH_3)CH(OCH_3)_2$ | S | O |
| iso-Propyl | H | tert.-Butyl | $CH_2-CON(C_2H_5)_2$ | S | O |

| $R^1$ | $R^3$ | $R^4$ | $R^5$ | X | Y |
|---|---|---|---|---|---|
| iso-Propyl | H | tert.-Butyl | Benzyl | S | O |
| cyclo-Propyl | H | tert.-Butyl | 2,4-(Cl,Cl)-Benzyl | S | O |
| cyclo-Propyl | H | tert.-Butyl | 3-Pyridyl-methyl | S | O |
| cyclo-Propyl | H | tert.-Butyl | 2-Thienyl-methyl | S | O |
| cyclo-Propyl | H | tert.-Butyl | 2-Tetrahydrofuranyl-methyl | S | O |
| cyclo-Propyl | H | tert.-Butyl | 2-Furanyl-methyl | S | O |
| cyclo-Propyl | H | tert.-Butyl | 2-Pyridyl-methyl | S | O |
| cyclo-Propyl | H | tert.-Butyl | Phenyl | S | O |
| Allyl | H | tert.-Butyl | 4-F-Phenyl | S | O |
| Allyl | H | tert.-Butyl | 4-Trifluormethylphenyl | S | O |
| Allyl | H | tert.-Butyl | $2-NO_2-4-F-Phenyl$ | S | O |
| Allyl | H | tert.-Butyl | $3,5-(CF_3,CF_3)-Phenyl$ | S | O |
| Allyl | H | tert.-Butyl | $4-OCH_3-Phenyl$ | S | O |
| Allyl | H | tert.-Butyl | $4-OCF_3-Phenyl$ | S | O |
| Allyl | H | tert.-Butyl | $4-NHCOCH_3-Phenyl$ | S | O |
| Ethinyl | H | tert.-Butyl | 2-Tetrahydropyranyl | S | O |
| Ethinyl | H | tert.-Butyl | 2-Tetrahydropyranyl | S | O |
| Ethinyl | H | tert.-Butyl | 1-Benzotriazolyl | S | O |
| Ethinyl | H | tert.-Butyl | Methyl | S | O |
| Ethinyl | H | tert.-Butyl | Ethyl | S | O |
| Ethinyl | H | tert.-Butyl | n-Propyl | S | O |
| Ethinyl | H | tert.-Butyl | iso-Propyl | S | O |
| Methoxy | H | tert.-Butyl | n-Butyl | S | O |
| Methoxy | H | tert.-Butyl | iso-Butyl | S | O |
| Methoxy | H | tert.-Butyl | sek.-Butyl | S | O |

| $R^1$ | $R^3$ | $R^4$ | $R^5$ | X | Y |
|---|---|---|---|---|---|
| Methoxy | H | tert.-Butyl | tert.-Butyl | S | O |
| Methoxy | H | tert.-Butyl | cyclo-Hexyl | S | O |
| Methoxy | H | tert.-Butyl | Cyclopropylmethyl | S | O |
| Methoxy | H | tert.-Butyl | Ethoxymethyl | S | O |
| 4-Cl-Phenoxy | H | tert.-Butyl | 2-Methoxy-ethoxy-methyl | S | O |
| 4-Cl-Phenoxy | H | tert.-Butyl | Benzyloxymethyl | S | O |
| 4-Cl-Phenoxy | H | tert.-Butyl | (4-Brombenzoyl)-methyl | S | O |
| 4-Cl-Phenoxy | H | tert.-Butyl | (4-Methoxybenzoyl)-methyl | S | O |
| 4-Cl-Phenoxy | H | tert.-Butyl | Phthalimidomethyl | S | O |
| 4-Cl-Phenoxy | H | tert.-Butyl | Methylthiomethyl | S | O |
| 4-Cl-Phenoxy | H | tert.-Butyl | 2-Thiomethyl-ethyl | S | O |
| Phenylthio | H | tert.-Butyl | $CH(C_6H_5)COOCH_3$ | S | O |
| Phenylthio | H | tert.-Butyl | Phenylethyl | S | O |
| Phenylthio | H | tert.-Butyl | 4-F-Phenylethyl | S | O |
| Phenylthio | H | tert.-Butyl | Phthalimido | S | O |
| Phenylthio | H | tert.-Butyl | Tetrahydrophthalimido | S | O |
| Phenylthio | H | tert.-Butyl | Maleinimido | S | O |
| Phenylthio | H | tert.-Butyl | Succinimido | S | O |

| $R^1$ | $R^3$ | $R^4$ | $R^5$ | X | Y |
|---|---|---|---|---|---|
| Chlor | H | cyclo-Propyl | 4-Hydroxy-2-butinyl | S | O |
| Chlor | H | cyclo-Propyl | $N=C(C_2H_5)_2$ | S | O |
| Chlor | H | cyclo-Propyl | $N=C(cyclo-C_3H_5)_2$ | S | O |
| Chlor | H | cyclo-Propyl | 2-Butanimino | S | O |
| Chlor | H | cyclo-Propyl | Cyclohexanimino | S | O |
| Chlor | H | cyclo-Propyl | Cyclooctanimino | S | O |
| Methyl | H | cyclo-Propyl | $N=CH-C_6H_5$ | S | O |
| Methyl | H | cyclo-Propyl | 2-Furyl-methanimino | S | O |
| Methyl | H | cyclo-Propyl | $CH_2CH_2N(CH_3)_2$ | S | O |
| Methyl | H | cyclo-Propyl | $CH_2CH_2N^+(CH_3)_3I^-$ | S | O |
| Methyl | H | cyclo-Propyl | $CH_2CF_3$ | S | O |
| Methyl | H | cyclo-Propyl | $CH_2CH_2Cl$ | S | O |
| Methyl | H | cyclo-Propyl | $CH_2CH_2CN$ | S | O |
| iso-Propyl | H | cyclo-Propyl | $CH_2CCl_3$ | S | O |
| iso-Propyl | H | cyclo-Propyl | $CH_2CH_2Si(CH_3)_3$ | S | O |
| iso-Propyl | H | cyclo-Propyl | $CH_2CH_2O-N=C(CH_3)_2$ | S | O |
| iso-Propyl | H | cyclo-Propyl | $CH_2PO(OC_2H_5)_2$ | S | O |
| iso-Propyl | H | cyclo-Propyl | $CH(CH_3)CH(OCH_3)_2$ | S | O |
| iso-Propyl | H | cyclo-Propyl | $CH_2-CON(C_2H_5)_2$ | S | O |
| iso-Propyl | H | cyclo-Propyl | Benzyl | S | O |

| $R^1$ | $R^3$ | $R^4$ | $R^5$ | X | Y |
|---|---|---|---|---|---|
| cyclo-Propyl | H | cyclo-Propyl | 2,4-(Cl,Cl)-Benzyl | S | O |
| cyclo-Propyl | H | cyclo-Propyl | 3-Pyridyl-methyl | S | O |
| cyclo-Propyl | H | cyclo-Propyl | 2-Thienyl-methyl | S | O |
| cyclo-Propyl | H | cyclo-Propyl | 2-Tetrahydrofuranyl-methyl | S | O |
| cyclo-Propyl | H | cyclo-Propyl | 2-Furanyl-methy | S | O |
| cyclo-Propyl | H | cyclo-Propyl | 2-Pyridyl-methyl | S | O |
| cyclo-Propyl | H | cyclo-Propyl | Phenyl | S | O |
| Allyl | H | cyclo-Propyl | 4-F-Phenyl | S | O |
| Allyl | H | cyclo-Propyl | 4-Trifluormethylphenyl | S | O |
| Allyl | H | cyclo-Propyl | 2-$NO_2$-F-Phenyl | S | O |
| Allyl | H | cyclo-Propyl | 3,5-($CF_3$,$CF_3$)-Phenyl | S | O |
| Allyl | H | cyclo-Propyl | 4-$OCH_3$-Phenyl | S | O |
| Allyl | H | cyclo-Propyl | 4-$OCF_3$-Phenyl | S | O |
| Allyl | H | cyclo-Propyl | 4-$NHCOCH_3$-Phenyl | S | O |
| Ethinyl | H | cyclo-Propyl | 2-Tetrahydropyranyl | S | O |
| Ethinyl | H | cyclo-Propyl | 2-Tetrahydrofuranyl | S | O |
| Ethinyl | H | cyclo-Propyl | 1-Benzotriazolyl | S | O |
| Ethinyl | H | cyclo-Propyl | Methyl | S | O |
| Ethinyl | H | cyclo-Propyl | Ethyl | S | O |
| Ethinyl | H | cyclo-Propyl | n-Propyl | S | O |
| Ethinyl | H | cyclo-Propyl | iso-Propyl | S | O |
| Methoxy | H | cyclo-Propyl | n-Butyl | S | O |
| Methoxy | H | cyclo-Propyl | iso-Butyl | S | O |
| Methoxy | H | cyclo-Propyl | sek.-Butyl | S | O |
| Methoxy | H | cyclo-Propyl | tert.-Butyl | S | O |

| $R^1$ | $R^3$ | $R^4$ | $R^5$ | X | Y |
|---|---|---|---|---|---|
| Methoxy | H | cyclo-Propyl | cyclo-Hexyl | S | O |
| Methoxy | H | cyclo-Propyl | Cyclopropylmethyl | S | O |
| Methoxy | H | cyclo-Propyl | Ethoxymethyl | S | O |
| 4-Cl-Phenoxy | H | cyclo-Propyl | 2-Methoxy-ethoxy-methyl | S | O |
| 4-Cl-Phenoxy | H | cyclo-Propyl | Benzyloxymethyl | S | O |
| 4-Cl-Phenoxy | H | cyclo-Propyl | (4-Brombenzoyl)-methyl | S | O |
| 4-Cl-Phenoxy | H | cyclo-Propyl | (4-Methoxybenzoyl)-methyl | S | O |
| 4-Cl-Phenoxy | H | cyclo-Propyl | Phthalimidomethyl | S | O |
| 4-Cl-Phenoxy | H | cyclo-Propyl | Methylthiomethyl | S | O |
| 4-Cl-Phenoxy | H | cyclo-Propyl | 2-Thiomethyl-ethyl | S | O |
| Phenylthio | H | cyclo-Propyl | $CH(C_6H_5)COOCH_3$ | S | O |
| Phenylthio | H | cyclo-Propyl | Phenylethyl | S | O |
| Phenylthio | H | cyclo-Propyl | 4-F-Phenylethyl | S | O |
| Phenylthio | H | cyclo-Propyl | Phthalimido | S | O |
| Phenylthio | H | cyclo-Propyl | Tetrahydrophthalimido | S | O |
| Phenylthio | H | cyclo-Propyl | Maleinimido | S | O |
| Phenylthio | H | cyclo-Propyl | Succinimido | S | O |

| $R^1$ | $R^3$ | $R^4$ | $R^5$ | X | Y |
|---|---|---|---|---|---|
| H | H | tert.-Butyl | $-N=C(CH_3)_2$ | S | O |
| F | H | tert.-Butyl | $-N=C(CH_3)_2$ | S | O |
| Cl | H | tert.-Butyl | $-N=C(CH_3)_2$ | S | O |
| Methyl | H | tert.-Butyl | $-N=C(CH_3)_2$ | S | O |
| Ethyl | H | tert.-Butyl | $-N=C(CH_3)_2$ | S | O |
| n-Propyl | H | tert.-Butyl | $-N=C(CH_3)_2$ | S | O |
| iso-Propyl | H | tert.-Butyl | $-N=C(CH_3)_2$ | S | O |
| n-Butyl | H | tert.-Butyl | $-N=C(CH_3)_2$ | S | O |
| iso-Butyl | H | tert.-Butyl | $-N=C(CH_3)_2$ | S | O |
| sek.-Butyl | H | tert.-Butyl | $-N=C(CH_3)_2$ | S | O |
| tert.-Butyl | H | tert.-Butyl | $-N=C(CH_3)_2$ | S | O |
| cyclo-Propyl | H | tert.-Butyl | $-N=C(CH_3)_2$ | S | O |
| cyclo-Butyl | H | tert.-Butyl | $-N=C(CH_3)_2$ | S | O |
| cyclo-Pentyl | H | tert.-Butyl | $-N=C(CH_3)_2$ | S | O |
| cyclo-Hexyl | H | tert.-Butyl | $-N=C(CH_3)_2$ | S | O |
| cyclo-Heptyl | H | tert.-Butyl | $-N=C(CH_3)_2$ | S | O |
| cyclo-Octyl | H | tert.-Butyl | $-N=C(CH_3)_2$ | S | O |
| 1-Methylcyclopropyl | H | tert.-Butyl | $-N=C(CH_3)_2$ | S | O |
| Trifluormethyl | H | tert.-Butyl | $-N=C(CH_3)_2$ | S | O |
| Chlordifluormethyl | H | tert.-Butyl | $-N=C(CH_3)_2$ | S | O |
| Pentafluormethyl | H | tert.-Butyl | $-N=C(CH_3)_2$ | S | O |

| $R^1$ | $R^3$ | $R^4$ | $R^5$ | X | Y |
|---|---|---|---|---|---|
| iso-Propoxy | H | tert.-Butyl | $-N=C(CH_3)_2$ | S | O |
| Methoxymethyl | H | tert.-Butyl | $-N=C(CH_3)_2$ | S | O |
| 1-Methylmethoxymethyl | H | tert.-Butyl | $-N=C(CH_3)_2$ | S | O |
| 1-Methylmethoxyethyl | H | tert.-Butyl | $-N=C(CH_3)_2$ | S | O |
| Ethoxymethyl | H | tert.-Butyl | $-N=C(CH_3)_2$ | S | O |
| Vinyl | H | tert.-Butyl | $-N=C(CH_3)_2$ | S | O |
| Allyl | H | tert.-Butyl | $-N=C(CH_3)_2$ | S | O |
| Methallyl | H | tert.-Butyl | $-N=C(CH_3)_2$ | S | O |
| Crotyl | H | tert.-Butyl | $-N=C(CH_3)_2$ | S | O |
| Ethinyl | H | tert.-Butyl | $-N=C(CH_3)_2$ | S | O |
| Propargyl | H | tert.-Butyl | $-N=C(CH_3)_2$ | S | O |
| Phenyleethinyl | H | tert.-Butyl | $-N=C(CH_3)_2$ | S | O |
| Methoxy | H | tert.-Butyl | $-N=C(CH_3)_2$ | S | O |
| Ethoxy | H | tert.-Butyl | $-N=C(CH_3)_2$ | S | O |
| Trifluormethoxy | H | tert.-Butyl | $-N=C(CH_3)_2$ | S | O |
| Methylthio | H | tert.-Butyl | $-N=C(CH_3)_2$ | S | O |
| Trifluormethylthio | H | tert.-Butyl | $-N=C(CH_3)_2$ | S | O |
| Phenoxy | H | tert.-Butyl | $-N=C(CH_3)_2$ | S | O |
| 4-Cl-Phenoxy | H | tert.-Butyl | $-N=C(CH_3)_2$ | S | O |
| 2,4-(Cl,Cl)-Phenoxy | H | tert.-Butyl | $-N=C(CH_3)_2$ | S | O |
| 4-$CF_3$-Phenoxy | H | tert.-Butyl | $-N=C(CH_3)_2$ | S | O |

| $R^1$ | $R^3$ | $R^4$ | $R^5$ | X | Y |
|---|---|---|---|---|---|
| H | H | cyclo-Propyl | $-N=C(CH_3)_2$ | S | O |
| F | H | cyclo-Propyl | $-N=C(CH_3)_2$ | S | O |
| Cl | H | cyclo-Propyl | $-N=C(CH_3)_2$ | S | O |
| Methyl | H | cyclo-Propyl | $-N=C(CH_3)_2$ | S | O |
| Ethyl | H | cyclo-Propyl | $-N=C(CH_3)_2$ | S | O |
| n-Propyl | H | cyclo-Propyl | $-N=C(CH_3)_2$ | S | O |
| iso-Propyl | H | cyclo-Propyl | $-N=C(CH_3)_2$ | S | O |
| n-Butyl | H | cyclo-Propyl | $-N=C(CH_3)_2$ | S | O |
| iso-Butyl | H | cyclo-Propyl | $-N=C(CH_3)_2$ | S | O |
| sek.-Butyl | H | cyclo-Propyl | $-N=C(CH_3)_2$ | S | O |
| tert.-Butyl | H | cyclo-Propyl | $-N=C(CH_3)_2$ | S | O |
| cyclo-Propyl | H | cyclo-Propyl | $-N=C(CH_3)_2$ | S | O |
| cyclo-Pentyl | H | cyclo-Propyl | $-N=C(CH_3)_2$ | S | O |
| 1-Methylcyclopropyl | H | cyclo-Propyl | $-N=C(CH_3)_2$ | S | O |
| Trifluormethyl | H | cyclo-propyl | $-N=C(CH_3)_2$ | S | O |
| Chlordifluormethyl | H | cyclo-Propyl | $-N=C(CH_3)_2$ | S | O |
| Pentafluorethyl | H | cyclo-Propyl | $-N=C(CH_3)_2$ | S | O |
| iso-Propoxy | H | cyclo-Propyl | $-N=C(CH_3)_2$ | S | O |

| $R^1$ | $R^3$ | $R^4$ | $R^5$ | X | Y |
|---|---|---|---|---|---|
| Methoxymethyl | H | cyclo-Propyl | $-N=C(CH_3)_2$ | S | O |
| 1-Methylmethoxymethyl | H | cyclo-Propyl | $-N=C(CH_3)_2$ | S | O |
| 1-Methylmethoxyethyl | H | cyclo-Propyl | $-N=C(CH_3)_2$ | S | O |
| Ethoxymethyl | H | cyclo-Propyl | $-N=C(CH_3)_2$ | S | O |
| Vinyl | H | cyclo-Propyl | $-N=C(CH_3)_2$ | S | O |
| Allyl | H | cyclo-Propyl | $-N=C(CH_3)_2$ | S | O |
| Methallyl | H | cyclo-Propyl | $-N=C(CH_3)_2$ | S | O |
| Crotyl | H | cyclo-Propyl | $-N=C(CH_3)_2$ | S | O |
| Ethinyl | H | cyclo-Propyl | $-N=C(CH_3)_2$ | S | O |
| Propargyl | H | cyclo-Propyl | $-N=C(CH_3)_2$ | S | O |
| Phenylethinyl | H | cyclo-Propyl | $-N=C(CH_3)_2$ | S | O |
| Methoxy | H | cyclo-Propyl | $-N=C(CH_3)_2$ | S | O |
| Ethoxy | H | cyclo-Propyl | $-N=C(CH_3)_2$ | S | O |
| Trifluormethoxy | H | cyclo-Propyl | $-N=C(CH_3)_2$ | S | O |
| Methylthio | H | cyclo-Propyl | $-N=C(CH_3)_2$ | S | O |
| Trifluormethylthio | H | cyclo-Propyl | $-N=C(CH_3)_2$ | S | O |
| Phenoxy | H | cyclo-Propyl | $-N=C(CH_3)_2$ | S | O |
| 4-Cl-Phenoxy | H | cyclo-Propyl | $-N=C(CH_3)_2$ | S | O |
| 2,4-(Cl,Cl)-Phenoxy | H | cyclo-Propyl | $-N=C(CH_3)_2$ | S | O |
| 4-$CF_3$-Phenoxy | H | cyclo-Propyl | $-N=C(CH_3)_2$ | S | O |
| 2-F-Phenylthio | H | cyclo-Propyl | $-N=C(CH_3)_2$ | S | O |

| $R^1$ | $R^3$ | $R^4$ | $R^5$ | X | Y |
|---|---|---|---|---|---|
| Chlor | H | Methyl | H | S | O |
| Chlor | H | Ethyl | H | S | O |
| Chlor | H | n-Propyl | H | S | O |
| Chlor | H | iso-Propyl | H | S | O |
| Chlor | H | n-Butyl | H | S | O |
| Chlor | H | iso-Butyl | H | S | O |
| Methyl | H | sek.-Butyl | H | S | O |
| Methyl | H | n-Pentyl | H | S | O |
| Methyl | H | 2-Pentyl | H | S | O |
| Methyl | H | 3-Pentyl | H | S | O |
| Methyl | H | n-Hexyl | H | S | O |
| Methyl | H | 2-Hexyl | H | S | O |
| iso-Propyl | H | 3-Hexyl | H | S | O |
| iso-Propyl | H | 2-Methyl-2-pentyl | H | S | O |
| iso-Propyl | H | cyclo-Propylmethyl | H | S | O |
| iso-Propyl | H | cyclo-Butyl | H | S | O |
| iso-Propyl | H | cyclo-Pentyl | H | S | O |
| iso-Propyl | H | cyclo-Hexyl | H | S | O |
| cyclo-Propyl | H | 1-Methylcyclohexyl | H | S | O |
| cyclo-Propyl | H | 3-Trifluormethylcyclohexyl | H | S | O |
| cyclo-Propyl | H | Allyl | H | S | O |
| cyclo-Propyl | H | 1-Buten-3-yl | H | S | O |
| cyclo-Propyl | H | Crotyl | H | S | O |

| $R^1$ | $R^3$ | $R^4$ | $R^5$ | X | Y |
|---|---|---|---|---|---|
| cyclo-Propyl | H | Propargyl | H | S | O |
| Allyl | H | 1-Butin-3-yl | H | S | O |
| Allyl | H | 3-Methyl-1-butin-3-yl | H | S | O |
| Allyl | H | 2-Pentin-4-yl | H | S | O |
| Allyl | H | Benzyl | H | S | O |
| Allyl | H | 2-Phenylethyl | H | S | O |
| Allyl | H | 2-Methylthioethyl | H | S | O |
| Ethinyl | H | 2-Chlorethyl | H | S | O |
| Ethinyl | H | 2-Methoxyethyl | H | S | O |
| Ethinyl | H | 2-(N,N-Dimethylamino)ethyl | H | S | O |
| Ethinyl | H | Phenyl | H | S | O |
| Ethinyl | H | 2-$CH_3$-Phenyl | H | S | O |
| Ethinyl | H | 4-$CH_3$-Phenyl | H | S | O |
| Methoxy | H | 2,4-($CH_3$,$CH_3$)-Phenyl | H | S | O |
| Methoxy | H | 2,3,5-($CH_3$,$CH_3$,$CH_3$)-Phenyl | H | S | O |
| Methoxy | H | 3-$CF_3$-Phenyl | H | S | O |
| Methoxy | H | 3-F-Phenyl | H | S | O |
| Methoxy | H | 2-Cl-Phenyl | H | S | O |
| Methoxy | H | 4-Cl-Phenyl | H | S | O |
| 4-Cl-Phenoxy | H | 2,4-(F,F)-Phenyl | H | S | O |
| 4-Cl-Phenoxy | H | 2,3,5-(Cl,Cl,Cl)-Phenyl | H | S | O |
| 4-Cl-Phenoxy | H | 2-CN-Phenyl | H | S | O |
| 4-Cl-Phenoxy | H | 2-$OCH_3$-Phenyl | H | S | O |
| 4-Cl-Phenoxy | H | 2,3-($OCH_3$,$OCH_3$)-Phenyl | H | S | O |
| 4-Cl-Phenoxy | H | 3,4,5-($OCH_3$,$OCH_3$,$OCH_3$)-Phenyl | H | S | O |

| $R^1$ | $R^3$ | $R^4$ | $R^5$ | X | Y |
|---|---|---|---|---|---|
| Phenylthio | H | 3-$OCF_3$-Phenyl | H | S | O |
| Phenylthio | H | 4-$OCF_2CHF_2$-Phenyl | H | S | O |
| Phenylthio | H | 2-$SCH_3$-Phenyl | H | S | O |
| Phenylthio | H | 2,4-($SCH_3$,$SCH_3$)-Phenyl | H | S | O |
| Phenylthio | H | 2-$SCF_3$-Phenyl | H | S | O |
| Phenylthio | H | 4-$NO_2$-Phenyl | H | S | O |
| iso-Propyl | Methyl | sek.-Butyl | H | S | O |

| $R^1$ | $R^3$ | $R^4$ | $R^5$ | X | Y |
|---|---|---|---|---|---|
| iso-Propyl | Methyl | n-Pentyl | H | S | O |
| iso-Propyl | Methyl | 2-Pentyl | H | S | O |
| iso-Propyl | Methyl | 3-Pentyl | H | S | O |
| iso-Propyl | Methyl | n-Hexyl | H | S | O |
| iso-Propyl | Methyl | 2-Hexyl | H | S | O |
| iso-Propyl | Methyl | 3-Hexyl | H | S | O |
| Chlor | H | Methyl | $-N=C(CH_3)_2$ | S | O |
| Chlor | H | Ethyl | $-N=C(CH_3)_2$ | S | O |
| Chlor | H | n-Propyl | $-N=C(CH_3)_2$ | S | O |
| Chlor | H | iso-Propyl | $-N=C(CH_3)_2$ | S | O |
| Chlor | H | n-Butyl | $-N=C(CH_3)_2$ | S | O |
| Chlor | H | iso-Butyl | $-N=C(CH_3)_2$ | S | O |
| Methyl | H | sek.-Butyl | $-N=C(CH_3)_2$ | S | O |
| Methyl | H | n-Pentyl | $-N=C(CH_3)_2$ | S | O |
| Methyl | H | 2-Pentyl | $-N=C(CH_3)_2$ | S | O |
| Methyl | H | 3-Pentyl | $-N=C(CH_3)_2$ | S | O |
| Methyl | H | n-Hexyl | $-N=C(CH_3)_2$ | S | O |
| Methyl | H | 2-Hexyl | $-N=C(CH_3)_2$ | S | O |
| iso-Propyl | H | 3-Hexyl | $-N=C(CH_3)_2$ | S | O |
| iso-Propyl | H | 2-Methyl-2-pentyl | $-N=C(CH_3)_2$ | S | O |
| iso-Propyl | H | cyclo-Proplymethyl | $-N=C(CH_3)_2$ | S | O |
| iso-Propyl | H | cyclo-Butyl | $-N=C(CH_3)_2$ | S | O |
| iso-Propyl | H | cyclo-Pentyl | $-N=C(CH_3)_2$ | S | O |
| iso-Propyl | H | cyclo-Hexyl | $-N=C(CH_3)_2$ | S | O |
| cyclo-Propyl | H | 1-Methylcyclohexyl | $-N=C(CH_3)_2$ | S | O |

| $R^1$ | $R^3$ | $R^4$ | $R^5$ | X | Y |
|---|---|---|---|---|---|
| cyclo-Propyl | H | 3-Trifluormethylcyclohexyl | $-N=C(CH_3)_2$ | S | O |
| cyclo-Propyl | H | Allyl | $-N=C(CH_3)_2$ | S | O |
| cyclo-Propyl | H | 1-Buten-3-yl | $-N=C(CH_3)_2$ | S | O |
| cyclo-Propyl | H | Crotyl | $-N=C(CH_3)_2$ | S | O |
| cyclo-Propyl | H | Propargyl | $-N=C(CH_3)_2$ | S | O |
| Allyl | H | 1-Butin-3-yl | $-N=C(CH_3)_2$ | S | O |
| Allyl | H | 3-Methyl-1-butin-3-yl | $-N=C(CH_3)_2$ | S | O |
| Allyl | H | 2-Pentin-4-yl | $-N=C(CH_3)_2$ | S | O |
| Allyl | H | Benzyl | $-N=C(CH_3)_2$ | S | O |
| Allyl | H | 2-Phenylethyl | $-N=C(CH_3)_2$ | S | O |
| Allyl | H | 2-Methylthioethyl | $-N=C(CH_3)_2$ | S | O |
| Ethinyl | H | 2-Chlorethyl | $-N=C(CH_3)_2$ | S | O |
| Ethinyl | H | 2-Methoxyethyl | $-N=C(CH_3)_2$ | S | O |
| Ethinyl | H | 2-(n,N-Dimethylamino)ethyl | $-N=C(CH_3)_2$ | S | O |
| Ethinyl | H | Phenyl | $-N=C(CH_3)_2$ | S | O |
| Ethinyl | H | 2-$CH_3$-Phenyl | $-N=C(CH_3)_2$ | S | O |
| Ethinyl | H | 4-$CH_3$-Phenyl | $-N=C(CH_3)_2$ | S | O |
| Methoxy | H | 2,4-($CH_3$,$CH_3$)-Phenyl | $-N=C(CH_3)_2$ | S | O |
| Methoxy | H | 2,3,5-($CH_3$,$CH_3$,$CH_3$)-Phenyl | $-N=C(CH_3)_2$ | S | O |
| Methoxy | H | 3-$CF_3$-Phenyl | $-N=C(CH_3)_2$ | S | O |
| Methoxy | H | 3-F-Phenyl | $-N=C(CH_3)_2$ | S | O |
| Methoxy | H | 2-Cl-Phenyl | $-N=C(CH_3)_2$ | S | O |
| Methoxy | H | 4-Cl-Phenyl | $-N=C(CH_3)_2$ | S | O |
| 4-Cl-Phenoxy | H | 2,4-(F,F)-Phenyl | $-N=C(CH_3)_2$ | S | O |
| 4-Cl-Phenoxy | H | 2,3,5-(Cl,Cl,Cl)-Phenyl | $-N=C(CH_3)_2$ | S | O |

| $R^1$ | $R^3$ | $R^4$ | $R^5$ | X | Y |
|---|---|---|---|---|---|
| 4-Cl-Phenoxy | H | 2-CN-Phenyl | $-N=C(CH_3)_2$ | S | O |
| 4-Cl-Phenoxy | H | $2-OCH_3$-Phenyl | $-N=C(CH_3)_2$ | S | O |
| 4-Cl-Phenoxy | H | $2,3-(OCH_3,OCH_3)$-Phenyl | $-N=C(CH_3)_2$ | S | O |
| 4-Cl-Phenoxy | H | $3,4,5-(OCH_3,OCH_3,OCH_3)$-Phenyl | $-N=C(CH_3)_2$ | S | O |
| Phenylthio | H | $3-OCF_3$-Phenyl | $-N=C(CH_3)_2$ | S | O |
| Phenylthio | H | $4-OCF_2CHF_2$-Phenyl | $-N=C(CH_3)_2$ | S | O |
| Phenylthio | H | $2-SCH_3$-Phenyl | $-N=C(CH_3)_2$ | S | O |
| Phenylthio | H | $2,4-(SCH_3,SCH_3)$-Phenyl | $-N=C(CH_3)_2$ | S | O |
| Phenylthio | H | $2-SCF_3$-Phenyl | $-N=C(CH_3)_2$ | S | O |
| Phenylthio | H | $4-NO_2$-Phenyl | $-N=C(CH_3)_2$ | S | O |

| $R^1$ | $R^3$ | $R^4$ | $R^5$ | X | Y |
|---|---|---|---|---|---|
| iso-Propyl | Methyl | sek.-Butyl | $-N=C(CH_3)_2$ | S | O |
| iso-Propyl | Methyl | n-Pentyl | $-N=C(CH_3)_2$ | S | O |
| iso-Propyl | Methyl | 2-Pentyl | $-N=C(CH_3)_2$ | S | O |
| iso-Propyl | Methyl | 3-Pentyl | $-N=C(CH_3)_2$ | S | O |
| iso-Propyl | Methyl | n-Hexyl | $-N=C(CH_3)_2$ | S | O |
| iso-Propyl | Methyl | 2-Hexyl | $-N=C(CH_3)_2$ | S | O |
| iso-Propyl | Methyl | 3-Hexyl | $-N=C(CH_3)_2$ | S | O |
| Methyl | H | tert.-Butyl | 2,4-(Cl,Cl)-Phenyl | S | S |
| Methyl | H | tert.-Butyl | 2-Pyridyl | S | S |
| Methyl | H | tert.-Butyl | Ethyl | S | S |
| Methyl | H | tert.-Butyl | iso-Propyl | S | S |
| Methyl | H | tert.-Butyl | Butyl | S | S |
| Methyl | H | tert.-Butyl | tert.-Butyl | S | S |
| Methyl | H | tert.-Butyl | Phenyl | S | S |
| iso-Phenyl | H | tert.-Butyl | 4-F-Phenyl | S | S |
| iso-Phenyl | H | tert.-Butyl | $3-CF_3$-Phenyl | S | S |
| iso-Propyl | H | tert.-Butyl | 2,4-(Cl,Cl)-Phenyl | S | S |
| iso-Propyl | H | tert.-Butyl | 2-Pyridyl | S | S |
| iso-Propyl | H | tert.-Butyl | Methyl | S | S |
| iso-Propyl | H | tert.-Butyl | Ethyl | S | S |
| iso-Propyl | H | tert.-Butyl | iso-Propyl | S | S |
| cyclo-Propyl | H | tert.-Butyl | Butyl | S | S |

| $R^1$ | $R^3$ | $R^4$ | $R^5$ | X | Y |
|---|---|---|---|---|---|
| cyclo-Propyl | H | tert.-Butyl | tert.-Butyl | S | S |
| cyclo-Propyl | H | tert.-Butyl | Phenyl | S | S |
| cyclo-Propyl | H | tert.-Butyl | 4-F-Phenyl | S | S |
| cyclo-Propyl | H | tert.-Butyl | 3-$CF_3$-Phenyl | S | S |
| cyclo-Propyl | H | tert.-Butyl | 2,4-(Cl,Cl)-Phenyl | S | S |
| cyclo-Propyl | H | tert.-Butyl | 2-Pyridyl | S | S |
| Allyl | H | tert.-Butyl | Methyl | S | S |
| Ally | H | tert.-Butyl | Ethyl | S | S |
| Ally | H | tert.-Butyl | iso-Propyl | S | S |
| Allyl | H | tert.-Butyl | Butyl | S | S |
| Allyl | H | tert.-Butyl | tert.-Butyl | S | S |
| Allyl | H | tert.-Butyl | Phenyl | S | S |
| Methoxy | H | tert.-Butyl | Methyl | S | S |
| Methoxy | H | tert.-Butyl | Ethyl | S | S |
| Methoxy | H | tert.-Butyl | iso-Propyl | S | S |
| Methoxy | H | tert.-Butyl | Butyl | S | S |
| Methoxy | H | tert.-Butyl | tert.-Butyl | S | S |
| Methoxy | H | tert.-Butyl | Phenyl | S | S |
| Methoxy | H | tert.-Butyl | 4-F-Phenyl | S | S |
| 4-Cl-Phenoxy | H | tert.-Butyl | 3-$CF_3$-Phenyl | S | S |
| 4-Cl-Phenoxy | H | tert.-Butyl | 2,4-(Cl,Cl)-Phenyl | S | S |
| 4-Cl-Phenoxy | H | tert.-Butyl | 2-Pyridyl | S | S |
| 4-Cl-Phenoxy | H | tert.-Butyl | Methyl | S | S |
| 4-Cl-Phenoxy | H | tert.-Butyl | Ethyl | S | S |
| 4-Cl-Phenoxy | H | tert.-Butyl | iso-Propyl | S | S |

| $R^1$ | $R^3$ | $R^4$ | $R^5$ | X | Y |
|---|---|---|---|---|---|
| 4-Cl-Phenoxy | H | tert.-Butyl | Butyl | S | S |
| Methyl | H | cyclo-Propyl | 2,4-(Cl,Cl)-Phenyl | S | S |
| Methyl | H | cyclo-Propyl | 2-Pyridyl | S | S |
| Methyl | H | cyclo-Propyl | Ethyl | S | S |
| Methyl | H | cyclo-Propyl | iso-Propyl | S | S |
| Methyl | H | cyclo-Propyl | Butyl | S | S |
| Methyl | H | cyclo-Propyl | tert.-Butyl | S | S |
| Methyl | H | cyclo-Propyl | Phenyl | S | S |
| iso-Propyl | H | cyclo-Propyl | 4-F-Phenyl | S | S |
| iso-Proply | H | cyclo-Propyl | 3-$CF_3$-Phenyl | S | S |
| iso-Propyl | H | cyclo-Propyl | 2,4-(Cl,Cl)-Phenyl | S | S |
| iso-Propyl | H | cyclo-Propyl | 2-Pyridyl | S | S |

| $R^1$ | $R^3$ | $R^4$ | $R^5$ | X | Y |
|---|---|---|---|---|---|
| iso-Propyl | H | cyclo-Propyl | Methyl | S | S |
| iso-Propyl | H | cyclo-Propyl | Ethyl | S | S |
| iso-Propyl | H | cyclo-Propyl | iso-Propyl | S | S |
| cyclo-Propyl | H | cyclo-Propyl | Butyl | S | S |
| cyclo-Propyl | H | cyclo-Propyl | tert.-Butyl | S | S |
| cyclo-Propyl | H | cyclo-Propyl | Phenyl | S | S |
| cyclo-Propyl | H | cyclo-Propyl | 4-F-Phenyl | S | S |
| cyclo-Propyl | H | cyclo-Propyl | 3-$CF_3$-Phenyl | S | S |
| cyclo-Propyl | H | cyclo-Propyl | 2,4-(Cl,Cl)-Phenyl | S | S |
| cyclo-Propyl | H | cyclo-Propyl | 2-Pyridyl | S | S |
| Allyl | H | cyclo-Propyl | Methyl | S | S |
| Allyl | H | cyclo-Propyl | Ethyl | S | S |
| Allyl | H | cyclo-Propyl | iso-Propyl | S | S |
| Allyl | H | cyclo-Propyl | Butyl | S | S |
| Allyl | H | cyclo-Propyl | tert.-Butyl | S | S |
| Allyl | H | cyclo-Propyl | Phenyl | S | S |
| Methoxy | H | cyclo-Propyl | Methyl | S | S |
| Methoxy | H | cyclo-Propyl | Ethyl | S | S |
| Methoxy | H | cyclo-Propyl | iso-Propyl | S | S |
| Methoxy | H | cyclo-Propyl | Butyl | S | S |
| Methoxy | H | cyclo-Propyl | tert.-Butyl | S | S |
| Methoxy | H | cyclo-Propyl | Phenyl | S | S |
| Methoxy | H | cyclo-Propyl | 4-F-Phenyl | S | S |
| 4-Cl-Phenoxy | H | cyclo-Propyl | 3-$CF_3$-Phenyl | S | S |
| 4-Cl-Phenoxy | H | cyclo-Propyl | 2,4-(Cl,Cl)-Phenyl | S | S |

| $R^1$ | $R^3$ | $R^4$ | $R^5$ | X | Y |
|---|---|---|---|---|---|
| 4-Cl-Phenoxy | H | cyclo-Propyl | 2-Pyridyl | S | S |
| 4-Cl-Phenoxy | H | cyclo-Propyl | Methyl | S | S |
| 4-Cl-Phenoxy | H | cyclo-Propyl | Ethyl | S | S |
| 4-Cl-Phenoxy | H | cyclo-Propyl | iso-Propyl | S | S |
| 4-Cl-Phenoxy | H | cyclo-Propyl | Butyl | S | S |
| Chlor | H | tert.-Butyl | 4-Hydroxy-2-butinyl | O | O |
| Chlor | H | tert.-Butyl | $N=C(C_2H_5)_2$ | O | O |
| Chlor | H | tert.-Butyl | $N=C(cyclo-C_3H_5)_2$ | O | O |
| Chlor | H | tert.-Butyl | 2-Butanimino | O | O |
| Chlor | H | tert.-Butyl | Cyclohexanimino | O | O |
| Chlor | H | tert.-Butyl | Cyclooctanimino | O | O |
| Methyl | H | tert.-Butyl | $N=CH-C_6H_5$ | O | O |

| $R^1$ | $R^3$ | $R^4$ | $R^5$ | X | Y |
|---|---|---|---|---|---|
| Methyl | H | tert.-Butyl | 2-Furyl-methanimino | O | O |
| Methyl | H | tert.-Butyl | $CH_2CH_2N(CH_3)_2$ | O | O |
| Methyl | H | tert.-Butyl | $CH_2CH_2N^+(CH_3)_3I^-$ | O | O |
| Methyl | H | tert.-Butyl | $CH_2CF_3$ | O | O |
| Methyl | H | tert.-Butyl | $CH_2CH_2Cl$ | O | O |
| Methyl | H | tert.-Butyl | $CH_2CH_2CN$ | O | O |
| iso-Propyl | H | tert.-Butyl | $CH_2CCl_3$ | O | O |
| iso-Propyl | H | tert.-Butyl | $CH_2CH_2Si(CH_3)_3$ | O | O |
| iso-Propyl | H | tert.-Butyl | $CH_2CH_2O\text{-}N{=}C(CH_3)_2$ | O | O |
| iso-Propyl | H | tert.-Butyl | $CH_2PO(OC_2H_5)_2$ | O | O |
| iso-Propyl | H | tert.-Butyl | $CH(CH_3)CH(OCH_3)_2$ | O | O |
| iso-Propyl | H | tert.-Butyl | $CH_2\text{-}CON(C_2H_5)_2$ | O | O |
| iso-Propyl | H | tert.-Butyl | Benzyl | O | O |
| cyclo-Propyl | H | tert.-Butyl | 2,4-(Cl,Cl)-Benzyl | O | O |
| cyclo-Propyl | H | tert.-Butyl | 3-Pyridyl-methyl | O | O |
| cyclo-Propyl | H | tert.-Butyl | 2-Thienyl-methyl | O | O |
| cyclo-Propyl | H | tert.-Butyl | 2-Tetrahydrofuranyl-methyl | O | O |
| cyclo-Propyl | H | tert.-Butyl | 2-Furanyl-methyl | O | O |
| cyclo-Propyl | H | tert.-Butyl | 2-Pyridyl-methyl | O | O |
| cyclo-Propyl | H | tert.-Butyl | Phenyl | O | O |
| Allyl | H | tert.-Butyl | 4-F-Phenyl | O | O |
| Allyl | H | tert.-Butyl | 4-Trifluormethylphenyl | O | O |
| Allyl | H | tert.-Butyl | $2\text{-}NO_2\text{-}4\text{-}F$-Phenyl | O | O |
| Allyl | H | tert.-Butyl | $3,5\text{-}(CF_3,CF_3)$-Phenyl | O | O |
| Allyl | H | tert.-Butyl | $4\text{-}OCH_3$-Phenyl | O | O |

| $R^1$ | $R^3$ | $R^4$ | $R^5$ | X | Y |
|---|---|---|---|---|---|
| Allyl | H | tert.-Butyl | 4-$OCF_3$-Phenyl | O | O |
| Allyl | H | tert.-Butyl | 4-$NHCOCH_3$-Phenyl | O | O |
| Ethinyl | H | tert.-Butyl | 2-Tetrahydropyranyl | O | O |
| Ethinyl | H | tert.-Butyl | 2-Tetrahydropyranyl | O | O |
| Ethinyl | H | tert.-Butyl | 1-Benzotriazolyl | O | O |
| Ethinyl | H | tert.-Butyl | Methyl | O | O |
| Ethinyl | H | tert.-Butyl | Ethyl | O | O |
| Ethinyl | H | tert.-Butyl | n-Propyl | O | O |
| Ethinyl | H | tert.-Butyl | iso-Propyl | O | O |
| Methoxy | H | tert.-Butyl | n-Butyl | O | O |
| Methoxy | H | tert.-Butyl | iso-Butyl | O | O |
| Methoxy | H | tert.-Butyl | sek.-Butyl | O | O |
| Methoxy | H | tert.-Butyl | tert.-Butyl | O | O |
| Methoxy | H | tert.-Butyl | cyclo-Hexyl | O | O |
| Methoxy | H | tert.-Butyl | Cyclopropylmethyl | O | O |
| Methoxy | H | tert.-Butyl | Ethoxymethyl | O | O |
| 4-Cl-Phenoxy | H | tert.-Butyl | 2-Methooxy-ethoxy-methyl | O | O |
| 4-Cl-Phenoxy | H | tert.-Butyl | Benzyloxymethyl | O | O |
| 4-Cl-Phenoxy | H | tert.-Butyl | (4-Brombenzoyl)-methyl | O | O |
| 4-Cl-Phenoxy | H | tert.-Butyl | (4-Methoxybenzoyl)-methyl | O | O |
| 4-Cl-Phenoxy | H | tert.-Butyl | Phthalimidomethyl | O | O |
| 4-Cl-Phenoxy | H | tert.-Butyl | Methylthiomethyl | O | O |
| 4-Cl-Phenoxy | H | tert.-Butyl | 2-Thiomethyl-ethyl | O | O |
| Phenylthio | H | tert.-Butyl | $CH(C_6H_5)COOCH_3$ | O | O |
| Phenylthio | H | tert.-Butyl | Phenylethyl | O | O |

| $R^1$ | $R^3$ | $R^4$ | $R^5$ | X | Y |
|---|---|---|---|---|---|
| Phenylthio | H | tert.-Butyl | 4-F-Phenylethyl | O | O |
| Phenylthio | H | tert.-Butyl | Phthalimido | O | O |
| Phenylthio | H | tert.-Butyl | Tetrahydrophthalimido | O | O |
| Phenylthio | H | tert.-Butyl | Maleinimido | O | O |
| Phenylthio | H | tert.-Butyl | Succinimido | O | O |
| Chlor | H | cyclo-Propyl | 4-Hydroxy-2-butinyl | O | O |
| Chlor | H | cyclo-Propyl | $N=C(C_2H_5)_2$ | O | O |
| Chlor | H | cyclo-Propyl | $N=C(cyclo-C_3H_5)_2$ | O | O |
| Chlor | H | cyclo-Propyl | 2-Butanimino | O | O |
| Chlor | H | cyclo-Propyl | Cyclohexanimino | O | O |
| Chlor | H | cyclo-Propyl | Cyclooctanimino | O | O |
| Methyl | H | cyclo-Propyl | $N=CH-C_6H_5$ | O | O |
| Methyl | H | cyclo-Propyl | 2-Furyl-methanimino | O | O |

| $R^1$ | $R^3$ | $R^4$ | $R^5$ | X | Y |
|---|---|---|---|---|---|
| Methyl | H | cyclo-Propyl | $CH_2CH_2N(CH_3)_2$ | O | O |
| Methyl | H | cyclo-Propyl | $CH_2CH_2N^+(CH_3)_3I^-$ | O | O |
| Methyl | H | cyclo-Propyl | $CH_2CF_3$ | O | O |
| Methyl | H | cyclo-Propyl | $CH_2CH_2Cl$ | O | O |
| Methyl | H | cyclo-Propyl | $CH_2CH_2CN$ | O | O |
| iso-Propyl | H | cyclo-Propyl | $CH_2CCl_3$ | O | O |
| iso-Propyl | H | cyclo-Propyl | $CH_2CH_2Si(CH_3)_3$ | O | O |
| iso-Propyl | H | cyclo-Propyl | $CH_2CH_2O-N=C(CH_3)_2$ | O | O |
| iso-Propyl | H | cyclo-Propyl | $CH_2PO(OC_2H_5)_2$ | O | O |
| iso-Propyl | H | cyclo-Propyl | $CH(CH_3)CH(OCH_3)_2$ | O | O |
| iso-Propyl | H | cyclo-Propyl | $CH_2-CON(C_2H_5)_2$ | O | O |
| iso-Propyl | H | cyclo-Propyl | Benzyl | O | O |
| cyclo-Propyl | H | cyclo-Propyl | 2,4-(Cl,Cl)-Benzyl | O | O |
| cyclo-Propyl | H | cyclo-Propyl | 3-Pyridyl-methyl | O | O |
| cyclo-Propyl | H | cyclo-Propyl | 2-Thienyl-methyl | O | O |
| cyclo-Propyl | H | cyclo-Propyl | 2-Tetrahydrofuranyl-methyl | O | O |
| cyclo-Propyl | H | cyclo-Propyl | 2-Furanyl-methyl | O | O |
| cyclo-Propyl | H | cyclo-Propyl | 2-Pyridyl-methyl | O | O |
| cyclo-Propyl | H | cyclo-Propyl | Phenyl | O | O |
| Allyl | H | cyclo-Propyl | 4-F-Phenyl | O | O |
| Allyl | H | cyclo-Propyl | 4-Trifluormethylphenyl | O | O |
| Allyl | H | cyclo-Propyl | $2-NO_2-4-F$-Phenyl | O | O |
| Allyl | H | cyclo-Propyl | $3,5-(CF_3,CF_3)$-Phenyl | O | O |
| Allyl | H | cyclo-Propyl | $4-OCH_3$-Phenyl | O | O |
| Allyl | H | cyclo-Propyl | $4-OCF_3$-Phenyl | O | O |

| $R^1$ | $R^3$ | $R^4$ | $R^5$ | X | Y |
|---|---|---|---|---|---|
| Allyl | H | cyclo-Propyl | 4-$NHCOCH_3$-Phenyl | 0 | 0 |
| Ethinyl | H | cyclo-Propyl | 2-Tetrahydropyranyl | 0 | 0 |
| Ethinyl | H | cyclo-Propyl | 2-Tetrahydropyranyl | 0 | 0 |
| Ethinyl | H | cyclo-Propyl | 1-Benzotriazolyl | 0 | 0 |
| Ethinyl | H | cyclo-Propyl | Methyl | 0 | 0 |
| Ethinyl | H | cyclo-Propyl | Ethyl | 0 | 0 |
| Ethinyl | H | cyclo-Propyl | n-Propyl | 0 | 0 |
| Ethinyl | H | cyclo-Propyl | iso-Propyl | 0 | 0 |
| Methoxy | H | cyclo-Propyl | n-Butyl | 0 | 0 |
| Methoxy | H | cyclo-Propyl | iso-Butyl | 0 | 0 |
| Methoxy | H | cyclo-Propyl | sek.-Butyl | 0 | 0 |
| Methoxy | H | cyclo-Propyl | tert.-Butyl | 0 | 0 |
| Methoxy | H | cyclo-Propyl | cyclo-Hexyl | 0 | 0 |
| Methoxy | H | cyclo-Propyl | Cyclopropylmethyl | 0 | 0 |
| Methoxy | H | cyclo-Propyl | Ethoxymethyl | 0 | 0 |
| 4-Cl-Phenoxy | H | cyclo-Propyl | 2-Methoxy-ethoxy-methyl | 0 | 0 |
| 4-Cl-Phenoxy | H | cyclo-Propyl | Benzyloxymethyl | 0 | 0 |
| 4-Cl-Phenoxy | H | cyclo-Propyl | (4-Brombenzoxyl)-methyl | 0 | 0 |
| 4-Cl-Phenoxy | H | cyclo-Propyl | (4-Methoxybenzoxyl)-methyl | 0 | 0 |
| 4-Cl-Phenoxy | H | cyclo-Propyl | Phthalimidomethyl | 0 | 0 |
| 4-Cl-Phenoxy | H | cyclo-Propyl | Methylthiomethyl | 0 | 0 |
| 4-Cl-Phenoxy | H | cyclo-Propyl | 2-Thiomethyl-ethyl | 0 | 0 |
| Phenylthio | H | cyclo-Propyl | $CH(C_6H_5)COOCH_3$ | 0 | 0 |
| Phenylthio | H | cyclo-Propyl | Phenylethyl | 0 | 0 |
| Phenylthio | H | cyclo-Propyl | 4-F-Phenylethyl | 0 | 0 |

| $R^1$ | $R^3$ | $R^4$ | $R^5$ | X | Y |
|---|---|---|---|---|---|
| Phenylthio | H | cyclo-Propyl | Phthalimido | O | O |
| Phenylthio | H | cyclo-Propyl | Tetrahydrophthalimido | O | O |
| Phenylthio | H | cyclo-Propyl | Maleinimido | O | O |
| Phenylthio | H | cyclo-Propyl | Succinimido | O | O |
| H | H | tert.-Butyl | $-N=C(CH_3)_2$ | O | O |
| F | H | tert.-Butyl | $-N=C(CH_3)_2$ | O | O |
| Cl | H | tert.-Butyl | $-N=C(CH_3)_2$ | O | O |
| Methyl | H | tert.-Butyl | $-N=C(CH_3)_2$ | O | O |
| Ethyl | H | tert.-Butyl | $-N=C(CH_3)_2$ | O | O |
| n-Propyl | H | tert.-Butyl | $-N=C(CH_3)_2$ | O | O |
| iso-Propyl | H | tert.-Butyl | $-N=C(CH_3)_2$ | O | O |
| n-Butyl | H | tert.-Butyl | $-N=C(CH_3)_2$ | O | O |
| iso-Butyl | H | tert.-Butyl | $-N=C(CH_3)_2$ | O | O |

| $R^1$ | $R^3$ | $R^4$ | $R^5$ | X | Y |
|---|---|---|---|---|---|
| sek.-Butyl | H | tert.-Butyl | $-N=C(CH_3)_2$ | O | O |
| tert.-Butyl | H | tert.-Butyl | $-N=C(CH_3)_2$ | O | O |
| cyclo-Propyl | H | tert.-Butyl | $-N=C(CH_3)_2$ | O | O |
| cyclo-Pentyl | H | tert.-Butyl | $-N=C(CH_3)_2$ | O | O |
| 1-Methylcyclopropyl | H | tert.-Butyl | $-N=C(CH_3)_2$ | O | O |
| Trifluormethyl | H | tert.-Butyl | $-N=C(CH_3)_2$ | O | O |
| Chlordifluormethyl | H | tert.-Butyl | $-N=C(CH_3)_2$ | O | O |
| Pentafluorethyl | H | tert.-Butyl | $-N=C(CH_3)_2$ | O | O |
| iso-Propoxy | H | tert.-Butyl | $-N=C(CH_3)_2$ | O | O |
| Methoxymethyl | H | tert.-Butyl | $-N=C(CH_3)_2$ | O | O |
| 1-Methylmethoxymethyl | H | tert.-Butyl | $-N=C(CH_3)_2$ | O | O |
| 1-Methylmethoxyethyl | H | tert.-Butyl | $-N=C(CH_3)_2$ | O | O |
| Ethoxymethyl | H | tert.-Butyl | $-N=C(CH_3)_2$ | O | O |
| Vinyl | H | tert.-Butyl | $-N=C(CH_3)_2$ | O | O |
| Allyl | H | tert.-Butyl | $-N=C(CH_3)_2$ | O | O |
| Methallyl | H | tert.-Butyl | $-N=C(CH_3)_2$ | O | O |
| Crotyl | H | tert.-Butyl | $-N=C(CH_3)_2$ | O | O |
| Ethinyl | H | tert.-Butyl | $-N=C(CH_3)_2$ | O | O |
| Propargyl | H | tert.-Butyl | $-N=C(CH_3)_2$ | O | O |
| Phenylethinyl | H | tert.-Butyl | $-N=C(CH_3)_2$ | O | O |
| Methoxy | H | tert.-Butyl | $-N=C(CH_3)_2$ | O | O |

| $R^1$ | $R^3$ | $R^4$ | $R^5$ | X | Y |
|---|---|---|---|---|---|
| Ethoxy | H | tert.-Butyl | $-N=C(CH_3)_2$ | O | O |
| Trifluormethoxy | H | tert.-Butyl | $-N=C(CH_3)_2$ | O | O |
| Methylthio | H | tert.-Butyl | $-N=C(CH_3)_2$ | O | O |
| Trifluormethylthio | H | tert.-Butyl | $-N=C(CH_3)_2$ | O | O |
| Phenoxy | H | tert.-Butyl | $-N=C(CH_3)_2$ | O | O |
| 4-Cl-Phenoxy | H | tert.-Butyl | $-N=C(CH_3)_2$ | O | O |
| 2,4-(Cl,Cl)-Phenoxy | H | tert.-Butyl | $-N=C(CH_3)_2$ | O | O |
| 4-$CF_3$-Phenoxy | H | tert.-Butyl | $-N=C(CH_3)_2$ | O | O |
| 2-F-Phenylthio | H | tert.-Butyl | $-N=C(CH_3)_2$ | O | O |

| $R^1$ | $R^3$ | $R^4$ | $R^5$ | X | Y |
|---|---|---|---|---|---|
| H | H | cyclo-Propyl | $-N=C(CH_3)_2$ | O | O |
| F | H | cyclo-Propyl | $-N=C(CH_3)_2$ | O | O |
| Cl | H | cyclo-Propyl | $-N=C(CH_3)_2$ | O | O |
| Methyl | H | cyclo-Propyl | $-N=C(CH_3)_2$ | O | O |
| Ethyl | H | cyclo-Propyl | $-N=C(CH_3)_2$ | O | O |
| n-Propyl | H | cyclo-Propyl | $-N=C(CH_3)_2$ | O | O |
| iso-Propyl | H | cyclo-Propyl | $-N=C(CH_3)_2$ | O | O |
| n-Butyl | H | cyclo-Propyl | $-N=C(CH_3)_2$ | O | O |
| iso-Butyl | H | cyclo-Propyl | $-N=C(CH_3)_2$ | O | O |
| sek.-Butyl | H | cyclo-Propyl | $-N=C(CH_3)_2$ | O | O |

| $R^1$ | $R^3$ | $R^4$ | $R^5$ | X | Y |
|---|---|---|---|---|---|
| tert.-Butyl | H | cyclo-Propyl | $-N=C(CH_3)_2$ | O | O |
| cyclo-Propyl | H | cyclo-Propyl | $-N=C(CH_3)_2$ | O | O |
| cyclo-Pentyl | H | cyclo-Propyl | $-N=C(CH_3)_2$ | O | O |
| 1-Methylcyclopropyl | H | cyclo-Propyl | $-N=C(CH_3)_2$ | O | O |
| Trifluormethyl | H | cyclo-Propyl | $-N=C(CH_3)_2$ | O | O |
| Chlordifluormethyl | H | cyclo-Propyl | $-N=C(CH_3)_2$ | O | O |
| Pentafluorethyl | H | cyclo-Propyl | $-N=C(CH_3)_2$ | O | O |
| iso-Propoxy | H | cyclo-Propyl | $-N=C(CH_3)_2$ | O | O |
| Methoxymethyl | H | cyclo-Propyl | $-N=C(CH_3)_2$ | O | O |
| 1-Methylmethoxymethyl | H. | cyclo-Propyl | $-N=C(CH_3)_2$ | O | O |
| 1-Methylmethoxyethyl | H | cyclo-Propyl | $-N=C(CH_3)_2$ | O | O |
| Ethoxymethyl | H | cyclo-Propyl | $-N=C(CH_3)_2$ | O | O |
| Vinyl | H | cyclo-Propyl | $-N=C(CH_3)_2$ | O | O |
| Allyl | H | cyclo-Propyl | $-N=C(CH_3)_2$ | O | O |
| Methallyl | H | cyclo-Propyl | $-N=C(CH_3)_2$ | O | O |
| Crotyl | H | cyclo-Propyl | $-N=C(CH_3)_2$ | O | O |
| Ethinyl | H | cyclo-Propyl | $-N=C(CH_3)_2$ | O | O |
| Propargyl | H | cyclo-Propyl | $-N=C(CH_3)_2$ | O | O |
| Phenylethinyl | H | cyclo-Propyl | $-N=C(CH_3)_2$ | O | O |
| Methoxy | H | cyclo-Propyl | $-N=C(CH_3)_2$ | O | O |
| Ethoxy | H | cyclo-Propyl | $-N=C(CH_3)_2$ | O | O |

| $R^1$ | $R^3$ | $R^4$ | $R^5$ | X | Y |
|---|---|---|---|---|---|
| Trifluormethoxy | H | cyclo-Propyl | $-N=C(CH_3)_2$ | O | O |
| Methylthio | H | cyclo-Propyl | $-N=C(CH_3)_2$ | O | O |
| Trifluormethylthio | H | cyclo-Propyl | $-N=C(CH_3)_2$ | O | O |
| Phenoxy | H | cyclo-Propyl | $-N=C(CH_3)_2$ | O | O |
| 4-Cl-Phenoxy | H | cyclo-Propyl | $-N=C(CH_3)_2$ | O | O |
| 2,4-(Cl,Cl)-Phenoxy | H | cyclo-Propyl | $-N=C(CH_3)_2$ | O | O |
| 4-$CF_3$-Phenoxy | H | cyclo-Propyl | $-N=C(CH_3)_2$ | O | O |
| 2-F-Phenylthio | H | cyclo-Propyl | $-N=C(CH_3)_2$ | O | O |

| $R^1$ | $R^3$ | $R^4$ | $R^5$ | X | Y |
|---|---|---|---|---|---|
| Chlor | H | Methyl | H | O | O |
| Chlor | H | Ethyl | H | O | O |
| Chlor | H | n-Propyl | H | O | O |
| Chlor | H | iso-Propyl | H | O | O |
| Chlor | H | n-Butyl | H | O | O |
| Chlor | H | iso-Butyl | H | O | O |
| Methyl | H | sek.-Butyl | H | O | O |
| Methyl | H | n-Pentyl | H | O | O |
| Methyl | H | 2-Pentyl | H | O | O |
| Methyl | H | 3-Pentyl | H | O | O |
| Methyl | H | n-Hexyl | H | O | O |

| $R^1$ | $R^3$ | $R^4$ | $R^5$ | X | Y |
|---|---|---|---|---|---|
| Methyl | H | 2-Hexyl | H | O | O |
| iso-Propyl | H | 3-Hexyl | H | O | O |
| iso-Propyl | H | 2-Methyl-2-pentyl | H | O | O |
| iso-Propyl | H | cyclo-Propylmethyl | H | O | O |
| iso-Propyl | H | cyclo-Butyl | H | O | O |
| iso-Propyl | H | cyclo-Pentyl | H | O | O |
| iso-Propyl | H | cyclo-Hexyl | H | O | O |
| cyclo-Propyl | H | 1-Methylcyclohexyl | H | O | O |
| cyclo-Propyl | H | 3-Trifluormethylcyclohexyl | H | O | O |
| cyclo-Propyl | H | Allyl | H | O | O |
| cyclo-Propyl | H | 1-Buten-3-yl | H | O | O |
| cyclo-Propyl | H | Crotyl | H | O | O |
| cyclo-Propyl | H | Propargyl | H | O | O |
| Allyl | H | 1-Butin-3-yl | H | O | O |
| Allyl | H | 3-Methyl-1-butin-3-yl | H | O | O |
| Allyl | H | 2-Pentin-4-yl | H | O | O |
| Allyl | H | Benzyl | H | O | O |
| Allyl | H | 2-Phenylethyl | H | O | O |
| Allyl | H | 2-Methylthioethyl | H | O | O |
| Ethinyl | H | 2-Chlorethyl | H | O | O |
| Ethinyl | H | 2-Methoxyethyl | H | O | O |
| Ethinyl | H | 2-(N,N-Dimethylamino)ethyl | H | O | O |
| Ethinyl | H | Phenyl | H | O | O |
| Ethinyl | H | 2-$CH_3$-Phenyl | H | O | O |
| Ethinyl | H | 4-$CH_3$-Phenyl | H | O | O |

| $R^1$ | $R^3$ | $R^4$ | $R^5$ | X | Y |
|---|---|---|---|---|---|
| Methoxy | H | 2,4-($CH_3$,$CH_3$)-Phenyl | H | O | O |
| Methoxy | H | 2,3,5-($CH_3$,$CH_3$,$CH_3$)-Phenyl | H | O | O |
| Methoxy | H | 3-$CF_3$-Phenyl | H | O | O |
| Methoxy | H | 3-F-Phenyl | H | O | O |
| Methoxy | H | 2-Cl-Phenyl | H | O | O |
| Methoxy | H | 4-Cl-Phenyl | H | O | O |
| 4-Cl-Phenoxy | H | 2,4-(F,F)-Phenyl | H | O | O |
| 4-Cl-Phenoxy | H | 2,3,5-(Cl,Cl,Cl)-Phenyl | H | O | O |
| 4-Cl-Phenoxy | H | 2-CN-Phenyl | H | O | O |
| 4-Cl-Phenoxy | H | 2-$OCH_3$-Phenyl | H | O | O |
| 4-Cl-Phenoxy | H | 2,3-($OCH_3$,$OCH_3$)-Phenyl | H | O | O |
| 4-Cl-Phenoxy | H | 3,4,5-($OCH_3$,$OCH_3$,$OCH_3$)-Phenyl | H | O | O |
| Phenylthio | H | 3-$OCF_3$-Phenyl | H | O | O |
| Phenylthio | H | 4-$OCF_2CHF_2$-Phenyl | H | O | O |
| Phenylthio | H | 2-$SCH_3$-Phenyl | H | O | O |
| Phenylthio | H | 2,4-($SCH_3$,$SCH_3$)-Phenyl | H | O | O |
| Phenylthio | H | 2-$SCF_3$-Phenyl | H | O | O |
| Phenylthio | H | 4-$NO_2$-Phenyl | H | O | O |
| | | | | O | O |

| $R^1$ | $R^3$ | $R^4$ | $R^5$ | X | Y |
|---|---|---|---|---|---|
| iso-Propyl | Methyl | sek.-Butyl | H | O | O |
| iso-Propyl | Methyl | n-Pentyl | H | O | O |
| iso-Propyl | Methyl | 2-Pentyl | H | O | O |
| iso-Pentyl | Methyl | 3-Pentyl | H | O | O |
| iso-Propyl | Methyl | n-Hexyl | H | O | O |
| iso-Propyl | Methyl | 2-Hexyl | H | O | O |
| iso-Propyl | Methyl | 3-Hexyl | H | O | O |
| Chlor | H | Methyl | $-N=C(CH_3)_2$ | O | O |
| Chlor | H | Ethyl | $-N=C(CH_3)_2$ | O | O |
| Chlor | H | n-Propyl | $-N=C(CH_3)_2$ | O | O |
| Chlor | H | iso-Propyl | $-N=C(CH_3)_2$ | O | O |
| Chlor | H | n-Butyl | $-N=C(CH_3)_2$ | O | O |
| Chlor | H | iso-Butyl | $-N=C(CH_3)_2$ | O | O |
| Methyl | H | sek.-Butyl | $-N=C(CH_3)_2$ | O | O |

| $R^1$ | $R^3$ | $R^4$ | $R^5$ | X | Y |
|---|---|---|---|---|---|
| Methyl | H | n-Pentyl | $-N=C(CH_3)_2$ | O | O |
| Methyl | H | 2-Pentyl | $-N=C(CH_3)_2$ | O | O |
| Methyl | H | 3-Pentyl | $-N=C(CH_3)_2$ | O | O |
| Methyl | H | n-Hexyl | $-N=C(CH_3)_2$ | O | O |
| Methyl | H | 2-Hexyl | $-N=C(CH_3)_2$ | O | O |
| iso-Propyl | H | 3-Hexyl | $-N=C(CH_3)_2$ | O | O |
| iso-Propyl | H | 2-Methyl-2-pentyl | $-N=C(CH_3)_2$ | O | O |
| iso-Propyl | H | cyclo-Propylmethyl | $-N=C(CH_3)_2$ | O | O |
| iso-Propyl | H | cyclo-Butyl | $-N=C(CH_3)_2$ | O | O |
| iso-Propyl | H | cyclo-Pentyl | $-N=C(CH_3)_2$ | O | O |
| iso-Propyl | H | cyclo-Hexyl | $-N=C(CH_3)_2$ | O | O |
| cyclo-Propyl | H | 1-Methylcyclohexyl | $-N=C(CH_3)_2$ | O | O |
| cyclo-Propyl | H | 3-Trifluormethylcyclohexyl | $-N=C(CH_3)_2$ | O | O |
| cyclo-Propyl | H | Allyl | $-N=C(CH_3)_2$ | O | O |
| cyclo-Propyl | H | 1-Buten-3-yl | $-N=C(CH_3)_2$ | O | O |
| cyclo-Propyl | H | Crotyl | $-N=C(CH_3)_2$ | O | O |
| cyclo-Propyl | H | Propargyl | $-N=C(CH_3)_2$ | O | O |
| Allyl | H | 1-Butin-3-yl | $-N=C(CH_3)_2$ | O | O |
| Allyl | H | 3-Methyl-1-butin-3-yl | $-N=C(CH_3)_2$ | O | O |
| Allyl | H | 2-Pentin-4-yl | $-N=C(CH_3)_2$ | O | O |
| Allyl | H | Benzyl | $-N=C(CH_3)_2$ | O | O |
| Allyl | H | 2-Phenylethyl | $-N=C(CH_3)_2$ | O | O |
| Allyl | H | 2-Methylthioethyl | $-N=C(CH_3)_2$ | O | O |
| Ethinyl | H | 2-Chlorethyl | $-N=C(CH_3)_2$ | O | O |
| Ethinyl | H | 2-Methoxyethyl | $-N=C(CH_3)_2$ | O | O |

| $R^1$ | $R^3$ | $R^4$ | $R^5$ | X | Y |
|---|---|---|---|---|---|
| Ethinyl | H | 2-(N,N-Dimethylamino)-ethyl | $-N=C(CH_3)_2$ | O | O |
| Ethinyl | H | Phenyl | $-N=C(CH_3)_2$ | O | O |
| Ethinyl | H | $2-CH_3$-Phenyl | $-N=C(CH_3)_2$ | O | O |
| Ethinyl | H | $4-CH_3$-Phenyl | $-N=C(CH_3)_2$ | O | O |
| Methoxy | H | $2,4-(CH_3,CH_3)$-Phenyl | $-N=C(CH_3)_2$ | O | O |
| Methoxy | H | $2,3,5-(CH_3,CH_3,CH_3)$-Phenyl | $-N=C(CH_3)_2$ | O | O |
| Methoxy | H | $3-CF_3$-Phenyl | $-N=C(CH_3)_2$ | O | O |
| Methoxy | H | 3-F-Phenyl | $-N=C(CH_3)_2$ | O | O |
| Methoxy | H | 2-Cl-Phenyl | $-N=C(CH_3)_2$ | O | O |
| Methoxy | H | 4-Cl-Phenyl | $-N=C(CH_3)_2$ | O | O |
| 4-Cl-Phenoxy | H | 2,4-(F,F)-Phenyl | $-N=C(CH_3)_2$ | O | O |
| 4-Cl-Phenoxy | H | 2,3,5-(Cl,Cl,Cl)-Phenyl | $-N=C(CH_3)_2$ | O | O |
| 4-Cl-Phenoxy | H | 2-CN-Phenyl | $-N=C(CH_3)_2$ | O | O |
| 4-Cl-Phenoxy | H | $2-OCH_3$-Phenyl | $-N=C(CH_3)_2$ | O | O |
| 4-Cl-Phenoxy | H | $2,3-(OCH_3,OCH_3)$-Phenyl | $-N=C(CH_3)_2$ | O | O |
| 4-Cl-Phenoxy | H | $3,4,5-(OCH_3,OCH_3,OCH_3)$-Phenyl | $-N=C(CH_3)_2$ | O | O |
| Phenylthio | H | $3-OCF_3$-Phenyl | $-N=C(CH_3)_2$ | O | O |
| Phenylthio | H | $4-OCF_2CHF_2$-Phenyl | $-N=C(CH_3)_2$ | O | O |
| Phenylthio | H | $2-SCH_3$-Phenyl | $-N=C(CH_3)_2$ | O | O |
| Phenylthio | H | $2,4-(SCH_3,SCH_3)$-Phenyl | $-N=C(CH_3)_2$ | O | O |
| Phenylthio | H | $2-SCF_3$-Phenyl | $-N=C(CH_3)_2$ | O | O |
| Phenylthio | H | $4-NO_2$-Phenyl | $-N=C(CH_3)_2$ | O | O |

| $R^1$ | $R^3$ | $R^4$ | $R^5$ | X | Y |
|---|---|---|---|---|---|
| iso-Propyl | Methyl | sek.-Butyl | $-N=C(CH_3)_2$ | O | O |
| iso-Propyl | Methyl | n-Pentyl | $-N=C(CH_3)_2$ | O | O |
| iso-Propyl | Methyl | 2-Pentyl | $-N=C(CH_3)_2$ | O | O |
| iso-Propyl | Methyl | 3-Pentyl | $-N=C(CH_3)_2$ | O | O |
| iso-Propyl | Methyl | n-Hexyl | $-N=C(CH_3)_2$ | O | O |
| iso-Propyl | Methyl | 2-Hexyl | $-N=C(CH_3)_2$ | O | O |
| iso-Propyl | Methyl | 3-Hexyl | $-N=C(CH_3)_2$ | O | O |
| Methyl | H | tert.-Butyl | 2,4-(Cl,Cl)-Phenyl | O | S |
| Methyl | H | tert.-Butyl | 2-Pyridyl | O | S |
| Methyl | H | tert.-Butyl | Ethyl | O | S |

| $R^1$ | $R^3$ | $R^4$ | $R^5$ | X | Y |
|---|---|---|---|---|---|
| Methyl | H | tert.-Butyl | iso-Propyl | O | S |
| Methyl | H | tert.-Butyl | Butyl | O | S |
| Methyl | H | tert.-Butyl | tert.-Butyl | O | S |
| Methyl | H | tert.-Butyl | Phenyl | O | S |
| iso-Propyl | H | tert.-Butyl | 4-F-Phenyl | O | S |
| iso-Propyl | H | tert.-Butyl | 3-$CF_3$-Phenyl | O | S |
| iso-Propyl | H | tert.-Butyl | 2,4-(Cl,Cl)-Phenyl | O | S |
| iso-Propyl | H | tert.-Butyl | 2-Pyridyl | O | S |
| iso-Propyl | H | tert.-Butyl | Methyl | O | S |
| iso-Propyl | H | tert.-Butyl | Ethyl | O | S |
| iso-propyl | H | tert.-Butyl | iso-Propyl | O | S |
| cyclo-Propyl | H | tert.-Butyl | Butyl | O | S |
| cyclo-Propyl | H | tert.-Butyl | tert.-Butyl | O | S |
| cyclo-Proply | H | tert.-Butyl | Phenyl | O | S |
| cyclo-Propyl | H | tert.-Butyl | 4-F-Phenyl | O | S |
| cyclo-Propyl | H | tert.-Butyl | 3-$CF_3$-Phenyl | O | S |
| cyclo-Propyl | H | tert.-Butyl | 2,4-(Cl,Cl)-Phenyl | O | S |
| cyclo-Propyl | H | tert.-Butyl | 2-Pyridyl | O | S |
| Allyl | H | tert.-Butyl | Methyl | O | S |
| Allyl | H | tert.-Butyl | Ethyl | O | S |
| Allyl | H | tert.-Butyl | iso-Propyl | O | S |
| Allyl | H | tert.-Butyl | Butyl | O | S |
| Allyl | H | tert.-Butyl | tert.-Butyl | O | S |
| Allyl | H | tert.-Butyl | Phenyl | O | S |
| Methoxy | H | tert.-Butyl | Methyl | O | S |

| $R^1$ | $R^3$ | $R^4$ | $R^5$ | X | Y |
|---|---|---|---|---|---|
| Methoxy | H | tert.-Butyl | Ethyl | O | S |
| Methoxy | H | tert.-Butyl | iso-Propyl | O | S |
| Methoxy | H | tert.-Butyl | Butyl | O | S |
| Methoxy | H | tert.-Butyl | tert.-Butyl | O | S |
| Methoxy | H | tert.-Butyl | Phenyl | O | S |
| Methoxy | H | tert.-Butyl | 4-F-Phenyl | O | S |
| 4-Cl-Phenoxy | H | tert.-Butyl | 3-$CF_3$-Phenyl | O | S |
| 4-Cl-Phenoxy | H | tert.-Butyl | 2,4-(Cl,Cl)-Phenyl | O | S |
| 4-Cl-Phenoxy | H | tert.-Butyl | 2-Pyridyl | O | S |
| 4-Cl-Phenoxy | H | tert.-Butyl | Methyl | O | S |
| 4-Cl-Phenoxy | H | tert.-Butyl | Ethyl | O | S |
| 4-Cl-Phenoxy | H | tert.-Butyl | iso-Propyl | O | S |
| 4-Cl-Phenoxy | H | tert.-Butyl | Butyl | O | S |

| $R^1$ | $R^3$ | $R^4$ | $R^5$ | X | Y |
|---|---|---|---|---|---|
| Methyl | H | cyclo-Propyl | 2,4-(Cl,Cl)-Phenyl | 0 | S |
| Methyl | H | cyclo-Propyl | 2-Pyridyl | 0 | S |
| Methyl | H | cyclo-Propyl | Ethyl | 0 | S |
| Methyl | H | cyclo-Propyl | iso-Propyl | 0 | S |
| Methyl | H | cyclo-Propyl | Butyl | 0 | S |
| Methyl | H | cyclo-Propyl | tert.-Butyl | 0 | S |
| Methyl | H | cyclo-Propyl | Phenyl | 0 | S |
| iso-Propyl | H | cyclo-Propyl | 4-F-Phenyl | 0 | S |
| iso-Propyl | H | cyclo-Propyl | 3-$CF_3$-Phenyl | 0 | S |
| iso-Propyl | H | cyclo-Propyl | 2,4-(Cl,Cl)-Phenyl | 0 | S |
| iso-Propyl | H | cyclo-Propyl | 2-Pyridyl | 0 | S |
| iso-Propyl | H | cyclo-Propyl | Methyl | 0 | S |
| iso-Propyl | H | cyclo-Propyl | Ethyl | 0 | S |
| iso-Propyl | H | cyclo-Propyl | iso-Propyl | 0 | S |
| cyclo-Propyl | H | cyclo-Propyl | Butyl | 0 | S |
| cyclo-Propyl | H | cyclo-Propyl | tert.-Butyl | 0 | S |
| cyclo-Propyl | H | cyclo-Propyl | Phenyl | 0 | S |
| cyclo-Propyl | H | cyclo-Propyl | 4-F-Phenyl | 0 | S |
| cyclo-Propyl | H | cyclo-Propyl | 3-$CF_3$-Phenyl | 0 | S |
| cyclo-Propyl | H | cyclo-Propyl | 2,4-(Cl,Cl)-Phenyl | 0 | S |
| cyclo-Propyl | H | cyclo-Propyl | 2-Pyridyl | 0 | S |
| Allyl | H | cyclo-Propyl | Methyl | 0 | S |
| Allyl | H | cyclo-Propyl | Ethyl | 0 | S |
| Allyl | H | cyclo-Propyl | iso-Propyl | 0 | S |

| $R^1$ | $R^3$ | $R^4$ | $R^5$ | X | Y |
|---|---|---|---|---|---|
| Allyl | H | cyclo-Propyl | Butyl | O | S |
| Allyl | H | cyclo-Propyl | tert.-Butyl | O | S |
| Allyl | H | cyclo-Propyl | Phenyl | O | S |
| Methoxy | H | cyclo-Propyl | Methyl | O | S |
| Methoxy | H | cyclo-Propyl | Ethyl | O | S |
| Methoxy | H | cyclo-Propyl | iso-Propyl | O | S |
| Methoxy | H | cyclo-Propyl | Butyl | O | S |
| Methoxy | H | cyclo-Propyl | tert.-Butyl | O | S |
| Methoxy | H | cyclo-Propyl | Phenyl | O | S |
| Methoxy | H | cyclo-Propyl | 4-F-Phenyl | O | S |
| 4-Cl-Phenoxy | H | cyclo-Propyl | $3-CF_3$-Phenyl | O | S |
| 4-Cl-Phenoxy | H | cyclo-Propyl | 2,4-(Cl,Cl)-Phenyl | O | S |
| 4-Cl-Phenoxy | H | cyclo-Propyl | 2-Pyridyl | O | S |
| 4-Cl-Phenoxy | H | cyclo-Propyl | Methyl | O | S |
| 4-Cl-Phenoxy | H | cyclo-Propyl | Ethyl | O | S |
| 4-Cl-Phenoxy | H | cyclo-Propyl | iso-Propyl | O | S |
| 4-Cl-Phenoxy | H | cyclo-Propyl | Butyl | O | S |

Die Oxazol- bzw. Thiazolcarbonsäureamide Ia' und Ib' bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen Ia' und Ib' eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen Ia und Ib können beispielsweise wie folgt formuliert werden:

I. Man vermischt 90 Gewichtsteile der Verbindung Nr. 1.003 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.010 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.004 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.011 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.011 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.003 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.004 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.010 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5, vorzugsweise 0,01 bis 2 kg/ha aktive Substanz (a.S.).

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Zur Verbreiterung des Wirkungsspektrums und zur Erzielung synergistischer Effekte können die Oxazol- bzw. Thiazolcarbonsäureamide Ia und Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen Ia und Ib allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen benutzt. Die so erhaltenen Verbindungen sind in den nachstehenden Tabellen mit physikalischen Angaben aufgeführt.

1. Verfahren zur Herstellung der Vorprodukte

Beispiel 1.1

4(5)-Ethoxycarbonyl-2-methyl-oxazol-5-(4)-carbonsäure

Zu 33,8 g (0,15 mol) 2-Methyl-oxazol-4, 5-dicarbonsäureester in 300 ml Ethanol tropfte man bei -10 °C unter $N_2$ innerhalb von 4 h eine Lösung von 6,0 g (0,15 mol) Natriumhydroxid in 150 ml Wasser und rührte 2 h bei -10 °C nach. Man engte die Lösung ein, nahm den Rückstand in 300 ml Wasser auf, stellte mit Salzsäure auf pH = 8 bis 9 ein und extrahierte zweimal mit je 300 ml Diethylether. Anschließend säuerte man mit konz. HCl auf pH = 2 an und extrahierte die wäßrige Phase viermal mit je 250 ml Dichlormethan. Die vereinigten organischen Phasen wurden über Magnesiumsulfat getrocknet und das Solvens im Vakuum abgezogen. Man erhielt 26,4 g (88 %) 4(5)-Ethoxycarbonyl-2-methyl-oxazol-5-(4)-carbonsäure als weißen Feststoff (Isomerenverhältnis: 3:1 ($^1$H-NMR, HPLC). Zu isomerenreiner 4-Ethoxycarbonyl-2-methyl-oxazol-5-carbonsäure gelangte man durch fraktionierte Kristallisation aus Cyclohexan/ Ethylacetat 2:1 oder Säulenchromatographie an Kieselgel (Lösungsmittel: Toluol, THF, Eisessig (7:3:1). $^1$H-NMR (250 MHz, $D_6$-DMSO); Hauptisomer: $\delta$ = 1,28 (t; 3H), 2,52 (s; 3H), 4,30 (q; 2H), 14,00 (bs; 1H).

Beispiel 1.2

4-Ethoxycarbonyl-2-methylthio-thiazol-5-carbonsäure

Eine Lösung von 7,00 g (25 mmol) 2-Methylthio-thiazol-4,5-dicarbonsäurediethylester in 100 ml Ethanol/Wasser (2:1) wurde bei Raumtemperatur innerhalb von einer Stunde mit einer Lösung von 1,10 g (27,5 mmol) Natriumhydroxid in 10 ml Wasser versetzt. Man rührte eine Stunde nach, entfernte dann das Lösungsmittelgemisch im Vakuum, nahm den Rückstand mit 100 ml Wasser auf, extrahierte einmal mit 50 ml Diethylether und säuerte die wäßrige Phase mit konzentrierter Salzsäure an. Das ausgefallene Produkt wurde abgesaugt und getrocknet.

Ausbeute: 4,50 g (73 %). Schmelzpunkt: 104 °C.

Die in der folgenden Tabelle genannten Carbonsäuren wurden gemäß dem vorstehenden Beispiel erhalten:

| Beisp. | $R^1$ | $R^5$ | X | phys. Daten |
|---|---|---|---|---|
| 1.9(b) | Phenyl | $CH_3$ | S | Fp.: 127-137 |
| 1.4(a) | n-Butylthio | $C_2H_5$ | S | 0,95 (t;3H), 1,40 (t;3H), 1,50 (sext;2H), 1,80 (quint;2H), 3,40 (t;2H), 4,35 (q;2H) |
| 1.5(b) | n-Butylthio | $C_2H_5$ | S | 0,95 (t;3H), 1,35 (t;3H), 1,50 (sect;2H), 1,80 (quint;2H), 3,30 (t;2H), 4,45 (q;2H) |
| 2.6(b) | iso-Propylthio | $C_2H_5$ | S | 1,50 (d;6H), 1,45 (t;3H), 3,90 (hept;1H), 4,55 (q;2H), 12,50 (s;1H) |
| 1.7(a) | iso-Propylthio | $C_2H_5$ | S | 1,45 (t;3H), 1,50 (d;6H), 4,05 (hept;1H), 4,50 (q;2H), 12,50 (s;1H) |
| 1.8(a) | Methylthio | $CH_3$ | S | 2,80 (s;3H), 4,05 (s;3H) |

Beispiel 1.9

2-Methylthiothiazol-4,5-dicarbonsäure-diethylester

Eine Lösung von 9,2 g (0,03 mol) 2-Chlor-thiazol-4,5-dicarbonsäure-diethylester in 30 ml Ethanol wurde bei 0 °C tropfenweise mit einer Lösung von 2,1 g (0,03 mol) Natrium-methylthiolat in 10 ml Ethanol versetzt. Man ließ das Gemisch auf 25 °C erwärmen und rührte zwei Stunden nach. Danach entfernte man das Lösungsmittel bei vermindertem Druck, nahm den Rückstand in 100 ml Diethylether auf und wusch nacheinander mit je 50 ml 5 %iger Natronlauge und Wasser. Man trocknete über Natriumsulfat, engte ein und behielt 7,2 g (87 %) Produkt als farbloses Öl zurück.

$^1$H-NMR ($CDCl_3$, 250 MHz, TMS als interner Standard): 1,35 (t, J = 7,0 Hz, 3H), 1,45 (t, J = 7,0 Hz; 3H), 2,75 (s, 3H), 4,30 (q, J = 7,0 Hz; 2H), 4,50 (q, J = 7,0 Hz; 2H).

2. Verfahren zur Herstellung der Verbindungen VIa und VIb

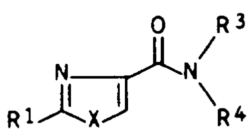

VIa

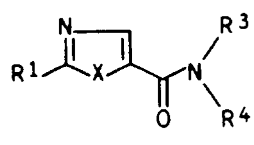

VIb

Beispiel 2.1

2-Methoxythiazol-4-carbonsäure-tert.-butylamid

Eine Lösung von 12,00 g (46 mmol) 2-Brom-thiazol-4-carbonsäure-tert.-butylamid in 150 ml Methanol wurde bei 25 °C mit 8,90 g einer 30 %igen Lösung (49 mmol) von Natriummethanolat in Methanol versetzt.

Man hielt das Gemisch vier Stunden unter Rückfluß auf Siedetemperatur, engte dann die klare Lösung ein, nahm den Rückstand in 300 ml Diethylether auf, filtrierte und entfernte das Lösungsmittel bei vermindertem Druck. Man erhielt 9,60 g (98 %) Produkt als gelbes Öl.

$^1$H-NMR ($CDCl_3$, 250 MHz, TMS als interner Standard): 1,45 (s; 9H), 4,10 (s; 3H), 7,00 (s, breit, 1H), 7,48 (s; 1H).

Beispiel 2.2

2-Isopropyl-oxazol-4-carbonsäure-cyclopropylamid

Zu einer Lösung von 31,0 g (0,20 mol) 2-Isopropyl-oxazol-4-carbonsäure in 200 ml Toluol und 2 ml Dimethylformamid tropfte man bei Raumtemperatur 47,6 g (0,40 mol) Thionylchlorid und rührte 1 h bei 80 °C. Man zog die Solventien im Vakuum ab, löste den Rückstand in 300 ml Dichlormethan und tropfte bei 0 bis 10 °C 24,0 g (0,42 mol) Cyclopropylamin in 20 ml Dichlormethan zu. Man rührte 12 h bei Raumtemperatur, gab 150 ml Wasser zu, trennte die Phasen, wusch die organische Phase einmal mit gesättigter Natriumhydrogencarbonatlösung, trocknete über Magnesiumsulfat und zog das Solvens im Vakuum ab. Man erhielt 37,2 g (96 %) 2-Isopropyl-oxazol-4-carbonsäure-cyclopropylamid.

$^1$H-NMR ($CDCl_3$, 250 MHz): Δ = 0,62 (m; 2H), 0,88 (m; 2H), 1,34 (d; 6H), 2,86 (m; 1H), 3,09 (m; 1H), 6,93 (bs; 1H; NH), 8,09 (s; 1H).

Die in der folgenden Tabelle genannten Amide wurden gemäß den vorstehenden Beispielen oder analog der zitierten Literatur erhalten:

| Nr. | $R^1$ | $R^3$ | $R^4$ | X | phys. Daten [Fp. (°C); NMR (δ in ppm)] |
|---|---|---|---|---|---|
| 2.3(a) | Phenylthio | H | tert.-Butyl | S | 1,50 (s;9H), 7,00 (s;1H), 7,45 (m;3H), 7,65 (m;2H), 7,85 (s;1H) |
| 2.4(a) | Brom | H | tert.-Butyl | S | 64-67 |
| 2.6(a) | Brom | H | cyclo-Propyl | S | 83-88 |
| 2.7(a) | Methoxy | H | cyclo-Propyl | S | 62-65 |
| 2.8(a) | Methyl | H | tert.-Butyl | S | 1,48 (s;9H), 2,72 (s;3H), 7,25 (s;1H,NH), 7,91 (s;1H) |
| 2.10(b) | Methoxy | H | tert.-Butyl | S | 126-129 |
| 2.11(a) | Methyl | H | tert.-Butyl | O | 60- 63 |
| 2.12(a) | cyclo-Propyl | H | tert.-Butyl | O | 72- 74 |
| 2.13(a) | Ethyl | H | tert.-Butyl | O | 1,36 (t;3H), 1,48 (s;9H), 2,80 (q;2H), 6,78 (s;1H, NH), 8,01 (s;1H) |
| 2.14(a) | Ethyl | H | cyclo-Propyl | O | 50- 55 |
| 2.15(a) | Ethyl | H | 3-$CF_3$-Phenyl | O | 40- 43 |
| 2.16(a) | iso-Propyl | H | tert.-Butyl | O | 1,36 (d;6H), 1,48 (s;9H), 3,06 (m;1H), 6,78 (s;1H,NH), 8,00 (s;1H) |
| 2.17(a) | iso-Propyl | H | cyclo-Propyl | O | 0,58-0,96(m;4H), 1,34(d;6H), 2,86 (m; 1H), 3,08 (m;1H), (s;1H,NH), 8,09 (s;1H) |
| 2.18(a) | iso-Propyl | H | iso-Propyl | O | 38- 41 |
| 2.19(a) | cyclo-Propyl | H | iso-Propyl | O | 57- 60 |
| 2.20(a) | cyclo-Propyl | H | cyclo-Propyl | O | 80- 83 |
| 2.21(a) | cyclo-Propyl | H | 4-Cl-Phenyl | O | 147-150 |

| Nr. | $R^1$ | $R^3$ | $R^4$ | X | phys. Daten [Fp. (°C); NMR (δ in ppm)] |
|---|---|---|---|---|---|
| 2.26(a) | Methyl | H | cyclo-Propyl | S | 109-114 |
| 2.27(a) | p-F-Benzyl | H | tert.-Butyl | S | 63- 64 |
| 2.28(a) | 2,6-Cl,Cl-Benzyl | H | Cyanomethyl | S | 82- 85 |
| 2.29(a) | tert.-Butyl | H | tert.-Butyl | S | 85- 86 |
| 2.31(a) | 3-$CF_3$-Benzyl | H | tert.-Butyl | S | 97- 99 |
| 2.32(a) | 2-Phenyl-ethyl | H | Cyano-methyl | S | 100 |
| 2.33(a) | 4-Cl-Phenoxy-methyl | H | tert.-Butyl | S | 102 |
| 2.34(a) | 2,6-Cl,Cl-Benzyl | H | tert.-Butyl | S | 105-106 |
| 2.35(a) | Benzyl | H | tert.-Butyl | S | 1,48 (s;9H), 4,30 (s;3H), 7,15-7,42 (m;6H), 7,90 (s;1H) |
| 2.36(a) | 2-Methoxy-ethyl | H | tert.-Butyl | S | 1,48 (s;9H), 3,25 (t;2H), 3,4 (s;3H), 3,75 (t;2H), 7,25 (s;1H), 7,95 (s;1H) |
| 2.37(a) | 2,4-Cl,Cl-Benzyl | H | tert.-Butyl | S | 1,48 (s;9H), 4,40 (s;2H), 7,13-7,48 (m;4H), 7,92 (s;1H) |
| 2.38(a) | 2-Phenyl-ethyl | H | tert.-Butyl | S | 1,48 (s;9H), 3,10 (t;2H), 3,30 (t;2H), 7,10-7,50 (m;6H), 7,90 (s;1H) |
| 2.39(a) | Methyl-thiomethyl | H | tert.-Butyl | S | 1,48 (s;9H), 2,16 (s;3H), 3,95 (s;2H), 7,20 (s;1H), 8,00 (s;1H) |
| 2.40(a) | tert.-Butyl | H | tert.-Butyl | S | 1,50 (s;9H), 3,86 (s;9H), 7,20 (s;1H), 7,45 (s;1H) |

| Nr. | $R^1$ | $R^3$ | $R^4$ | X | phys. Daten [Fp. (°C); NMR (δ in ppm)] |
|---|---|---|---|---|---|
| 2.41(a) | Methoxymethyl | H | tert.-Butyl | S | 1,49 (s;9H), 3,52 (s;3H), 4,7 (s; 2H), 7,10 (s;1H), 8,04 (s;1H) |
| 2.42(a) | 1-Phenyl-ethyl | H | tert.-Butyl | S | 1,48 (s;9H), 1,76 (d;2H), 4,43 (q; 1H), 7,15-7,43 (m;6H), 7,93 (s;1H) |
| 2.45(a) | n-Propyl | H | tert.-Butyl | O | 1,00 (t;3H), 1,44 (s;9H), 1,81 (m; 2H), 2,74 (t;2H), 6,75 (bs;1H),NH), 8.05 (s;1H) |
| 2.46(a) | n-Propyl | H | cyclo-Propyl | O | 54- 57 |
| 2.47(a) | n-Propyl | H | 2,4-$(CH_3)_2$-Phenyl | O | 44- 47 |
| 2.52(a) | Methoxymethyl | H | tert.-Butyl | O | 1,46 (s;9H), 3,50 (s;3H), 4,50 (s;2H), 6,80 (bs;1H,NH), 8,12 (s;1H) |
| 2.53(a) | tert.-Butyl | H | tert.-Butyl | O | 83- 87 |
| 2.54(a) | tert.-Butyl | H | cyclo-Propyl | O | 78- 80 |
| 2.55(a) | tert.-Butyl | H | CH(cyclo-Propyl)$_2$$CH_3$ | O | 132-134 |
| 2.56(a) | Methoxymethyl | H | cyclo-Propyl | O | 0,60-0,90 (m;4H), 2,88 (m;1H), 3,44 (s;3H), 4,53 (s;2H), 6,94 (bs;1H,NH), 8,21 (s;1H) |

3. Verfahren zur Herstellung der Verbindungen Ia und Ib

Beispiel 1

4-Cyclopropylaminocarbonyl-2-isopropyl-oxazol-5-carbonsäure

Zu einer Lösung von 10,4 g (0,054 mol) 2-Isopropyl-oxazol-4-carbonsäure-cyclopropylamid in 250 ml Tetrahydrofuran tropfte man unter Stickstoffatmosphäre bei -70 °C 0,12 mol n-Butyllithium (80,0 ml einer 1,5 molaren Lösung in Hexan) und rührte 30 min bei dieser Temperatur. Anschließend goß man das Reaktionsgemisch auf 500 g festes $CO_2$ und ließ über Nacht stehen. Man engte ein, nahm den Rückstand in 200 ml Wasser und 30 ml 2H NaOH auf, extrahierte zweimal mit je 100 ml Diethylether, säuerte die wäßrige Phase mit konz. Salzsäure auf pH 2 an und extrahierte dreimal mit je 200 ml Ethylacetat. Man trocknete über Magnesiumsulfat und zog das Solvens im Vakuum ab.

Man erhielt 10,4 g (81 %) 4-Cyclopropylaminocarbonyl-2-isopropyl-oxazol-5-carbonsäure als weißes Pulver vom Smp. 109 bis 112 °C.

(Wirkstoffbeispiel 3.007).

Beispiel 2

4-tert.-Butylaminocarbonyl-2-methoxy-thiazol-5-carbonsäure

Zu einer Lösung von 8,00 g (37 mmol) 2-Methoxy-thiazol-4-carbonsäure-tert.-butylamid in 150 ml Tetrahydrofuran tropfte man bei -70 °C 65 ml einer 1,5 m Lösung (97 mmol) von n-Butyllithium in n-Hexan und rührte 30 Minuten bei dieser Temperatur. Anschließend goß man das Reaktionsgemisch auf 500 g festes Kohlendioxid und ließ innerhalb von 14 Stunden auf Raumtemperatur erwärmen. Man entfernte das Lösungsmittel im Vakuum, nahm den Rückstand in einer Mischung aus 150 ml Wasser und 16 ml 2 m Natronlauge auf, filtrierte, säuerte das Filtrat mit konzentrierter Salzsäure an und saugte die ausgefallene Carbonsäure ab.

Man erhielt 7,80 g (82 %) 4-tert.-Butylaminocarbonyl-2-methoxythiazol-5-carbonsäure als weißes Pulver vom Fp.: 120 bis 122 °C.

(Wirkstoffbeispiel 1.003).

Beispiel 3

5-tert.-Butylaminocarbonyl-2-methoxy-thiazol-4-carbonsäure

Zu einer Lösung von 5,4 g (25,2 mmol) 2-Methoxy-thiazol-4-carbonsäure-tert.-butylamid in 150 ml Tetrahydrofuran tropfte man unter Stickstoffatmosphäre bei -70 °C 56 mmol n-Butyllithium (37,3 ml einer 1,5 molaren Lösung in Hexan) und rührte 30 min bei dieser Temperatur. Anschließend goß man das Reaktionsgemisch auf 500 g festes $CO_2$ und ließ über Nacht stehen. Man engte ein, nahm den Rückstand in 150 ml Wasser und 10 ml 2N NaOH auf, extrahierte zweimal mit je 50 ml Diethylether, säuerte die wäßrige Phase mit konz. Salzsäure auf pH 2 an und extrahierte dreimal mit je 100 ml Ethylacetat. Man trocknete über Magnesiumsulfat und zog das Solvens im Vakuum ab.

Man erhielt 3,9 g (60 %) 5-tert.-Butylaminocarbonyl-2-methoxy-thiazol-4-carbonsäure als weißes Pulver vom Smp. 105 bis 110 °C.

(Wirkstoffbeispiel 2.001)

Beispiel 4

a) 4-Ethoxycarbonyl-2-methyl-oxazol-5-carbonsäurechlorid

Zu 12,2 g (61,3 mmol) 4-Ethoxycarbonyl-2-methyl-oxazol-5-carbonsäure tropfte man bei 0 °C 40 ml Thionylchlorid und 1 ml Dimethylformamid und erhitzte 1 h unter Rückfluß. Man zog das überschüssige Thionylchlorid im Vakuum ab und destillierte den Rückstand im Ölpumpenvakuum.

Man erhielt 10,9 g (82 %) 4-Ethoxycarbonyl-2-methyl-oxazol-5-carbonsäurechlorid als gelbes Öl vom Sdp. 103 bis 105 °C/0,1 Torr. $^1H$-NMR (250 MHz, $CDCl_3$): $\delta$ = 1,42 (t; 3H), 2,66 (s; 3H), 4,66 (q; 2H).

b) 4-Ethoxycarbonyl-2-methyl-oxazol-5-carbonsäure-tert.-butylamid

Zu 10,9 g (50,3 mmol) 4-Ethoxycarbonyl-2-methyl-oxazol-5-carbonsäurechlorid in 150 ml Dichlormethan tropfte man bei 0 °C eine Lösung von 11,0 g (150 mmol) tert.-Butylamin in 20 ml Dichlormethan

und rührte 12 h bei Raumtemperatur. Man nahm das Reaktionsgemisch in 200 ml Wasser auf, trennte die Phasen, wusch die organische Phase einmal mit gesättigter Natriumhydrogencarbonatlösung sowie gesättigter Natriumchloridlösung, trocknete über Magnesiumsulfat und zog das Solvens im Vakuum am Rotationsverdampfer ab.

Man erhielt 11,9 g (93 %) 4-Ethoxycarbonyl-2-methyl-oxazol-5-carbonsäure-tert.-butylamid als weißen Feststoff vom Smp. 152 bis 155 °C.

(Wirkstoffbeispiel 4.001).

Beispiel 5

5-tert.-Butylaminocarbonyl-2-methyl-oxazol-4-carbonsäure

Zu 7,4 g (29,1 mmol) 4-Ethoxycarbonyl-2-methyl-oxazol-5-carbonsäure-tert.-butylamid in 150 ml Ethanol und 50 ml THF tropfte man bei 0 °C unter $N_2$ eine Lösung von 1,2 g (30,0 mmol) Natriumhydroxid in 50 ml Wasser. Man rührte 2 h bei 20 °C, zog die Solventien am Rotationsverdampfer im Vakuum ab, nahm den Rückstand in 300 ml Wasser auf, stellte auf pH = 9 ein und extrahierte die wäßrige Phase dreimal mit je 100 ml Diethylether. Anschließend säuerte man mit 6N HCl auf pH = 2 an und extrahierte viermal mit je 150 ml Dichlormethan. Die organische Phase wurde über Natriumsulfat getrocknet und das Solvens im Vakuum abgezogen.

Man erhielt 6,1 g (93 %) 5-tert.-Butylaminocarbonyl-2-methyl-oxazol-4-carbonsäure als weißen Feststoff vom Smp. 186 bis 188 °C.

(Wirkstoffbeispiel 4.002).

Beispiel 6

a) 4-Ethoxycarbonyl-2-methylthio-thiazol-5-carbonsäurechlorid

3,40 g (13,7 mmol) 4-Ethoxycarbonyl-2-methylthio-thiazol-5-carbonsäure wurden in 50 ml Thionylchlorid gelöst und bis zur Beendigung der Gasentwicklung zum Rückfluß erhitzt. Man entfernte überschüssiges Thionylchlorid im Vakuum und behielt 3,55 g (98 %) Säurechlorid als farbloses Öl zurück.

$^1$H-NMR ($CDCl_3$, 250 MHz, TMS als interner Standard): 1,50 (t, J = 7,0 Hz; 3H), 2,75 (s; 3H), 4,60 (q, J = 7,0 Hz; 2H).

b) 4-Ethoxycarbonyl-2-methylthio-thiazol-5-carbonsäure-tert.-butylamid

3,50 g (13,2 mmol) 4-Ethoxycarbonyl-2-methylthio-thiazol-5-carbonsäurechlorid wurden in 20 ml Dichlormethan gelöst und bei 0 °C zu einer Lösung von 3,20 g (44 mmol) tert.-Butylamin in 50 ml Dichlormethan getropft. Man ließ das Gemisch auf Raumtemperatur erwärmen, rührte 14 Stunden nach und gab dann 100 ml 10 %ige Salzsäure zu. Die organische Phase wurde abgetrennt, mit 50 ml Wasser gewaschen und über Natriumsulfat getrocknet. Man entfernte das Lösungsmittel im Vakuum und behielt 4,00 g (100 %) Produkt als gelben Kristallbrei zurück.

$^1$H-NMR ($CDCl_3$, 250 MHz, TMS als interner Standard): 1,45 (t, J = 7,0 Hz; 3H), 1,45 (s; 9H), 2,75 (s; 3H), 4,50 (q, J = 7,0 Hz; 2H), 9,90 (s, breit; 1H).

(Wirkstoffbeispiel 2.007)

Beispiel 7

5-tert.-Butylaminocarbonyl-2-methylthio-thiazol-4-carbonsäure

4,00 g (13,2 mmol) 4-Ethoxycarbony1-2-methylthio-thiazol-5-carbonsäure-tert.-butylamid wurden in 50 ml Wasser/Ethanol (2:1) gelöst, mit 0,82 g (14,6 mmol) Kaliumhydroxid in 10 ml Wasser versetzt und zwei Stunden zum Rückfluß erhitzt. Anschließend entfernte man das Lösungsmittelgemisch im Vakuum, nahm den Rückstand mit 50 ml Wasser auf und säuerte mit konzentrierter Salzsäure an. Das ausgefallene Produkt wurde abgesaugt und getrocknet.

Ausbeute: 3,40 g (94 %); Schmelzpunkt: 100 °C. (Wirkstoffbeispiel 2.005).

Beispiel 8

4-tert.-Butylaminocarbonyl-2-methoxy-thiazol-5-carbonsäure-acetonoximester

Zu einer Lösung von 3,1 g (12,0 mmol) 4-tert.-Butylaminocarbonyl-2-methoxy-thiazol-5-carbonsäure und 1,2 g (16,4 mmol) Acetonoxim in 100 ml Dichlormethan tropfte man bei Raumtemperatur 4,4 g (43,6 mmol) 4-Methylmorpholin sowie 1,5 g (12,3 mmol) 4-Dimethylaminopyridin und rührte 5 min. Anschließend fügte man 10,1 g einer 50 %igen Lösung von Propanphosphonsäureanhydrid in Dichlormethan (= 15,9 mmol) zu und erhitzte 7 h unter Rückfluß. Man engte ein, nahm den Rückstand in 100 ml Ethylacetat auf, extrahierte zweimal mit gesättigter Nariumhydrogencarbonatlösung sowie je einmal mit 5 %iger Zitronensäurelösung, gesättigter Natriumcarbonatlösung und gesättigter Natriumchloridlösung. Die organische Phase wurde über Magnesiumsulfat getrocknet und das Solvens im Vakuum abgezogen.

Man erhielt 3,1 g (82 %) 4-tert.-Butylaminocarbonyl-2-methoxy-thiazol-5-carbonsäure-acetonoximester als weißes Pulver vom Smp. 128 bis 131 °C.
(Wirkstoffbeispiel 1.011).

Beispiel 9

5-tert.-Butylaminocarbonyl-2-methyl-oxazol-4-carbonsäure-acetonoximester

Zu einer Lösung von 3,80 g (16,8 mmol) 5-tert.-Butylamino-carbonyl-2-methyl-oxazol-4-carbonsäure und 1,23 g (16,8 mmol) Acetonoxim in 40 ml Tetrahydrofuran tropfte man bei Raumtemperatur 3,46 9 (16,8 mmol) Dicyclohexylcarbodiimid in 20 ml Tetrahydrofuran. Man rührte 14 h, saugte den ausgefallenen Niederschlag ab, zog das Solvens im Vakuum ab und chromatographierte den Rückstand an Kieselgel (Lösungsmittel: Cyclohexan:Ethylacetat (1:1)). Man erhielt 2,7 g (57 %) 5-tert.-Butylaminocarbonyl-2-methyl-oxazol-4-carbonsäure-acetonoximester als weißen Feststoff vom Smp. 107 bis 111 °C.

(Wirkstoffbeispiel 4.003).

Die in den folgenden Tabellen aufgeführten Wirkstoffe wurden analog zu den voranstehenden Verbindungen hergestellt.

Tabelle 1

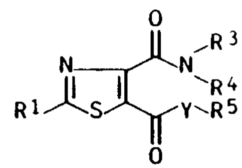

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 1.001 | Methyl | H | tert.-Butyl | H | O | 141-144 |
| 1.002 | Methyl | H | cyclo-Propyl | H | O | 138-142 |
| 1.003 | Methoxy | H | tert.-Butyl | H | O | 120-122 |
| 1.004 | Methoxy | H | cyclo-Propyl | H | O | 146-148 |
| 1.005* | Phenyl | H | tert.-Butyl | H | O | 194-195 |
| 1.006 | Phenylthio | H | tert.-Butyl | H | O | 1,50 (s; 9H), 7,55 (m; 3H), 7,75 (m, 2H), 7,90 (s; 1H), 16.60 (s; 1H) |
| 1.007 | 4-Cl-Phenylthio | H | tert.-Butyl | H | O | 1,50 (s; 9H), 7,50 (d; 2H), 7,65 (d, 2H), 8,85 (s; 1H), 16,50 (s; 1H) |
| 1.008 | Methylthio | H | tert.-Butyl | H | O | 137 |
| 1.009 | Methyl | H | tert.-Butyl | $-N=C(CH_3)_2$ | O | 101-107 |
| 1.010 | Methoxy | H | cyclo-Propyl | $-N=C(CH_3)_2$ | O | 128-131 |
| 1.011 | Methoxy | H | tert.-Butyl | $-N=C(CH_3)_2$ | O | 128-131 |

* nicht erfindungsgemäße Verbindung

Tabelle 1 (Fortsetzung)

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 1.012 | Methyl | H | cyclo-Propyl | $-N=C(CH_3)_2$ | O | 0,66 (m; 2H), 0,89 (m; 2H), 2,10 (s, 3H), 2,12 (s; 3H), 2,77 (s; 3H), 2,94 (m; 1H), 8,23 (s; 1H) |
| 1.013* | Phenyl | H | tert.-Butyl | $-N=C(CH_3)_2$ | O | 143-146 |
| 1.014 | n-Butyl-S- | H | tert.-Butyl | H | O | 71 |
| 1.015 | iso-Propyl-S- | H | tert.-Butyl | H | O | 97 |
| 1.016 | 4-F-Benzyl | H | tert.-Butyl | H | O | 93 |
| 1.017 | 2-Phenyl-ethyl | H | tert.-Butyl | H | O | 94 |
| 1.018 | Methoxy-methyl | H | tert.-Butyl | H | O | 100 |
| 1.019 | 2,4-Cl,Cl-Benzyl | H | tert.-Butyl | H | O | 100-102 |
| 1.020 | $3-CF_3$-Benzyl | H | tert.-Butyl | H | O | 109-110 |
| 1.021 | Benzyl | H | tert.-Butyl | H | O | 128 |
| 1.022 | tert.-Butyl | H | tert.-Butyl | H | O | 132 |
| 1.023 | cyclo-Propyl | H | tert.-Butyl | H | O | 142 |
| 1.024 | 4-Cl-Phenoxy-methyl | H | tert.-Butyl | H | O | 148-150 |
| 1.025 | iso-Propyl | H | tert.-Butyl | H | O | 150-153 |
| 1.026* | 4-Phenoxy-phenyl | H | tert.-Butyl | H | O | 158-161 |
| 1.027 | 3,4,5-Trimethoxy-benzyl | H | tert.-Butyl | H | O | 162-164 |

* nicht erfindungsgemäße Verbindung

Tabelle 1 (Fortsetzung)

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 1.028* | 2-Pyridyl | H | tert.-Butyl | H | O | 201 |
| 1.029 | 4-F-Benzyl | H | tert.-Butyl | Na | O | 199 |
| 1.030 | H | H | tert.-Butyl | H | O | 147 |
| 1.031 | 1-Phenyl-ethyl | H | tert.-Butyl | H | O | 1,51 (s;9H), 1,78 (d;2H), 4,40 (q;1H), 7,16-7,45 (m;5H), 8,00 (s;1H), 16-80 (s;1H) |
| 1.032 | 2,6-Cl,Cl-Benzyl | H | tert.-Butyl | H | O | 1,50 (s;9H), 4,61 (s;2H), 7,25-7,45 (m;3H), 7,95 (s;1H), 16-70 (s;1H) |

* nicht erfindungsgemäße Verbindung

Tabelle 2

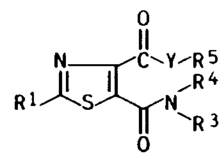

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 2.001 | Methoxy | H | tert.-Butyl | H | O | 105-110 |
| 2.002* | Phenyl | H | tert.-Butyl | H | O | 120 |
| 2.003* | Phenyl | H | 4-Cl-Phenyl | Methyl | O | 136 |
| 2.004* | Phenyl | H | 4-Cl-Phenyl | H | O | 167 |
| 2.005 | Methylthio | H | tert.-Butyl | H | O | 100 |
| 2.006 | 4-Cl-Phenylthio | H | tert.-Butyl | H | O | 75-77 |
| 2.007 | $SCH_3$ | H | tert.-Butyl | Ethyl | O | 1,45 (t,3H); 2,75 (s,3H); 4,5 (q,2H), 9,9 (s,1H) |
| 2.008 | n-Butyl-S- | H | tert.-Butyl | H | O | 81 |
| 2.009 | iso-Propyl-S- | H | tert.-Butyl | H | O | 1,45 (s,9H); 1,50 (d,6H); 3,80 ("sept.",1H); 10,0 (s,1H) |
| 2.010 | cyclo-Propyl | H | tert.-Butyl | H | O | 84 |
| 2.011 | iso-Propyl | H | tert.-Butyl | H | O | 105-106 |
| 2.012 | 3,4,5-Trimeth-oxy-benzyl | H | tert.-Butyl | H | O | 115 |
| 2.013 | Methoxy-methyl | H | tert.-Butyl | H | O | 120-121 |
| 2.014 | tert.-Butyl | H | tert.-Butyl | H | O | 143 |

* nicht erfindungsgemäße Verbindung

Tabelle 2 (Fortsetzung)

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 2.015 | Benzyl | H | tert.-Butyl | Ethyl | O | 152-156 |
| 2.016 | cyclo-Propyl | H | tert.-Butyl | Ethyl | O | 0,95-1,08 (m, ;2H), 1,15-1,28 (m;2H), 1,45 (t;3H), 1,45 (s;9H), 2,28-2,42 (m;1H), 4,45 (q;2H), 9,92 (s;1H) |
| 2.017 | Methoxy-methyl | H | tert.-Butyl | H | O | 1,45 (s;9H), 3,55 (s;3H), 4,75 (s;2H), 9,18-9,65 (s;1H), 9,98 (s;1H), |
| 2.018 | Methoxy-methyl | H | tert.-Butyl | Ethyl | O | 1,42 (t;3H), 1,45 (s;9H), 3,50 (s;3H), 4,50 (q;2H), 4,75 (s;2H), 9,95 (s;1H) |
| 2.019 | 4-F-Benzyl | H | tert.-Butyl | H | O | 1,45 (s;9H), 4,30 (s;2H), 6,95-7,15 (m;2H), 7,20-7,35 (m;2H), 9,95 (s;1H) |

Tabelle 3

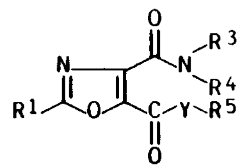

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 3.001 | Methyl | H | tert.-Butyl | H | O | 152-157 |
| 3.002 | Ethyl | H | tert.-Butyl | H | O | 130-131 |
| 3.003 | Ethyl | H | cyclo-Propyl | H | O | 135-138 |
| 3.004 | Ethyl | H | 3-$CF_3$-Phenyl | H | O | 169-172 |
| 3.005 | cyclo-Propyl | H | tert.-Butyl | H | O | 117 |
| 3.006 | iso-Propyl | H | tert.-Butyl | H | O | 151-153 |
| 3.007 | iso-Propyl | H | cyclo-Propyl | H | O | 109-112 |
| 3.008 | iso-Propyl | H | iso-Propyl | H | O | 64-70 |
| 3.009 | Ethyl | H | tert.-Butyl | $-N=C(CH_3)_2$ | O | 107-109 |
| 3.010 | Ethyl | H | cyclo-Propyl | $-N=C(CH_3)_2$ | O | 87-90 |
| 3.011 | Ethyl | H | 3-$CF_3$-Phenyl | $-N=C(CH_3)_2$ | O | 118-120 |
| 3.012 | iso-Propyl | H | tert.-Butyl | $-N=C(CH_3)_2$ | O | 121-125 |
| 3.013 | iso-Propyl | H | cyclo-Propyl | $-N=C(CH_3)_2$ | O | 62-65 |
| 3.014 | iso-Propyl | H | iso-propyl | $-N=C(CH_3)_2$ | O | 76-79 |
| 3.015 | cyclo-Propyl | H | tert.-Butyl | $-N=C(CH_3)_2$ | O | 101-104 |
| 3.016 | cyclo-Propyl | H | iso-Propyl | H | O | 148-151 |
| 3.017 | cyclo-Propyl | H | iso-Propyl | $-N=C(CH_3)_2$ | O | 103-106 |
| 3.018 | cyclo-Propyl | H | cyclo-Propyl | H | O | 126-129 |

Tabelle 3 (Fortsetzung)

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 3.019 | cyclo-Propyl | H | cyclo-Propyl | $-N=C(CH_3)_2$ | O | 108-110 |
| 3.020 | cyclo-Propyl | H | 4-Cl-Phenyl | H | O | 213-215 |
| 3.021 | Ethyl | H | iso-Propyl | H | O | 103-106 |
| 3.022 | Ethyl | H | iso-Propyl | $-N=C(CH_3)_2$ | O | 93-95 |
| 3.023 | Ethyl | H | tert.-Butyl | -N=C(cyclo-Propyl)$_2$ | O | 107-110 |
| 3.024 | iso-Propyl | H | tert.-Butyl | $CH_2-C\equiv CH$ | O | 1,42 (d;6H), 1,44 (s;9H), 2,61 (t;1H), 3,18 (m;1H), 5,00 (d;2H), 8,46 (bs;1H,NH) |
| 3.025* | Phenyl | H | tert.-Butyl | H | O | 203-206 |
| 3.026* | Phenyl | H | iso-Propyl | H | O | 144-146 |
| 3.027* | Phenyl | H | cyclo-Propyl | H | O | 217-218 |
| 3.028 | iso-Propyl | H | tert.-Butyl | 4-$CH_3O$-Phenyl | O | 137-139 |
| 3.029 | cyclo-Propyl | H | 4-Cl-Phenyl | $-N=C(CH_3)_2$ | O | 126-128 |
| 3.030* | Phenyl | H | tert.-Butyl | $-N=C(CH_3)_2$ | O | 149-154 |
| 3.031* | Phenyl | H | cyclo-Propyl | $-N=C(CH_3)_2$ | O | 160-164 |
| 3.032* | cyclo-Hexyl | H | tert.-Butyl | $-N=C(CH_3)_2$ | O | 121-125 |
| 3.033* | cyclo-Hexyl | H | cyclo-Propyl | H | O | 117-119 |
| 3.034* | cyclo-Hexyl | H | cyclo-Propyl | $-N=C(CH_3)_2$ | O | 119-122 |
| 3.035* | Phenyl | H | iso-Propyl | $-N=C(CH_3)_2$ | O | 137-139 |
| 3.036 | n-Propyl | H | tert.-Butyl | H | O | 108-110 |
| 3.037 | n-Propyl | H | tert.-Butyl | $-N=C(CH_3)_2$ | O | 69-72 |
| 3.038 | n-Propyl | H | cyclo-Propyl | H | O | 120-123 |
| 3.039 | n-Propyl | H | 2,4-$(CH_3)_2$-Phenyl | H | O | 142-145 |

* nicht erfindungsgemäße Verbindung

Tabelle 3 (Fortsetzung)

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 3.040 | Methoxymethyl | H | tert.-Butyl | H | O | 104-109 |
| 3.041 | tert.-Butyl | H | tert.-Butyl | H | O | 176-178 |
| 3.042 | tert.-Butyl | H | cyclo-Propyl | H | O | 132-134 |
| 3.043 | tert.-Butyl | H | $-CH(CH_3)-$cyclo-Propyl | H | O | 114-118 |
| 3.044 | tert.-Butyl | H | tert.-Butyl | $-N=C(CH_3)_2$ | O | 125-128 |
| 3.045 | tert.-Butyl | H | cyclo-Propyl | $-N=C(CH_3)_2$ | O | 123-126 |
| 3.046 | tert.-Butyl | H | $-CH(CH_3)-$cyclo-Propyl | $-N=C(CH_3)_2$ | O | 124-127 |

Tabelle 4

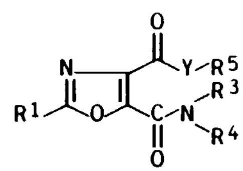

| Beispiel Nr. | $R^1$ | $R^3$ | $R^4$ | $R^5$ | Y | phys. Daten [Fp. (°C); NMR (δ in ppm)] |
|---|---|---|---|---|---|---|
| 4.001 | Methyl | H | tert.-Butyl | Ethyl | O | 152-155 |
| 4.002 | Methyl | H | tert.-Butyl | H | O | 186-188 |
| 4.003 | Methyl | H | tert.-Butyl | $-N=C(CH_3)_2$ | O | 107-111 |
| 4.004* | Phenyl | H | tert.-Butyl | Ethyl | O | 155-166 |
| 4.005* | Phenyl | H | tert.-Butyl | H | O | 230-232 |

* **nicht erfindungsgemäße Verbindung**

Anwendungsbeispiele

Die herbizide Wirkung der Oxazol- bzw. Thiazolcarbonsäureamide der Formeln Ia' und Ib' ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 1,0 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Cassia tora | Gemüse-Kassie |
| Chenopodium album | Weißer Gänsefuß |
| Chrysanthemum coronarium | Kronenwucherblume |
| Ipomoea spp. | Prunkwindearten |
| Triticum aestivum | Sommerweizen |
| Veronica spp. | Ehrenpreisarten |

Mit 1,0 kg/ha a.S. im Nachauflaufverfahren eingesetzt, lassen sich mit den Beispielen 1.001, 1.003, 1.004, 1.009, 1.010, 1.011, 3,002, 3,005 und 3,024 breitblättrige unerwünschte Pflanzen sehr gut bekämpfen. Verbindungen 1.001, 1.003 und 1.009 zeigen gleichzeitig Kulturpflanzenverträglichkeit an Weizen. Verbindung 3.005 wird sehr gut von der Kulturpflanze Mais toleriert.

**Patentansprüche**

**1.** Oxazol- bzw. Thiazolcarbonsäureamide der Formeln Ia und Ib

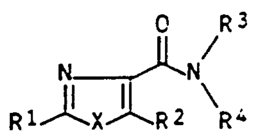

Ia

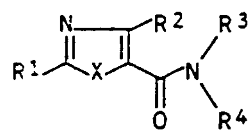

Ib

in denen die Substituenten folgende Bedeutung haben:

X Sauerstoff oder Schwefel;

$R^1$ Wasserstoff; Halogen; $C_1$-$C_6$-Alkyl, welches ein bis fünf Halogenatome und/oder einen oder zwei dar folgenden Reste tragen kann: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio oder Cyano;

Benzyl, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

Cyclopropyl oder Cyclopentyl, welche ein bis drei der folgenden Reste tragen können: $C_1$-$C_4$-Alkyl oder Halogen;

$C_2$-$C_6$-Alkenyl, welches ein bis drei der folgenden Reste tragen kann: Halogen: $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_2$-$C_6$-Alkinyl, welches ein bis drei der folgenden Reste tragen kann: Halogen oder $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
$C_1$-$C_4$-Alkoxy; $C_1$-$C_4$-Alkylthio; $C_1$-$C_4$-Halogenalkoxy; $C_1$-$C_4$-Halogenalkylthio;
Phenoxy oder Phenylthio, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$R^2$ Formyl, 4,5-Dihydrooxazol-2-yl oder den Rest -COY$R^5$;

Y Sauerstoff oder Schwefel;

$R^5$ Wasserstoff;
$C_1$-$C_6$-Alkyl, welches ein bis fünf Halogenatome oder Hydroxygruppen und/oder einen der folgenden Reste tragen kann: $C_1$-$C_4$-Alkoxy, $C_2$-$C_4$-Alkoxy-$C_1$-$C_4$-alkoxy, Cyano, Trimethylsilyl, $C_1$-$C_3$-Alkylthio, $C_1$-$C_3$-Alkylamino, $C_1$-$C_3$-Dialkylamino, $C_3$-$C_7$-Cycloalkylamino, $C_1$-$C_3$-Alkylsulfinyl, $C_1$-$C_3$-Alkylsulfonyl, Carboxyl, $C_1$-$C_3$-Alkoxycarbonyl, $C_1$-$C_3$-Dialkylaminocarbonyl, $C_1$-$C_3$-Dialkoxyphosphonyl, Alkaniminoxy, Thienyl, Furyl, Tetrahydrofuryl, Phthalimido, Pyridyl, Benzyloxy, Benzoyl, wobei die cyclischen Reste ihrerseits eine bis drei der folgenden Gruppen tragen können: $C_1$-$C_3$-Alkyl, $C_1$-$C_3$-Alkoxy oder Halogen;
Benzyl, das eine bis drei der folgenden Gruppen tragen kann: $C_1$-$C_3$-Alkyl, $C_1$-$C_3$-Alkoxy, $C_1$-$C_3$-Halogenalkyl, Halogen, Nitro und Cyano;
$C_3$-$C_8$-Cycloalkyl;
Phenyl, des eine bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoxycarbonyl, Halogen, Nitro und Cyano;
$C_3$-$C_8$-Alkenyl, $C_5$-$C_6$-Cycloalkenyl oder $C_3$-$C_8$-Alkinyl, wobei diese Reste eine der folgenden Gruppen tragen können: Hydroxy, $C_1$-$C_4$-Alkoxy, Halogen oder einen Phenylring, welcher seinerseits eine bis drei der folgenden Gruppen tragen kann: $C_1$-$C_4$-Alkyl, $C_1$-$C_4$-Alkoxy, $C_1$-$C_4$-Halogenalkyl, Halogen, Nitro und Cyano;
einen fünf- bis sechsgliedrigen heterocyclischen Rest enthaltend ein oder zwei Heteroatome, ausgewählt aus der Gruppe Sauerstoff, Schwefel und Stickstoff oder einen Benzotriazolrest;
Phthalimido; Tetrahydrophthalimido; Succinimido; Maleinimido;
ein Äquivalent eines Kations aus der Gruppe der Alkali- oder Erdalkalimetalle, Mangan, Kupfer, Eisen, Ammonium und substituiertes Ammonium;
einen Rest $-N=CR^6R^7$;
$R^6$, $R^7$ Wasserstoff; $C_1$-$C_4$-Alkyl; $C_3$-$C_6$-Cycloalkyl; Phenyl oder Furyl oder zusammen eine Methylenkette der Formel $-(CH_2)_m-$ mit m = 4 bis 7 Kettengliedern;

$R^3$ Wasserstoff; $C_1$-$C_6$-Alkyl, das einen bis drei der folgenden Substituenten tragen kann: Hydroxy, Halogen, $C_1$-$C_4$-Alkoxy, $C_1$-$C_4$-Alkylthio oder Di-$C_1$-$C_3$-Alkylamino;
$C_3$-$C_8$-Cycloalkyl, welches ein bis drei der folgenden Reste tragen kann: $C_1$-$C_4$-Alkyl, Halogen und $C_1$-$C_4$-Halogenalkyl;

$R^4$ Hydroxy; $C_1$-$C_4$-Alkoxy;
$C_1$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, Halogen, $C_3$-$C_8$-Cycloalkyl oder Phenyl, welches seinerseits ein bis drei der folgenden Reste tragen kann: Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;
$C_3$-$C_8$-Cycloalkyl, das eine bis drei der folgenden Grupen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano;
$C_3$-$C_6$-Alkenyl oder $C_3$-$C_6$-Alkinyl, das ein- bis dreimal durch Halogen und/oder einmal durch Phenyl substituiert sein kann, wobei der Phenylring seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
ein 5- bis 6-gliedriger heterocyclischer Rest enthaltend ein oder zwei Heteroatome, ausgewählt aus der Gruppe Sauerstoff, Schwefel oder Stickstoff, welcher ein bis drei der folgenden Reste tragen kann: $C_1$-$C_4$-Alkyl oder Halogen;
Phenyl, das eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, $C_1$-$C_4$-Alkanoyl, $C_1$-$C_4$-Halogenalkanoyl oder $C_1$-$C_4$-Alkoxycarbonyl;
Naphthyl, das ein- bis dreimal durch $C_1$-$C_4$-Alkyl oder Halogen substituiert sein kann,

oder
$R^3$ und $R^4$ gemeinsam einen Rest der Struktur $-(CH_2)_n-Y_p-(CH_2)_q-$, wobei n und q 1, 2 oder 3, p O oder 1 und Y Sauerstoff, Schwefel oder N-Methyl bedeuten oder den Rest der Formel $(CH_2)_3$-CO- bilden, sowie deren umweltverträgliche Salze, ausgenommen Verbindungen der Formel Ia, in der $R^1$ für unsubstituiertes Ethyl steht, wenn gleichzeitig X = Schwefel, $R^2$ = COOH, $R^3$ Wasserstoff und $R^4$ eine $C_1$-$C_4$-Dialkylamino-$C_1$-$C_3$-alkylgruppe bedeuten.

**2.** Oxazol- oder Thiazolcarbonsäureamide der Formeln Ia und Ib nach Anspruch 1, in denen $R^3$ Wasserstoff bedeutet.

**3.** Oxazol- oder Thiazolcarbonsäureamide der Formeln Ia und Ib nach Anspruch 1, in denen die Substituenten folgende Bedeutung haben:

$R^1$ Wasserstoff; $C_1$-$C_4$-Alkyl; $C_1$-$C_4$-Alkoxy; $C_1$-$C_4$-Halogenalkoxy; $C_1$-$C_4$-Alkylthio oder $C_1$-$C_4$-Halogenalkylthio;

$R^2$ einen Rest $-COYR^5$;

$R^5$ Wasserstoff; Phthalimido; Succinimido; Maleinimido oder ein Rest $-N=R^6R^7$

$R^6$, $R^7$ Wasserstoff; $C_1$-$C_4$-Alkyl und $C_3$-$C_6$-Cycloalkyl oder zusammen eine Methylenkette der Formel $-(CH_2)_m-$ mit m = 4 bis 7 Kettengliedern,

$R^3$ Wasserstoff und

$R^4$ $C_1$-$C_4$-Alkyl oder $C_3$-$C_8$-Cycloalkyl.

**4.** Verfahren zur Herstellung der Verbindungen Ia und Ib gemäß Anspruch 1, in denen $R^2$ $CO_2R^5$ und $R^5$ $C_1$-$C_6$-Alkyl bedeutet, dadurch gekennzeichnet, daß man einen Diester der Formel II

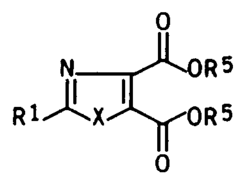

II

in an sich bekannter Weise mit einem Äquivalent einer wäßrigen Base zu einem Gemisch der Monoester IIIa und IIIb

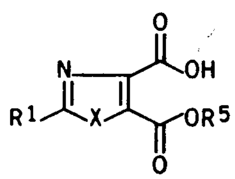

IIIa

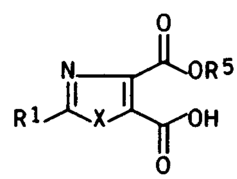

IIIb

hydrolysiert und IIIa und IIIb danach getrennt oder im Gemisch zunächst in das Halogenid oder eine andere aktivierte Form der Carbonsäure überführt und diese Derivate anschließend mit einem Amin der Formel IV

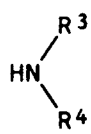

IV

amidiert.

**5.** Verfahren zur Herstellung der Verbindungen Ia und Ib gemäß Anspruch 1, in denen X Schwefel und $R^2$ $CO_2H$ bedeutet, dadurch gekennzeichnet, daß man ein Dicarbonsäureanhydrid der Formel V

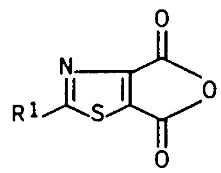

V

in an sich bekannter Weise mit einem Amin der Formel IV gemäß Anspruch 4 zu den Isomeren Ia und Ib umsetzt und anschließend das Gemisch in die Isomeren auftrennt.

**6.** Verfahren zur Herstellung der Verbindungen Ia und Ib gemäß Anspruch 1, in denen $R^1$ nicht Halogen und $R^2$ Carboxyl oder Formyl bedeutet, dadurch gekennzeichnet, daß man eine Carbonsäure der Formel IIIc bzw. IIId

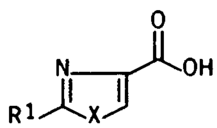

IIIc

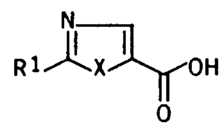

IIId

in an sich bekannter Weise zunächst gemäß Anspruch 4 aktiviert und amidiert und das so erhaltene Amid VIa bzw. VIb

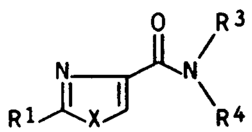

VIa

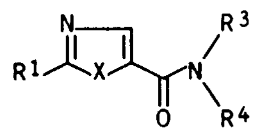

VIb

anschließend in Gegenwart einer Base mit einem Carboxylierungs- oder einem Formylierungsreagens umsetzt.

**7.** Verfahren zur Herstellung der Verbindungen Ia und Ib gemäß Anspruch 1, in denen $R^2$ $CO_2H$ bedeutet, dadurch gekennzeichnet, daß man ein Carbonsäureamid Ia bzw. Ib in dem $R^2$ $CO_2R^5$ und $R^5$ $C_1$-$C_6$-Alkyl bedeutet in an sich bekannter Weise mit einem Äquivalent einer wäßrigen Base hydrolysiert.

**8.** Verfahren zur Herstellung der Verbindungen Ia und Ib, in denen $R^2$ $COYR^5$ bedeutet, dadurch gekennzeichnet, daß man eine entsprechende Carbonsäure Ia bzw. Ib ($R^2$ = $CO_2H$) gemäß Anspruch 4 aktiviert und anschließend in an sich bekannter Weise mit einer Verbindung VII

$HYR^5$ VII

umsetzt.

**9.** Verfahren zur Herstellung der Verbindungen Ia und Ib, in denen $R^2$ 4,5-Dihydrooxazol-2-yl bedeutet, dadurch gekennzeichnet, daß man eine entsprechende Carbonsäure Ia bzw. Ib ($R^2$ = $CO_2H$, $CO_2R'$; R' = $C_1$-$C_4$-Alkyl) in an sich bekannter Weise mit 2-Aminoethanol VIII

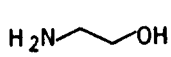

VIII

umsetzt.

**10.** Herbizides Mittel, enthaltend neben inerten Zusatzstoffen mindestens ein Oxazol- bzw. Thiazolcarbonsäureamid der Formeln Ia bzw. Ib gemäß Anspruch 1.

**11.** Verfahren zur Bekämpfung unerwünschten Pflanzenwuchses, dadurch gekennzeichnet, daß man die unerwünschten Pflanzen und/oder die von unerwünschtem Pflanzenwuchs freizuhaltende Fläche mit einer herbizid wirksamen Menge eines Oxazol- bzw. Thiazolcarbonsäureamids der Formeln Ia' bzw. Ib'

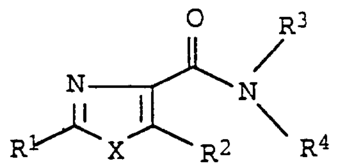

Ia'.

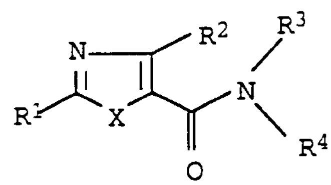

Ib'

in denen die Substituenten folgende Bedeutung haben:

X Sauerstoff oder Schwefel;

$R^1$ Wasserstoff; Halogen; $C_1$-$C_6$-Alkyl, welches ein bis fünf Halogenatome und/oder einen oder zwei der folgenden Reste tragen kann: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio oder Cyano;
Benzyl, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
$C_3$-$C_8$-Cycloalkyl, welches ein bis drei der folgenden Reste tragen kann: $C_1$-$C_4$-Alkyl oder Halogen;
$C_2$-$C_6$-Alkenyl, welches ein bis drei der folgenden Reste tragen kann: Halogen, $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
$C_2$-$C_6$-Alkinyl, welches ein bis drei der folgenden Reste tragen kann: Halogen oder $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
$C_1$-$C_4$-Alkoxy; $C_1$-$C_4$-Alkylthio; $C_1$-$C_4$-Halogenalkoxy; $C_1$-$C_4$-Halogenalkylthio;
Phenoxy oder Phenylthio, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
ein 5- bis 6-gliedriger heterocyclischer Rest, enthaltend ein oder zwei Heteroatome, ausgewählt aus der Gruppe Sauerstoff, Schwefel und Stickstoff, wobei der Ring ein oder zwei der folgenden Reste tragen kann: $C_1$-$C_3$-Alkyl, Halogen, $C_1$-$C_3$-Alkoxy oder $C_1$-$C_3$-Alkoxycarbonyl;
Phenyl, welches eine bis drei der folgenden Gruppen tragen kann: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro und Cyano,

$R^2$ Formyl, 4,5-Dihydrooxazol-2-yl oder den Rest -$COYR^5$;

Y Sauerstoff oder Schwefel;

$R^5$ Wasserstoff;
$C_1$-$C_6$-Alkyl, welches ein bis fünf Halogenatome oder Hydroxygruppen und/oder einen der folgenden Reste tragen kann: $C_1$-$C_4$-Alkoxy, $C_2$-$C_4$-Alkoxy-$C_1$-$C_4$-alkoxy, Cyano, Trimethylsilyl, $C_1$-$C_3$-Alkylthio, $C_1$-$C_3$-Alkylamino, $C_1$-$C_3$-Dialkylamino, $C_3$-$C_7$-Cycloalkylamino, $C_1$-$C_3$-Alkylsulfinyl, $C_1$-$C_3$-Alkylsulfonyl, Carboxyl, $C_1$-$C_3$-Alkoxycarbonyl, $C_1$-$C_3$-Dialkylaminocarbonyl, $C_1$-$C_3$-Dialkoxyphosphonyl, Alkaniminoxy, Thienyl, Furyl, Tetrahydrofuryl, Phthalimido, Pyridyl, Benzyloxy, Benzoyl, wobei die cyclischen Reste ihrerseits eine bis drei der folgenden Gruppen tragen können: $C_1$-$C_3$-Alkyl, $C_1$-$C_3$-Alkoxy oder Halogen;
Benzyl, das eine bis drei der folgenden Gruppen tragen kann: $C_1$-$C_3$-Alkyl, $C_1$-$C_3$-Alkoxy, $C_1$-

$C_3$-Halogenalkyl, Halogen, Nitro und Cyano;
$C_3$-$C_8$-Cycloalkyl;
Phenyl, das eine bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoxycarbonyl, Halogen, Nitro und Cyano;
$C_3$-$C_8$-Alkenyl, $C_5$-$C_6$-Cycloalkenyl oder $C_3$-$C_8$-Alkinyl, wobei diese Reste eine der folgenden Gruppen tragen können: Hydroxy, $C_1$-$C_4$-Alkoxy, Halogen oder einen Phenylring, welcher seinerseits eine bis drei der folgenden Gruppen tragen kann: $C_1$-$C_4$-Alkyl, $C_1$-$C_4$-Alkoxy, $C_1$-$C_4$-Halogenalkyl, Halogen, Nitro und Cyano;
einen fünf- bis sechsgliedrigen heterocyclischen Rest enthaltend ein oder zwei Heteroatome, ausgewählt aus der Gruppe Sauerstoff, Schwefel und Stickstoff oder einen Benzotriazolrest;
Phthalimido; Tetrahydrophthalimido; Succinimido; Maleinimido;
ein Äquivalent eines Kations aus der Gruppe der Alkali- oder Erdalkalimetalle, Mangan, Kupfer, Eisen, Ammonium und substituiertes Ammonium;
einen Rest $-N=CR^6R^7$;
$R^6$, $R^7$ Wasserstoff; $C_1$-$C_4$-Alkyl; $C_3$-$C_6$-Cycloalkyl; Phenyl oder Furyl oder zusammen eine Methylenkette der Formel $-(CH_2)_m-$ mit m = 4 bis 7 Kettengliedern;

$R^3$ Wasserstoff; $C_1$-$C_6$-Alkyl, das einen bis drei der folgenden Substituenten tragen kann: Hydroxy, Halogen, $C_1$-$C_4$-Alkoxy, $C_1$-$C_4$-Alkylthio oder Di-$C_1$-$C_3$-Alkylamino;
$C_3$-$C_8$-Cycloalkyl, welches ein bis drei der folgenden Reste tragen kann: $C_1$-$C_4$-Alkyl, Halogen und $C_1$-$C_4$-Halogenalkyl;

$R^4$ Hydroxy; $C_1$-$C_4$-Alkoxy;
$C_1$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, Halogen, $C_3$-$C_8$-Cycloalkyl oder Phenyl, welches seinerseits ein bis drei der folgenden Reste tragen kann: Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;
$C_3$-$C_8$-Cycloalkyl, das eine bis drei der folgenden Grupen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano;
$C_3$-$C_6$-Alkenyl oder $C_3$-$C_6$-Alkinyl, das ein- bis dreimal durch Halogen und/oder einmal durch Phenyl substituiert sein kann, wobei der Phenylring seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
ein 5- bis 6-gliedriger heterocyclischer Rest enthaltend ein oder zwei Heteroatome, ausgewählt aus der Gruppe Sauerstoff, Schwefel oder Stickstoff, welcher ein bis drei der folgenden Reste tragen kann: $C_1$-$C_4$-Alkyl oder Halogen;
Phenyl, das eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, $C_1$-$C_4$-Alkanoyl, $C_1$-$C_4$-Halogenalkanoyl oder $C_1$-$C_4$-Alkoxycarbonyl;
Naphthyl, das ein- bis dreimal durch $C_1$-$C_4$-Alkyl oder Halogen substituiert sein kann,
oder
$R^3$ und $R^4$ gemeinsam einen Rest der Struktur $-(CH_2)_n-Y_p-(CH_2)_q-$, wobei n und q 1, 2 oder 3, p 0 oder 1 und Y Sauerstoff, Schwefel oder N-Methyl bedeuten oder den Rest der Formel $-(CH_2)_3-CO-$ bilden,

behandelt.

## Claims

1. Oxazole- and thiazolecarboxamides of the formulae Ia and Ib

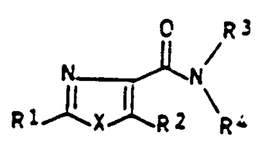 Ia 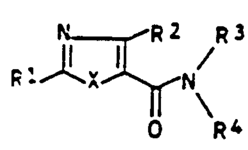 Ib

where

X is oxygen or sulfur;

$R^1$ is hydrogen; halogen; $C_1$-$C_6$-alkyl which can carry from one to five halogen atoms and/or one or two of the following: $C_3$-$C_6$-cycloalkyl, $C_1$-$C_4$-alkoxy, $C_1$-$C_4$-haloalkoxy, $C_1$-$C_4$-alkylthio, $C_1$-$C_4$-haloalkylthio or cyano;
benzyl which can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
cyclopropyl or cyclopentyl which can carry from one to three of the following: $C_1$-$C_4$-alkyl or halogen;
$C_2$-$C_6$-alkenyl which can carry from one to three of the following: halogen, $C_1$-$C_3$-alkoxy and/or one phenyl which in turn can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_2$-$C_6$-alkynyl which can carry from one to three of the following: halogen, $C_1$-$C_3$-alkoxy and/or one phenyl which in turn can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_1$-$C_4$-alkoxy; $C_1$-$C_4$-alkylthio; $C_1$-$C_4$-haloalkoxy; $C_1$-$C_4$-haloalkylthio;
phenoxy or phenylthio, which can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;

$R^2$ is formyl, 4,5-dihydro-2-oxazolyl or -$COYR^5$;

Y is oxygen or sulfur;

$R^5$ is hydrogen;
$C_1$-$C_6$-alkyl which can carry from one to five halogen atoms or hydroxyl groups and/or one of the following: $C_1$-$C_4$-alkoxy, $C_2$-$C_4$-alkoxy-$C_1$-$C_4$-alkoxy, cyano, trimethylsilyl, $C_1$-$C_3$-alkylthio, $C_1$-$C_3$-alkylamino, $C_1$-$C_3$-dialkylamino, $C_3$-$C_7$-cycloalkylamino, $C_1$-$C_3$-alkylsulfinyl, $C_1$-$C_3$-alkylsulfonyl, carboxyl, $C_1$-$C_3$-alkoxycarbonyl, $C_1$-$C_3$-dialkylaminocarbonyl, $C_1$-$C_3$-dialkoxyphosphoryl, alkaneiminoxy, thienyl, furyl, tetrahydrofuryl, phthalimido, pyridyl, benzyloxy or benzoyl, it being possible for the cyclic radicals in turn to carry from one to three of the following: $C_1$-$C_3$-alkyl, $C_1$-$C_3$-alkoxy or halogen;
benzyl which can carry from one to three of the following: $C_1$-$C_3$-alkyl, $C_1$-$C_3$-alkoxy, $C_1$-$C_3$-haloalkyl, halogen, nitro and cyano;
$C_3$-$C_8$-cycloalkyl;
phenyl which can carry from one to three of the following: $C_1$-$C_4$-alkyl, $C_1$-$C_4$-alkoxy, $C_1$-$C_4$-haloalkyl, $C_1$-$C_4$-haloalkoxy, $C_1$-$C_4$-alkoxycarbonyl, halogen, nitro and cyano;
$C_3$-$C_8$-alkenyl, $C_5$-$C_6$-cycloalkenyl or $C_3$-$C_8$-alkynyl, it being possible for these radicals to carry one of the following: hydroxyl, $C_1$-$C_4$-alkoxy, halogen or a phenyl ring which in turn can carry from one to three of the following: $C_1$-$C_4$-alkyl, $C_1$-$C_4$-alkoxy, $C_1$-$C_4$-haloalkyl, halogen, nitro and cyano;
a 5- to 6-membered heterocyclic radical containing one or two hetero atoms selected from the group comprising oxygen, sulfur and nitrogen or a benzotriazolyl radical;
phthalimido; tetrahydrophthalimido; succinimido; maleimido;
one equivalent of a cation from the group comprising the alkali metals or alkaline earth metals, manganese, copper, iron, ammonium and substituted ammonium;
$-N=CR^6R^7$;
$R^6$ and $R^7$ are hydrogen; $C_1$-$C_4$-alkyl; $C_3$-$C_6$-cycloalkyl; phenyl or furyl, or together form a methylene chain $-(CH_2)_m-$ with m = 4 to 7;

$R^3$ is hydrogen; $C_1$-$C_6$-alkyl which can carry from one to three of the following: hydroxyl, halogen, $C_1$-$C_4$-alkoxy, $C_1$-$C_4$-alkylthio or di-$C_1$-$C_3$-alkylamino;
$C_3$-$C_8$-cycloalkyl which can carry from one to three of the following: $C_1$-$C_4$-alkyl, halogen and $C_1$-$C_4$-haloalkyl;

$R^4$ is hydroxyl; $C_1$-$C_4$-alkoxy;
$C_1$-$C_6$-alkyl which can carry from one to three of the following: $C_1$-$C_4$-alkoxy, $C_1$-$C_4$-haloalkoxy, $C_1$-$C_4$-alkylthio, $C_1$-$C_4$-haloalkylthio, $C_1$-$C_4$-dialkylamino, halogen, $C_3$-$C_8$-cycloalkyl or phenyl which in turn can carry from one to three of the following: halogen, cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio;
$C_3$-$C_8$-cycloalkyl which can carry from one to three of the following: $C_1$-$C_6$-alkyl, $C_1$-$C_6$-haloalkyl, $C_1$-$C_4$-alkoxy, $C_1$-$C_4$-haloalkoxy, halogen, nitro or cyano;

$C_3$-$C_6$-alkenyl or $C_3$-$C_6$-alkynyl, which can be substituted from once to three times by halogen and/or once by phenyl which in turn can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;

a 5- to 6-membered heterocyclic radical which contains one or two hetero atoms selected from the group comprising oxygen, sulfur or nitrogen, and which can carry from one to three of the following: $C_1$-$C_4$-alkyl or halogen;

phenyl which can carry from one to four of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, nitro, cyano, formyl, $C_1$-$C_4$-alkanoyl, $C_1$-$C_4$-haloalkanoyl or $C_1$-$C_4$-alkoxycarbonyl; naphthyl which can be substituted from once to three times by $C_1$-$C_4$-alkyl or halogen,

or

$R^3$ and $R^4$ together form $-(CH_2)_n-Y_p-(CH_2)_q-$ where n and g are each 1, 2 or 3, p is 0 or 1 and Y is oxygen, sulfur or N-methyl, or $(CH_2)_3$-CO-, and the environmentally compatible salts thereof, with the exception of compounds of the formula Ia where R' is unsubstituted ethyl if at the same time X is sulfur, $R^2$ is COOH, $R^3$ is hydrogen and $R^4$ is $C_1$-$C_4$-dialkylamino-$C_1$-$C_3$-alkyl.

**2.** Oxazole- or thiazolecarboxamides of the formulae Ia and Ib as claimed in claim 1, where $R^3$ is hydrogen.

**3.** Oxazole- or thiazolecarboxamides of the formulae Ia and Ib as claimed in claim 1, where

$R^1$ is hydrogen; $C_1$-$C_4$-alkyl; $C_1$-$C_4$-alkoxy; $C_1$-$C_4$-haloalkoxy; $C_1$-$C_4$-alkylthio or $C_1$-$C_4$-haloalkylthio;

$R^2$ is $-COYR^5$;

$R^5$ is hydrogen; phthalimido; succininido; maleimido or $-N=R^6R^7$

$R^6$ and $R^7$ are hydrogen; $C_1$-$C_4$-alkyl and $C_3$-$C_6$-cycloalkyl, or together form a methylene chain $-(CH_2)_m$-with m = 4 to 7,

$R^3$ is hydrogen and

$R^4$ is $C_1$-$C_4$-alkyl or $C_3$-$C_8$-cycloalkyl.

**4.** A process for preparing compounds Ia and Ib as claimed in claim 1, where $R^2$ is $CO_2R^5$ and $R^5$ is $C_1$-$C_6$-alkyl, which comprises hydrolyzing a diester of the formula II

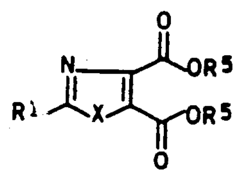

II

in a conventional manner with one equivalent of an aqueous base to give a mixture of the monoesters IIIa and IIIb

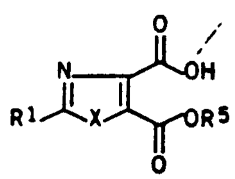

IIIa

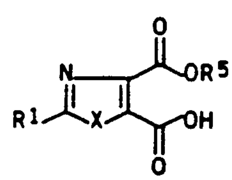

IIIb

and then separating IIIa and IIIb or initially converting the mixture to the halide or another active form of the carboxylic acid and subsequently amidating these derivatives with an amine of the formula IV

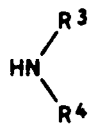

IV.

5. A process for preparing compounds Ia and Ib as claimed in claim 1, where X is sulfur and $R^2$ is $CO_2H$, which comprises reacting a dicarboxylic anhydride of the formula V

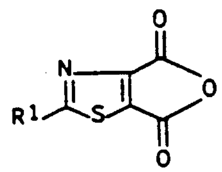

V

in a conventional manner with an amine of the formula IV as set forth in claim 4 to give the isomers Ia and Ib and subsequently fractionating the mixture to give the isomers.

6. A process for preparing compounds Ia and Ib as claimed in claim 1, where $R^1$ is not halogen and $R^2$ is carboxyl or formyl, which comprises initially activating and amidating a carboxylic acid of the formula IIIc or IIId

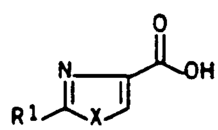

IIIc

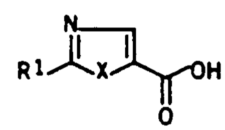

IIId

in a conventional manner as claimed in claim 4, and then reacting the resulting amide VIa and VIb

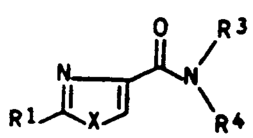

VIa

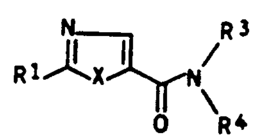

VIb

with a carboxylating or formylating reagent in the presence of a base.

7. A process for preparing compounds Ia and Ib as claimed in claim 1, where $R^2$ is $CO_2H$, which comprises hydrolyzing a carboxamide Ia or Ib where $R^2$ is $CO_2R^5$ and $R^5$ is $C_1$-$C_6$-alkyl in a conventional manner with one equivalent of an aqueous base.

8. A process for preparing compounds Ia and Ib where $R^2$ is $COYR^5$, which comprises activating a corresponding carboxylic acid Ia or Ib ($R^2$ = $CO_2H$) as claimed in claim 4 and subsequent reaction in a conventional manner with a compound VII

$HYR^5$ VII.

9. A process for preparing compounds Ia and Ib where $R^2$ is 4,5-dihydro-2-oxazolyl, which comprises reacting an appropriate carboxylic acid Ia or Ib ($R^2$ = $CO_2H$ or $CO_2R'$; R' = $C_1$-$C_4$-alkyl) in a conventional manner with 2-aminoethanol VIII

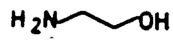

VIII.

**10.** A herbicide containing, in addition to inert additives, at least one oxazole- or thiazolecarboxamide of the formulae Ia and Ib as claimed in claim 1.

**11.** A method for controlling unwanted plant growth, which comprises treating the unwanted plants and/or the surface to be kept free of undesired plant growth with a herbicidally effective amount of an oxazole- or thiazole-carboxamide of the formulae Ia' and Ib'

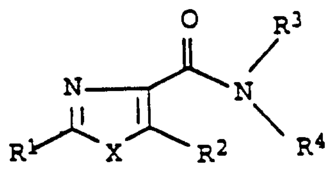

Ia'

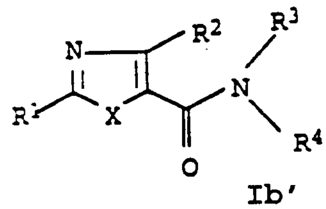

Ib'

where

X is oxygen or sulfur;

$R^1$ is hydrogen; halogen; $C_1$-$C_6$-alkyl which can carry from one to five halogen atoms and/or one or two of the following: $C_3$-$C_6$-cycloalkyl, $C_1$-$C_4$-alkoxy, $C_1$-$C_4$-haloalkoxy, $C_1$-$C_4$-alkylthio, $C_1$-$C_4$-haloalkylthio or cyano;
benzyl which can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_3$-$C_8$ cycloalkyl which can carry from one to three of the following: $C_1$-$C_4$-alkyl or halogen;
$C_2$-$C_6$-alkenyl which can carry from one to three of the following: halogen, $C_1$-$C_3$-alkoxy and/or one phenyl which in turn can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_2$-$C_6$-alkynyl which can carry from one to three of the following: halogen, $C_1$-$C_3$-alkoxy and/or one phenyl which in turn can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_1$-$C_4$-alkoxy; $C_1$-$C_4$-alkylthio; $C_1$-$C_4$-haloalkoxy; $C_1$-$C_4$-haloalkylthio;
phenoxy or phenylthio, which can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
a 5- to 6-membered heterocyclic radical containing one or two hetero atoms selected from the group comprising oxygen, sulfur and nitrogen, it being possible for the ring to carry one or two of the following: $C_1$-$C_3$-alkyl, halogen, $C_1$-$C_3$-alkoxy or $C_1$-$C_3$-alkoxycarbonyl;
phenyl which can carry from one to three of the following: $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro and cyano;

$R^2$ is formyl, 4,5-dihydro-2-oxazolyl or -COY$R^5$;

Y is oxygen or sulfur;

$R^5$ is hydrogen;
$C_1$-$C_6$-alkyl which can carry from one to five halogen atoms or hydroxyl groups and/or one of the following: $C_1$-$C_4$-alkoxy, $C_2$-$C_4$-alkoxy-$C_1$-$C_4$-alkoxy, cyano, trimethylsilyl, $C_1$-$C_3$-alkylthio, $C_1$-$C_3$-alkylamino, $C_1$-$C_3$-dialkylamino, $C_3$-$C_7$-cycloalkylamino, $C_1$-$C_3$-alkylsulfinyl, $C_1$-$C_3$-alkylsulfonyl, carboxyl, $C_1$-$C_3$-alkoxycarbonyl, $C_1$-$C_3$-dialkylaminocarbonyl, $C_1$-$C_3$-dialkoxyphosphoryl, alkaneiminoxy, thienyl, furyl, tetrahydrofuryl, phthalimido, pyridyl, benzyloxy or benzoyl, it being possible for the cyclic radicals in turn to carry from one to three of the following: $C_1$-$C_3$-alkyl, $C_1$-$C_3$-alkoxy or halogen;
benzyl which can carry from one to three of the following: $C_1$-$C_3$-alkyl, $C_1$-$C_3$-alkoxy, $C_1$-$C_3$-

haloalkyl, halogen, nitro and cyano;
$C_3$-$C_8$-cycloalkyl;
phenyl which can carry from one to three of the following: $C_1$-$C_4$-alkyl, $C_1$-$C_4$-alkoxy, $C_1$-$C_4$-haloalkyl, $C_1$-$C_4$-haloalkoxy, $C_1$-$C_4$-alkoxycarbonyl, halogen, nitro and cyano;
$C_3$-$C_8$-alkenyl, $C_5$-$C_6$-cycloalkenyl or $C_3$-$C_8$-alkynyl, it being possible for these radicals to carry one of the following: hydroxyl, $C_1$-$C_4$-alkoxy, halogen or a phenyl ring which in turn can carry from one to three of the following: $C_1$-$C_4$-alkyl, $C_1$-$C_4$-alkoxy, $C_1$-$C_4$-haloalkyl, halogen, nitro and cyano;
a 5- to 6-membered heterocyclic radical containing one or two hetero atoms selected from the group comprising oxygen, sulfur and nitrogen or a benzotriazolyl radical;
phthalimido; tetrahydrophthalimido; succinimido; maleimido;
one equivalent of a cation from the group comprising the alkali metals or alkaline earth metals, manganese, copper, iron, ammonium and substituted ammonium;
$-N=CR^6R^7$;
$R^6$ and $R^7$ are hydrogen; $C_1$-$C_4$-alkyl; $C_3$-$C_6$-cycloalkyl; phenyl or furyl, or together form a methylene chain $-(CH_2)_m-$ with m = 4 to 7;

$R^3$ is hydrogen; $C_1$-$C_6$-alkyl which can carry from one to three of the following: hydroxyl, halogen, $C_1$-$C_4$-alkoxy, $C_1$-$C_4$-alkylthio or di-$C_1$-$C_3$-alkylamino;
$C_3$-$C_8$-cycloalkyl which can carry from one to three of the following: $C_1$-$C_4$-alkyl, halogen and $C_1$-$C_4$-haloalkyl;

$R^4$ is hydroxyl; $C_1$-$C_4$-alkoxy;
$C_1$-$C_6$-alkyl which can carry from one to three of the following: $C_1$-$C_4$-alkoxy, $C_1$-$C_4$-haloalkoxy, $C_1$-$C_4$-alkylthio, $C_1$-$C_4$-haloalkylthio, $C_1$-$C_4$-dialkylamino, halogen, $C_3$-$C_8$-cycloalkyl or phenyl which in turn can carry from one to three of the following: halogen, cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio;
$C_3$-$C_8$-cycloalkyl which can carry from one to three of the following: $C_1$-$C_6$-alkyl, $C_1$-$C_6$-haloalkyl, $C_1$-$C_4$-alkoxy, $C_1$-$C_4$-haloalkoxy, halogen, nitro or cyano;
$C_3$-$C_6$-alkenyl or $C_3$-$C_6$-alkynyl, which can be substituted from once to three times by halogen and/or once by phenyl which in turn can carry from one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
a 5- to 6-membered heterocyclic radical which contains one or two hetero atoms selected from the group comprising oxygen, sulfur or nitrogen, and which can carry from one to three of the following: $C_1$-$C_4$-alkyl or halogen;
phenyl which can carry from one to four of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, nitro, cyano, formyl, $C_1$-$C_4$-alkanoyl, $C_1$-$C_4$-haloalkanoyl or $C_1$-$C_4$-alkoxycarbonyl;
naphthyl which can be substituted from once to three times by $C_1$-$C_4$-alkyl or halogen,
or
$R^3$ and $R^4$ together form $-(CH_2)_n-Y_p-(CH_2)_q-$ where n and q are each 1, 2 or 3, p is 0 or 1 and Y is oxygen, sulfur or N-methyl, or $-(CH_2)_3-CO-$.

## Revendications

**1.** Amides d'acide oxazole- ou thiazolecarboxylique de formules Ia et Ib

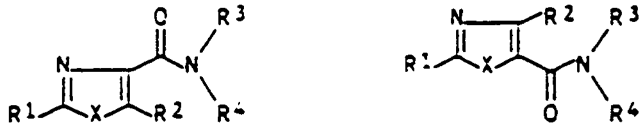

Ia Ib

dans lesquelles les substituants ont la signification suivante:

X désigne l'oxygène ou le soufre;

$R^1$ représente l'hydrogène ou un radical halogéno; alkyle en $C_1$-$C_6$, qui peut porter un à

cinq atomes d'halogène et/ou un ou deux des restes suivants: cycloalkyle en $C_3$-$C_6$, alcoxy en $C_1$-$C_4$, halogénoalcoxy en $C_1$-$C_4$, alkylthio en $C_1$-$C_4$, halogénoalkylthio en $C_1$-$C_4$ ou cyano;
benzyle, qui peut porter un à trois des restes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;
cyclopropyle ou cyclopentyle, qui peuvent porter un à trois des restes suivants: alkyle en $C_1$-$C_4$ ou halogéno;
alcényle en $C_2$-$C_6$, qui peut porter un à trois des restes suivants: halogéno, alcoxy en $C_1$-$C_3$ et/ou un noyau phényle qui peut à son tour porter un à trois des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;
alcynyle en $C_2$-$C_6$, qui peut porter un à trois des restes suivants: halogéno ou alcoxy en $C_1$-$C_3$ et/ou un radical phényle qui peut à son tour porter un à trois des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;
alcoxy en $C_1$-$C_4$; alkylthio en $C_1$-$C_4$; halogénoalcoxy en $C_1$-$C_4$; halogénoalkylthio en $C_1$-$C_4$;
phénoxy ou phénylthio, qui peut porter un à trois des restes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;

$R^2$ représente un radical formyle, 4,5-dihydroxazole-2-yle ou le reste -$COYR^5$;

Y désigne l'oxygène ou le soufre;

$R^5$ désigne l'hydrogène ou un radical
alkyle en $C_1$-$C_6$, qui peut porter un à cinq atomes d'halogène ou des groupes hydroxy et/ou un des restes suivants: alcoxy en $C_1$-$C_4$, alcoxy($C_2$-$C_4$)-alcoxy en $C_1$-$C_4$, cyano, triméthylsilyle, alkylthio en $C_1$-$C_3$, alkylamino en $C_1$-$C_3$, dialkyl($C_1$-$C_3$)amino, cycloalkylamino en $C_3$-$C_7$, alkyl($C_1$-$C_3$)sulfinyle, alkyl($C_1$-$C_3$)sulfonyle, carboxyle, alcoxy($C_1$-$C_3$)-carbonyle, dialkyl($C_1$-$C_3$)aminocarbonyle, dialcoxy-($C_1$-$C_3$)phosphonyle, alcaniminoxy, thiényle, furyle, tétrahydrofuryle, phtalimido, pyridyle, benzyloxy, benzoyle, où les restes cycliques peuvent à leur tour porter un à trois des groupes suivants: alkyle en $C_1$-$C_3$, alcoxy en $C_1$-$C_3$ ou halogéno;
benzyle, qui peut porter un à trois des groupes suivants: alkyle en $C_1$-$C_3$, alcoxy en $C_1$-$C_3$, halogénoalkyle en $C_1$-$C_3$, halogéno, nitro et cyano;
cycloalkyle en $C_3$-$C_8$;
phényle, oui peut porter un à trois des groupes suivants: alkyle en $C_1$-$C_4$, alcoxy en $C_1$-$C_4$, halogénoalkyle en $C_1$-$C_4$, halogénoalcoxy en $C_1$-$C_4$, alcoxy($C_1$-$C_4$)carbonyle, halogéno, nitro et cyano;
alcényle en $C_3$-$C_8$, cycloalcényle en $C_5$-$C_6$ ou alcynyle en $C_3$-$C_8$, tandis que ces restes peuvent porter un des groupes suivants: hydroxy, alcoxy en $C_1$-$C_4$, halogéno ou un noyau phényle, qui peut à son tour porter un à trois des groupes suivants: alkyle en $C_1$-$C_4$, alcoxy en $C_1$-$C_4$, halogénoalkyle en $C_1$-$C_4$, halogéno, nitro et cyano;
un reste hétérocyclique à cinq ou six chaînons contenant un ou deux hétéroatomes, choisis dans le groupe comprenant l'oxygène, le soufre et l'azote, ou un reste benzotriazole;
phtalimido; tétrahydrophtalimido; succinimido; maléinimido;
un équivalent d'un cation du groupe comprenant les métaux alcalins ou alcalino-terreux, le manganèse, le cuivre, le fer, l'ammonium et l'ammonium substitué;
un reste $-N=CR^6R^7$;
où $R^6$, $R^7$ désignent l'hydrogène ou un radical alkyle en $C_1$-$C_4$; cycloalkyle en $C_3$-$C_6$; phényle ou furyle ou ensemble forment une chaîne méthylène de formule $-(CH_2)_m-$ avec m = 4 à 7 chaînons;

$R^3$ représente l'hydrogène ou un radical alkyle en $C_1$-$C_6$, qui peut porter un à trois des substituants suivants: hydroxy, halogéno, alcoxy en $C_1$-$C_4$, alkylthio en $C_1$-$C_4$ ou dialkyl($C_1$-$C_3$)amino;
cycloalkyle en $C_3$-$C_8$, qui peut porter un à trois des restes suivants: alkyle en $C_1$-$C_4$, halogéno et halogénoalkyle en $C_1$-$C_4$;

$R^4$ représente un radical hydroxy ou alcoxy en $C_1$-$C_4$;

alkyle en $C_1$-$C_6$, qui peut porter un à trois des groupes suivants: alcoxy en $C_1$-$C_4$, halogénoalcoxy en $C_1$-$C_4$, alkylthio en $C_1$-$C_4$, halogénoalkylthio en $C_1$-$C_4$, dialkyl($C_1$-$C_4$)amino, halogéno, cycloalkyle en $C_3$-$C_8$ ou phényle, qui peut à son tour porter un à trois des restes suivants: halogéno, cyano, nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ ou halogénoalkylthio en $C_1$-$C_4$;

cycloalkyle en $C_3$-$C_8$, qui peut porter un à trois des groupes suivants: alkyle en $C_1$-$C_6$, halogénoalkyle en $C_1$-$C_6$, alcoxy en $C_1$-$C_4$, halogénoalcoxy en $C_1$-$C_4$,halogéno, nitro ou cyano;

alcényle en $C_3$-$C_6$ ou alcynyle en $C_3$-$C_6$, oui peut être substitué une à trois fois par un halogène et/ou une fois par un radical phényle, tandis que le noyau phényle peut à son tour porter un à trois des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;

un reste hétérocyclique à 5 à 6 chaînons, contenant un ou deux hétéroatomes, choisi dans le groupe comprenant l'oxygène, le soufre ou l'azote, qui peut porter un à trois des restes suivants: alkyle en $C_1$-$C_4$ ou halogéno;

phényle, qui peut porter un à quatre des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, nitro, cyano, formyle, alcanoyle en $C_1$-$C_4$, halogénoalcanoyle en $C_1$-$C_4$ ou alcoxy($C_1$-$C_4$)carbonyle;

naphtyle, qui peut être substitué une à trois fois par un radical alkyle en $C_1$-$C_4$ ou halogéno;

ou

$R^3$ et $R^4$ pris ensemble représentent un reste de structure $-(CH_2)_n-Y_p-(CH_2)_q-$, où n et q valent 1, 2 ou 3, p vaut 0 ou 1 et les Y représentent l'oxygène, le soufre ou un radical N-méthyle, ou forment le reste de formule $-(CH_2)_3-CO-$, ainsi que leurs sels acceptables pour l'environnement, à l'exception des composés de formule Ia, dans lesquels $R^1$ désigne un radical éthyle non-substitué, lorsque simultanément X = soufre, $R^2$ = COOH, $R^3$ désigne l'hydrogène et $R^4$ représente un groupe dialkyl($C_1$-$C_4$)amino-alkyle en $C_1$-$C_3$.

2. Amides d'acide oxazole- ou thiazolecarboxylique de formules Ia et Ib selon la revendication 1, dans lesquels $R^3$ représente l'hydrogène.

3. Amides d'acide oxazole- ou thiazolecarboxylique de formules Ia et Ib selon la revendication 1, dans lesquels les substituants ont les significations suivantes:

$R^1$ désigne l'hydrogène ou un radical alkyle en $C_1$-$C_4$; alcoxy en $C_1$-$C_4$; halogénoalcoxy en $C_1$-$C_4$; alkylthio en $C_1$-$C_4$ ou halogénoalkylthio en $C_1$-$C_4$;

$R^2$ représente un reste $-COYR^5$;

où $R^5$ désigne l'hydrogène ou un radical phtalimido; succinimido; maléinimido ou un reste $-N=R^6R^7$;

où $R^6$ et $R^7$ représentent l'hydrogène ou un radical alkyle en $C_1$-$C_4$ ou cycloalkyle en $C_3$-$C_6$ ou forment ensemble une chaîne méthylène de formule $-(CH_2)_m-$ avec m = 4 à 7 chaînons,

$R^3$ représente l'hydrogène et

$R^4$ représente un radical alkyle en $C_1$-$C_4$ ou cycloalkyle en $C_3$-$C_8$.

4. Procédé pour la préparation des composés Ia et Ib selon la revendication 1, dans lesquels $R^2$ représente un groupe $CO_2R^5$ et $R^5$ un radical alkyle en $C_1$-$C_6$, caractérisé en ce qu'on hydrolyse un diester de formule II

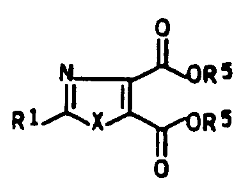

II

de manière connue en soi avec un équivalent d'une base aqueuse pour former un mélange de monoesters IIIa et IIIb

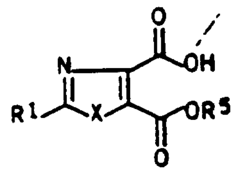

IIIa

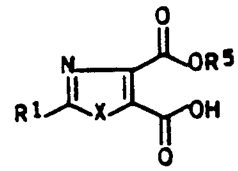

IIIb

et on transforme d'abord IIIa et IIIb, séparés ensuite ou en mélange, en l'halogénure ou en une autre forme activée de l'acide carboxylique et on amide ensuite ces dérivés avec une amine de formule IV

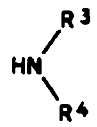

IV

**5.** Procédé pour la préparation des composés Ia et Ib selon la revendication 1, dans lesquels X représente le soufre et $R^2$ désigne $CO_2H$, caractérisé en ce qu'on convertit un anhydride d'acide dicarboxylique de formule V

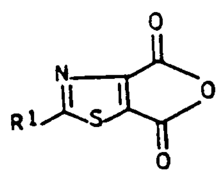

V

de manière connue en soi avec une amine de formule IV selon la revendication 4 en les isomères Ia et Ib et on sépare ensuite les isomères du mélange.

**6.** Procédé pour la préparation des composés Ia et Ib selon la revendication 1, dans lesquels $R^1$ n'est pas un halogène et $R^2$ représente un radical carboxyle ou formyle, caractérisé en ce qu'on active et amide d'abord un acide carboxylique de formule respectivement IIIc ou IIId

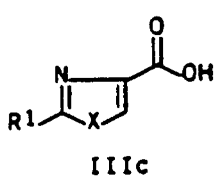

IIIc

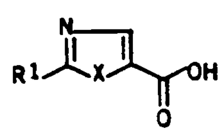

IIId

de manière connue en soi selon la revendication 4 et on fait ensuite réagir l'amide respectivement VIa ou VIb ainsi obtenu

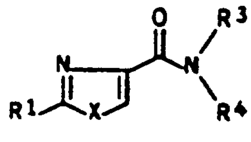

VIa

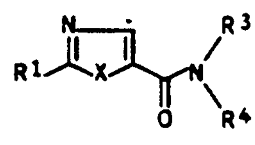

VIb

en présence d'une base avec un réactif de carboxylation ou de formylation.

**7.** Procédé pour la préparation des composés Ia et Ib selon la revendication 1, dans lesquels $R^2$ représente $CO_2H$, caractérisé en ce qu'on hydrolyse un amide d'acide carboxylique respectivement Ia ou Ib dans lequel $R^2$ désigne $CO_2R^5$ et $R^5$ représente un radical alkyle en $C_1$-$C_6$, de manière connue en soi avec un équivalent d'une base aqueuse.

**8.** Procédé pour la préparation des composés Ia et Ib, dans lesquels $R^2$ représente $COYR^5$, caractérisé en ce qu'on active un acide carboxylique correspondant respectivement Ia ou Ib ($R^2$ = $CO_2H$) selon la revendication 4 et on fait ensuite réagir de manière connue en soi avec un composé VII

$HYR^5$ VII

**9.** Procédé pour la préparation de composés Ia et Ib, dans lesquels $R^2$ représente un radical 4,5-dihydrooxazole-2-yle, caractérisé en ce qu'on fait réagir un acide carboxylique correspondant respectivement Ia ou Ib ($R^2$ = $CO_2H$, $CO_2R'$; R' = alkyle en $C_1$-$C_4$) de manière connue en soi avec du 2-aminoéthanol VIII

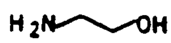

VIII

**10.** Agent herbicide contenant, outre des additifs inertes, au moins un amide d'acide oxazole- ou thiazole-carboxylique de formules respectivement Ia ou Ib selon la revendication 1.

**11.** Procédé pour lutter contre la croissance de plantes parasites, caractérisé en ce qu'on traite les plantes parasites et/ou la surface qui doit rester exempte de plantes parasites avec une quantité efficace du point de vue herbicide d'un amide d'acide oxazole- ou thiazole-carboxylique de formules respectivement Ia' ou Ib'

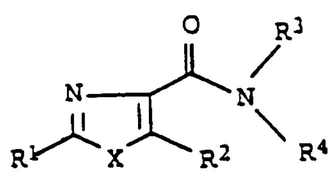

Ia'.

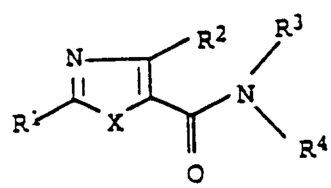

Ib'

où les substituants ont la signification suivante:

X désigne l'oxygène ou le soufre;

$R^1$ représente l'hydrogène ou un radical halogéno; alkyle en $C_1$-$C_6$, qui peut porter un à cinq atomes d'halogène et/ou un ou deux des restes suivants: cycloalkyle en $C_3$-$C_6$, alcoxy en $C_1$-$C_4$, halogénoalcoxy en $C_1$-$C_4$, alkylthio en $C_1$-$C_4$, halogénoalkylthio en $C_1$-$C_4$ ou cyano;

benzyle, qui peut porter un à trois des restes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;

cycloalkyle en $C_3$-$C_8$, qui peut porter un à trois des restes suivants: alkyle en $C_1$-$C_4$ ou halogéno;

alcényle en $C_2$-$C_6$, qui peut porter un à trois des restes suivants: halogéno, alcoxy en $C_1$-$C_3$ et/ou un noyau phényle qui peut à son tour porter un à trois des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;

alcynyle en $C_2$-$C_6$, qui peut porter un à trois des restes suivants: halogéno ou alcoxy en $C_1$-$C_3$ et/ou un radical phényle qui peut à son tour porter un à trois des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;

alcoxy en $C_1$-$C_4$; alkylthio en $C_1$-$C_4$; halogénoalcoxy en $C_1$-$C_4$; halogénoalkylthio en $C_1$-$C_4$;

phénoxy ou phénylthio, qui peut porter un à trois des restes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, cyano ou nitro;

un reste hétérocyclique à 5 à 6 chaînons, contenant un ou deux hétéroatomes, choisis dans le groupe comprenant l'oxygène, le soufre et l'azote, tandis que le cycle peut porter un ou deux des restes suivants: alkyle en $C_1$-$C_3$, halogéno, alcoxy en $C_1$-$C_3$ ou alcoxy($C_1$-$C_3$)carbonyle;

phényle, qui peut porter un à trois des groupes suivants: alkyle en $C_1$-$C_6$, halogénoalkyle en $C_1$-$C_6$, alcoxy en $C_1$-$C_6$, halogénoalcoxy en $C_1$-$C_6$, alkylthio en $C_1$-$C_6$, halogénoalkylthio en $C_1$-$C_6$, halogéno, nitro et cyano;

$R^2$ représente un radical formyle, 4,5-dihydroxazole-2-yle ou le reste $-COYR^5$;

Y désigne l'oxygène ou le soufre;

$R^5$ désigne l'hydrogène ou un radical

alkyle en $C_1$-$C_6$, qui peut porter un à cinq atomes d'halogène ou des groupes hydroxy et/ou un des restes suivants: alcoxy en $C_1$-$C_4$, alcoxy($C_2$-$C_4$)-alcoxy en $C_1$-$C_4$, cyano, triméthylsilyle, alkylthio en $C_1$-$C_3$, alkylamino en $C_1$-$C_3$, dialkyl($C_1$-$C_3$)amino, cycloalkylamino en $C_3$-$C_7$, alkyl($C_1$-$C_3$)sulfinyle, alkyl($C_1$-$C_3$)sulfonyle, carboxyle, alcoxy($C_1$-$C_3$)-carbonyle, dialkyl($C_1$-$C_3$)aminocarbonyle, dialcoxy-($C_1$-$C_3$)phosphonyle, alcaniminoxy, thiényle, furyle, tétrahydrofuryle, phtalimido, pyridyle, benzyloxy, benzoyle, où les restes cycliques peuvent à leur tour porter un à trois des groupes suivants: alkyle en $C_1$-$C_3$, alcoxy en $C_1$-$C_3$ ou halogéno;

benzyle, qui peut porter un à trois des groupes suivants: alkyle en $C_1$-$C_3$, alcoxy en $C_1$-$C_3$, halogénoalkyle en $C_1$-$C_3$, halogéno, nitro et cyano;

cycloalkyle en $C_3$-$C_8$;

phényle, qui peut porter un à trois des groupes suivants: alkyle en $C_1$-$C_4$, alcoxy en $C_1$-$C_4$, halogénoalkyle en $C_1$-$C_4$, halogénoalcoxy en $C_1$-$C_4$, alcoxy($C_1$-$C_4$)carbonyle, halogéno, nitro et cyano;

alcényle en $C_3$-$C_8$, cycloalcényle en $C_5$-$C_6$ ou alcynyle en $C_3$-$C_8$, tandis que ces restes peuvent porter un des groupes suivants: hydroxy, alcoxy en $C_1$-$C_4$, halogéno ou un noyau phényle, qui peut à son tour porter un à trois des groupes suivants: alkyle en $C_1$-$C_4$, alcoxy en $C_1$-$C_4$, halogénoalkyle en $C_1$-$C_4$, halogéno, nitro et cyano;

un reste hétérocyclique à cinq ou six chaînons contenant un ou deux hétéroatomes, choisis dans le groupe comprenant l'oxygène, le soufre et l'azote, ou un reste benzotriazole;

phtalimido; tétrahydrophtalimido; succinimido; maléinimido;

un équivalent d'un cation du groupe comprenant les métaux alcalins ou alcalino-terreux, le manganèse, le cuivre, le fer, l'ammonium et l'ammonium substitué;

un reste $-N=CR^6R^7$;

où $R^6$, $R^7$ désignent l'hydrogène ou un radical alkyle en $C_1$-$C_4$; cycloalkyle en $C_3$-$C_6$; phényle ou furyle ou ensemble forment une chaîne méthylène de formule $-(CH_2)_m-$ avec m = 4 à 7 chaînons;

$R^3$ représente l'hydrogène ou un radical alkyle en $C_1$-$C_6$, qui peut porter un à trois des substituants suivants: hydroxy, halogéno, alcoxy en $C_1$-$C_4$, alkylthio en $C_1$-$C_4$ ou dialkyl($C_1$-$C_3$)amino;

cycloalkyle en $C_3$-$C_8$, qui peut porter un à trois des restes suivants: alkyle en $C_1$-$C_4$, halogéno et halogénoalkyle en $C_1$-$C_4$;

$R^4$ représente un radical hydroxy ou alcoxy en $C_1$-$C_4$;

alkyle en $C_1$-$C_6$, qui peut porter un à trois des groupes suivants: alcoxy en $C_1$-$C_4$, halogénoalcoxy en $C_1$-$C_4$, alkylthio en $C_1$-$C_4$, halogénoalkylthio en $C_1$-$C_4$, dialkyl($C_1$-$C_4$)amino, halogéno, cycloalkyle en $C_3$-$C_8$ ou phényle, qui peut à son tour porter un à trois des restes suivants: halogéno, cyano, nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ ou halogénoalkylthio

en $C_1$-$C_4$;

cycloalkyle en $C_3$-$C_8$, qui peut porter un à trois des groupes suivants: alkyle en $C_1$-$C_6$, halogénoalkyle en $C_1$-$C_6$, alcoxy en $C_1$-$C_4$, halogénoalcoxy en $C_1$-$C_4$,halogéno, nitro ou cyano;

alcényle en $C_3$-$C_6$ ou alcynyle en $C_3$-$C_6$, qui peut être substitué une à trois fois par un halogène et/ou une fois par un radical phényle, tandis que le noyau phényle peut à son tour porter un à trois des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$,, halogéno, cyano ou nitro;

un reste hétérocyclique à 5 à 6 chaînons, contenant un ou deux hétéroatomes, choisi dans le groupe comprenant l'oxygène, le soufre ou l'azote, qui peut porter un à trois des restes suivants: alkyle en $C_1$-$C_4$ ou halogéno;

phényle, qui peut porter un à quatre des groupes suivants: alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, halogéno, nitro, cyano, formyle, alcanoyle en $C_1$-$C_4$, halogénoalcanoyle en $C_1$-$C_4$ ou alcoxy($C_1$-$C_4$)carbonyle;

naphtyle, qui peut être substitué une à trois fois par un radical alkyle en $C_1$-$C_4$ ou halogéno;

ou

$R^3$ et $R^4$ pris ensemble représentent un reste de structure $-(CH_2)_n-Y_p-(CH_2)_q-$, où n et q valent 1, 2 ou 3, p vaut 0 ou 1 et les Y représentent l'oxygène, le soufre ou un radical N-méthyle, ou forment le reste de formule $-(CH_2)_3-CO-$.